# EUROPEAN PATENT APPLICATION

(11) **EP 2 182 057 A1**
(43) Date of publication of application: **05.05.2010**
(21) Application number: 10151010.5
(22) Date of filing: 31.08.2006
(51) Int. Cl.: C12N 9/12

(54) **Antibody agains phosphorylated Tyrosine for the detection of protein phosphorylation in carcinoma signaling pathways**

(30) Priority: 31.08.2005 US 713041 P
(62) Divisional of application: 06814000.3
(71) Applicant: Cell Signaling Technology, Inc., Danvers MA 01923 (US)
(72) Inventor: Polakiewicz, Roberto, Lexington, MA 02421 (US)
(74) Representative: Hill, Justin John

(57) **Abstract**

The invention relates to antibodies and peptide reagents for the detection of protein phosphorylation, and to protein phosphorylation in cancer.

## Description

### RELATED APPLICATIONS

This application claims the benefit of, and priority to, U.S.S.N. 60/713,041, filed August 31, 2005, presently pending, the disclosure of which is incorporated herein, in its entirety, by reference.

### FIELD OF THE INVENTION

The invention relates generally to antibodies and peptide reagents for the detection of protein phosphorylation, and to protein phosphorylation in cancer.

### BACKGROUND OF THE INVENTION

The activation of proteins by post-translational modification is an important cellular mechanism for regulating most aspects of biological organization and control, including growth, development, homeostasis, and cellular communication. Protein phosphorylation, for example, plays a critical role in the etiology of many pathological conditions and diseases, including cancer, developmental disorders, autoimmune diseases, and diabetes. Yet, in spite of the importance of protein modification, it is not yet well understood at the molecular level, due to the extraordinary complexity of signaling pathways, and the slow development of technology necessary to unravel it.

Protein phosphorylation on a proteome-wide scale is extremely complex as a result of three factors: the large number of modifying proteins, *e*.*g*. kinases, encoded in the genome, the much larger number of sites on substrate proteins that are modified by these enzymes, and the dynamic nature of protein expression during growth, development, disease states, and aging. The human genome, for example, encodes over 520 different protein kinases, making them the most abundant class of enzymes known. See Hunter, Nature 411: 355-65 (2001). Moist kinases phosphorylate many different substrate proteins, at distinct tyrosine, serine, and/or threonine residues. Indeed, it is estimated that one-third of all proteins encoded by the human genome are phosphorylated, and many are phosphorylated at multiple sites by different kinases.

Many of these phosphorylation sites regulate critical biological processes and may prove to be important diagnostic or therapeutic targets for molecular medicine. For example, of the more than 100 dominant oncogenes identified to date, 46 are protein kinases. *See* Hunter, *supra.* Understanding which proteins are modified by these kinases will greatly expand our understanding of the molecular mechanisms underlying oncogenic transformation. Therefore, the identification of, and ability to detect, phosphorylation sites on a wide variety of cellular proteins is crucially important to understanding the key signaling proteins and pathways implicated in the progression of diseases like cancer.

Carcinoma is one of the two main categories of cancer, and is generally characterized by the formation of malignant tumors or cells of epithelial tissue original, such as skin, digestive tract, glands, etc. Carcinomas are malignant by definition, and tend to metastasize to other areas of the body. The most common forms of carcinoma are skin cancer, lung cancer, breast cancer, and colon cancer, as well as other numerous but less prevalent carcinomas. Current estimates show that, collectively, various carcinomas will account for approximately 1.65 million cancer diagnoses in the United States alone, and more than 300,000 people will die from some type of carcinoma during 2005. (Source: American Cancer Society (2005)). The worldwide incidence of carcinoma is much higher.

As with many cancers, deregulation of receptor tyrosine kinases (RTKs) appears to be a central theme in the etiology of carcinomas. Constitutively active RTKs can contribute not only to unrestricted cell proliferation, but also to other important features of malignant tumors, such as evading apoptosis, the ability to promote blood vessel growth, the ability to invade other tissues and build metastases at distant sites (see Blume-Jensen et al., Nature 411: 355-365 (2001)). These effects are mediated not only through aberrant activity of RTKs themselves, but, in turn, by aberrant activity of their downstream signaling molecules and substrates.

The importance of RTKs in carcinoma progression has led to a very active search for pharmacological compounds that can inhibit RTK activity in tumor cells, and more recently to significant efforts aimed at identifying genetic mutations in RTKs that may occur in, and affect progression of, different types of carcinomas (*see, e*.*g*., Bardell et al., Science 300: 949 (2003); Lynch et al., N. Eng. J. Med. 350: 2129-2139 (2004)). For example, non-small cell lung carcinoma patients carrying activating mutations in the epidermal growth factor receptor (EGFR), an RTK, appear to respond better to specific EGFR inhibitors than do patients without such mutations (Lynch *et al., supra*.; Paez et al., Science 304: 1497-1500 (2004)).

Clearly, identifying activated RTKs and downstream signaling molecules driving the oncogenic phenotype of carcinomas would be highly beneficial for understanding the underlying mechanisms of this prevalent form of cancer, identifying novel drug targets for the treatment of such disease, and for assessing appropriate patient treatment with selective kinase inhibitors of relevant targets when and if they become available.

However, although a few key RTKs involved in carcinoma progression are knowns, there is relatively scarce information about kinase-driven signaling pathways and phosphorylation sites that underly the different types of carcinoma. Therefore there is presently an incomplete and inaccurate understanding of how protein activation within signaling pathways is driving these complex cancers. Accordingly, there is a continuing and pressing need to unravel the molecular mechanisms of kinase-driven oncogenesis in carcinoma by identifying the downstream signaling proteins mediating cellular transformation in these cancers. Identifying particular phosphorylation sites on such signaling proteins and providing new reagents, such as phospho-specific antibodies and AQUA peptides, to detect and quantify them remains especially important to advancing our understanding of the biology of this disease.

Presently, diagnosis of carcinoma is made by tissue biopsy and detection of different cell surface markers. However, misdiagnosis can occur since some carcinoma cases can be negative for certain markers and because these markers may not indicate which genes or protein kinases may be deregulated. Although the genetic translocations and/or mutations characteristic of a particular form of carcinoma can be sometimes detected, it is clear that other downstream effectors of constitutively active kinases having potential diagnostic, predictive, or therapeutic value, remain to be elucidated. Accordingly, identification of downstream signaling molecules and phosphorylation sites involved in different types of carcinoma and development of new reagents to detect and quantify these sites and proteins may lead to improved diagnostic/prognostic markers, as well as novel drug targets, for the detection and treatment of this disease.

### SUMMARY OF THE INVENTION

The invention discloses nearly 474 novel phosphorylation sites identified in signal transduction proteins and pathways underlying human carcinomas and provides new reagents, including phosphorylation-site specific antibodies and AQUA peptides, for the selective detection and quantification of these phosphorylated sites/proteins. Also provided are methods of using the reagents of the invention for the detection, quantification, and profiling of the disclosed phosphorylation sites.

### BRIEF DESCRIPTION OF THE DRAWINGS

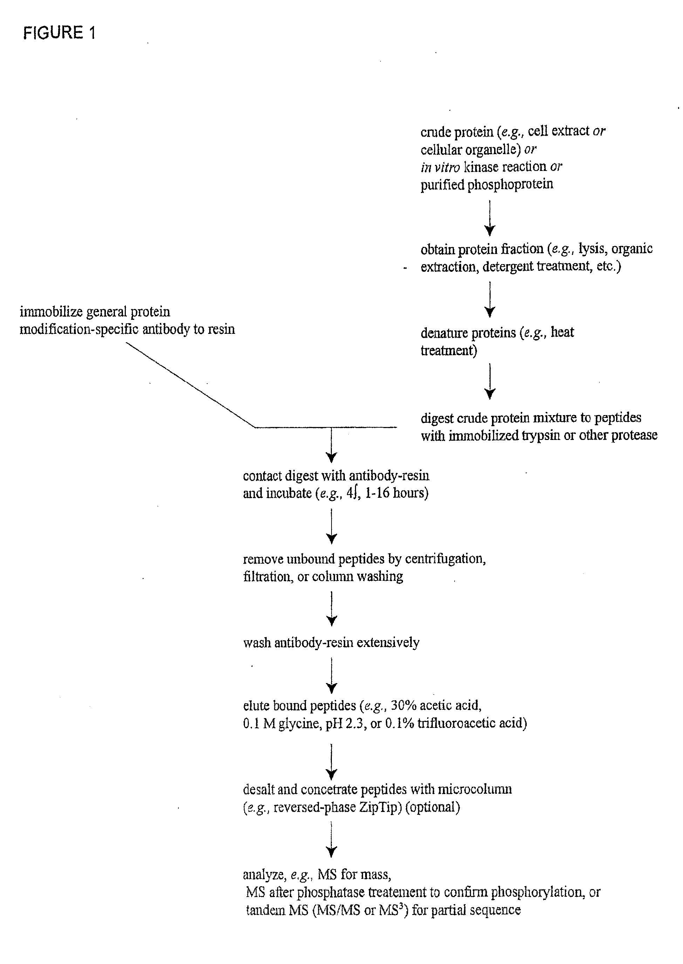

**FIGURE 1** **-** Is a diagram broadly depicting the immunoaffinity isolation and mass-spectrometric characterization methodology (IAP) employed to identify the novel phosphorylation sites disclosed herein.

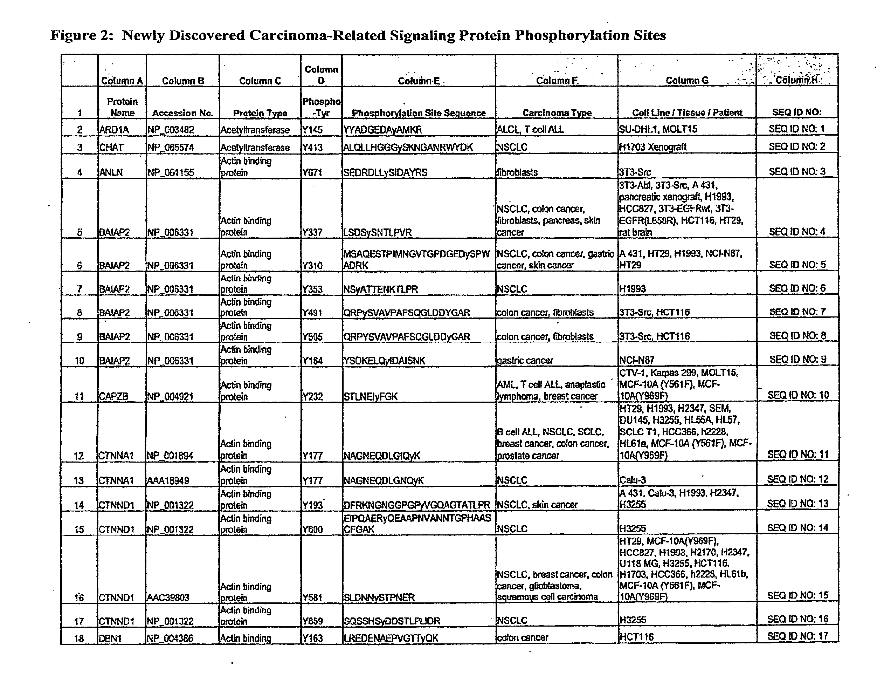

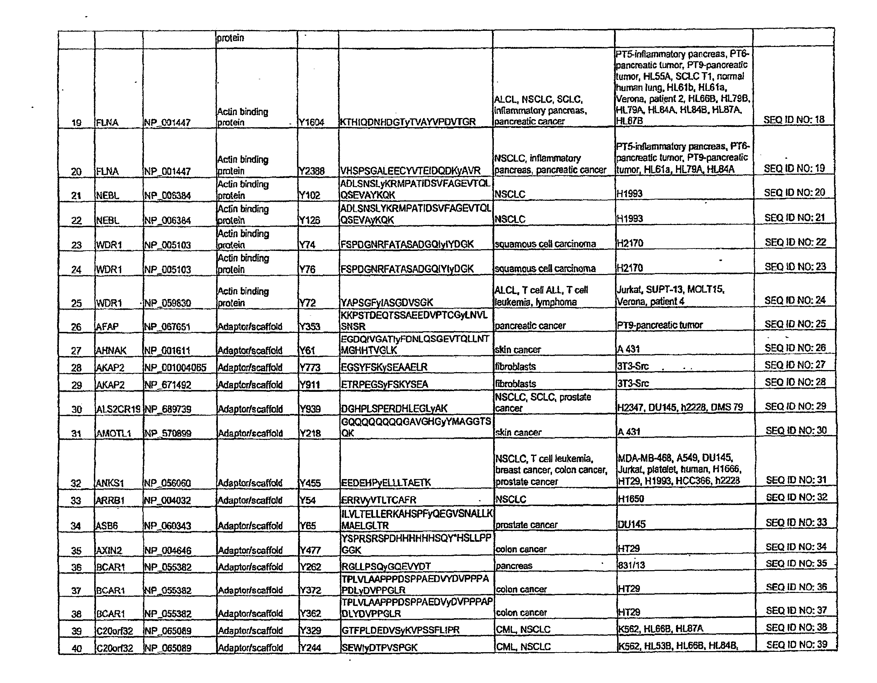

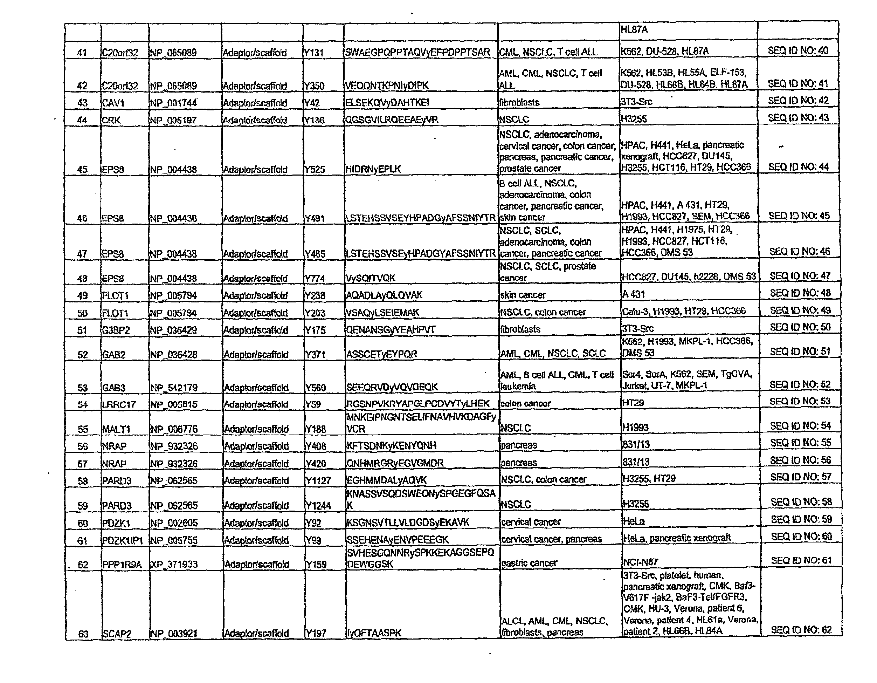

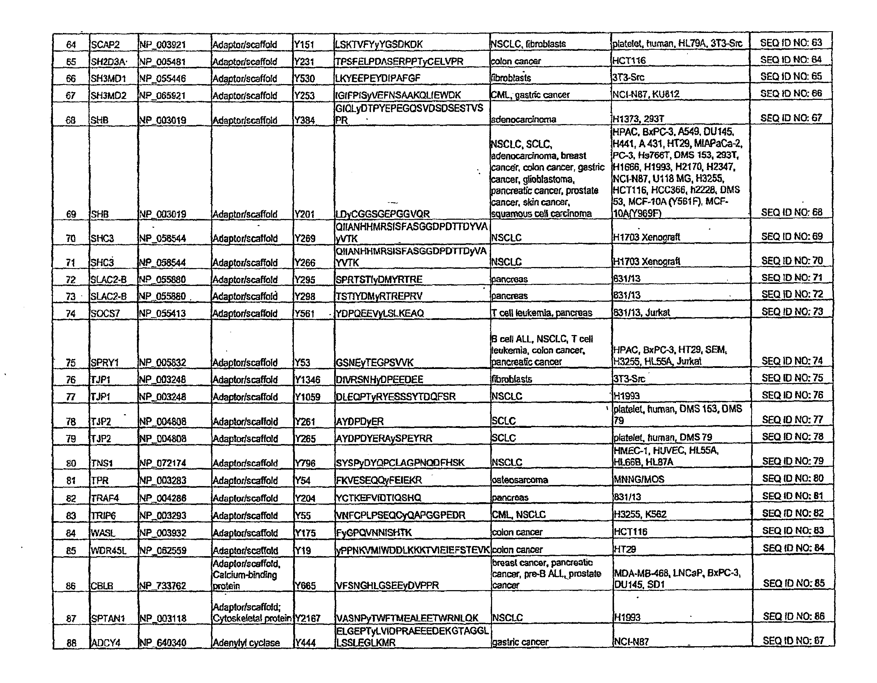

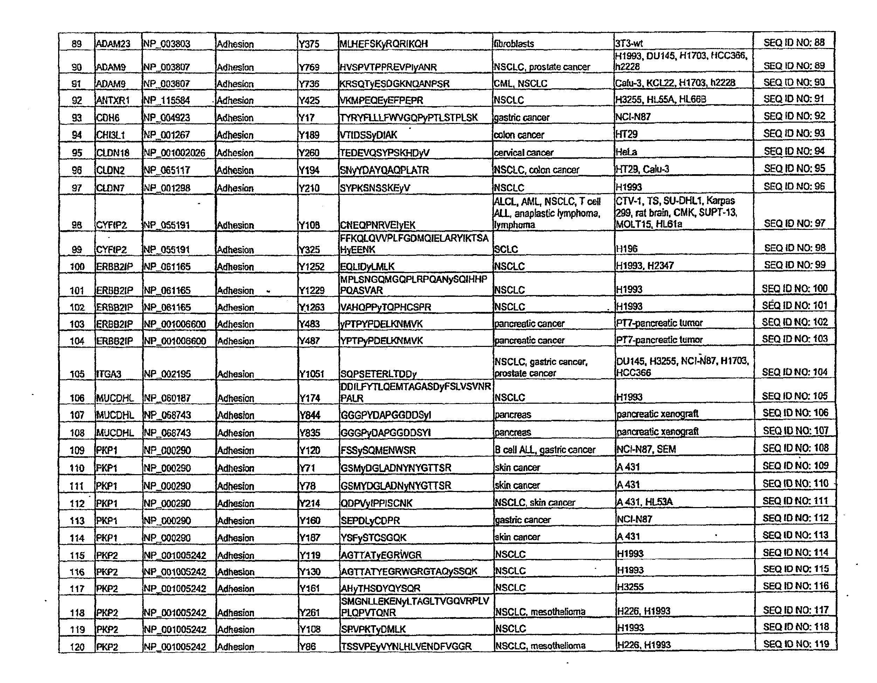

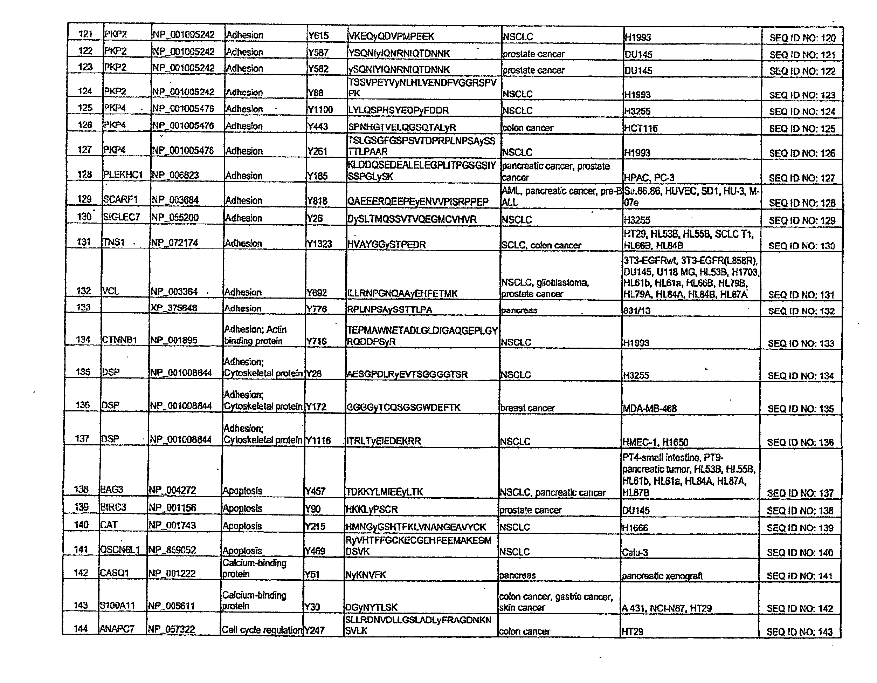

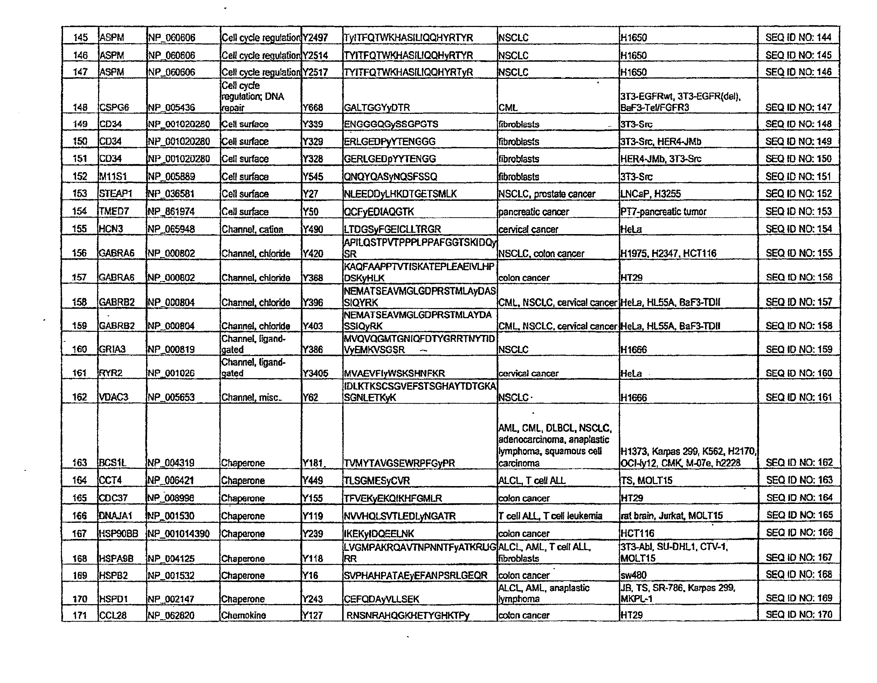

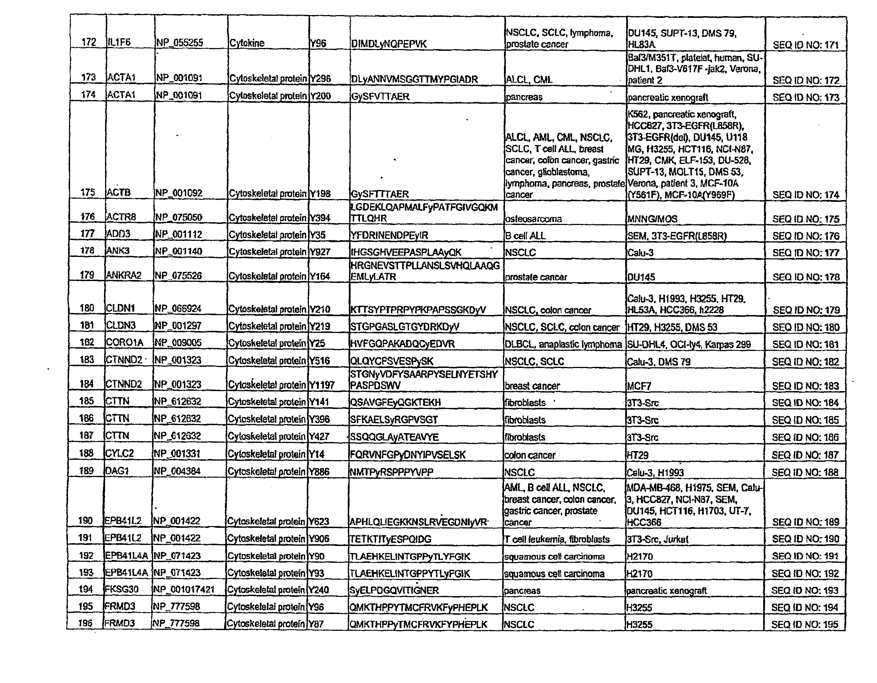

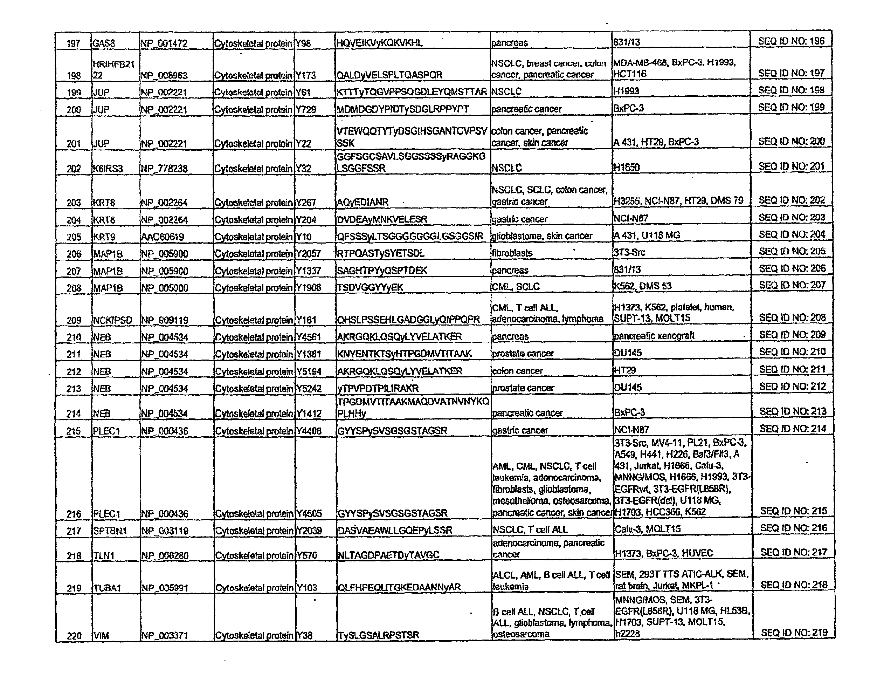

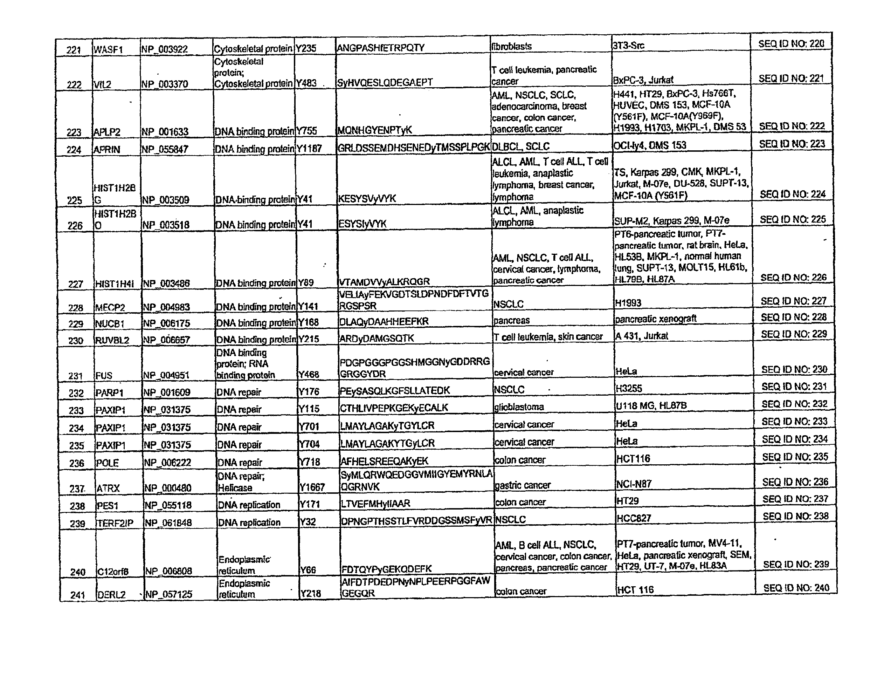

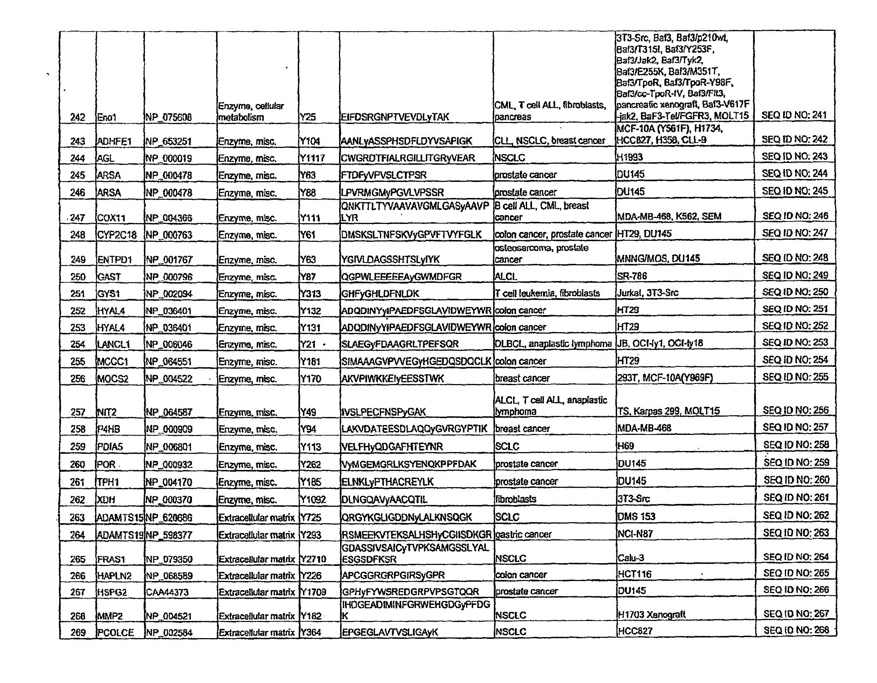

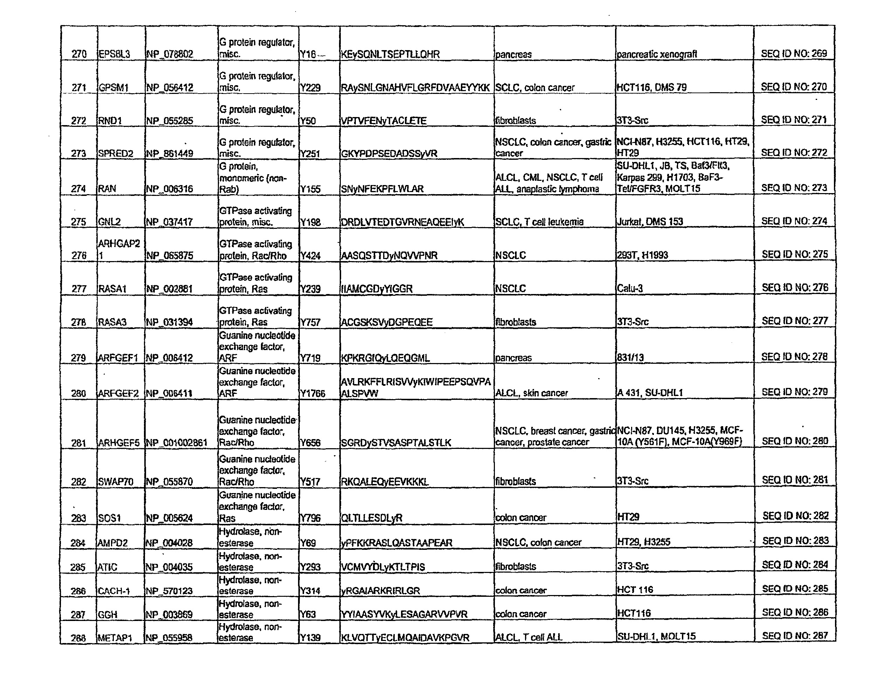

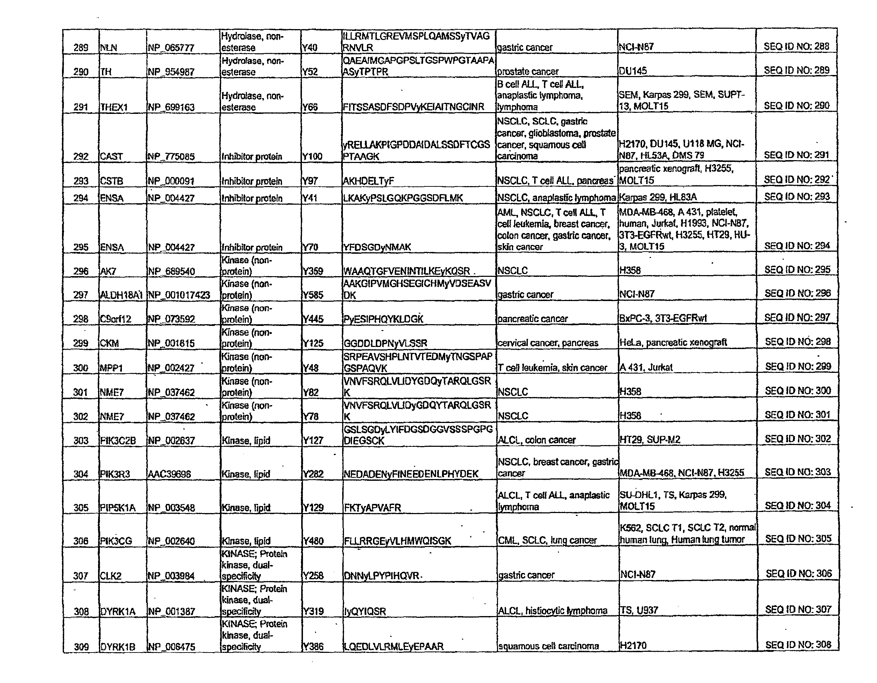

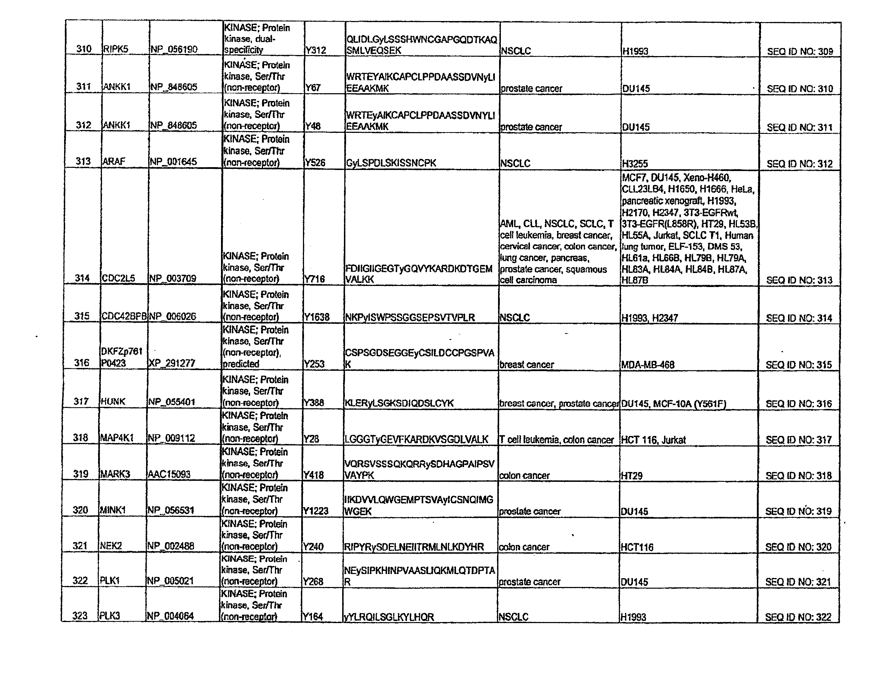

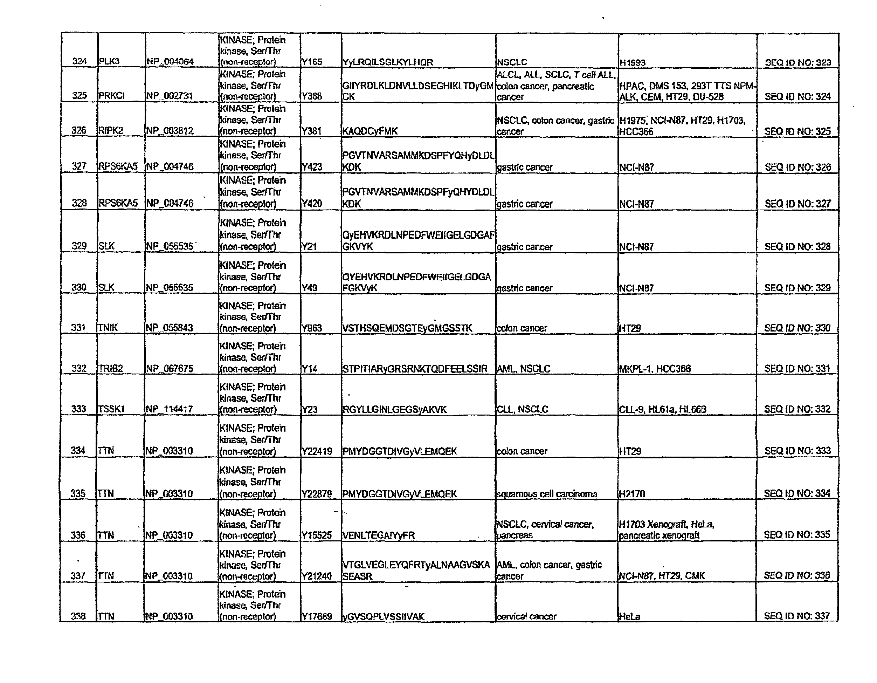

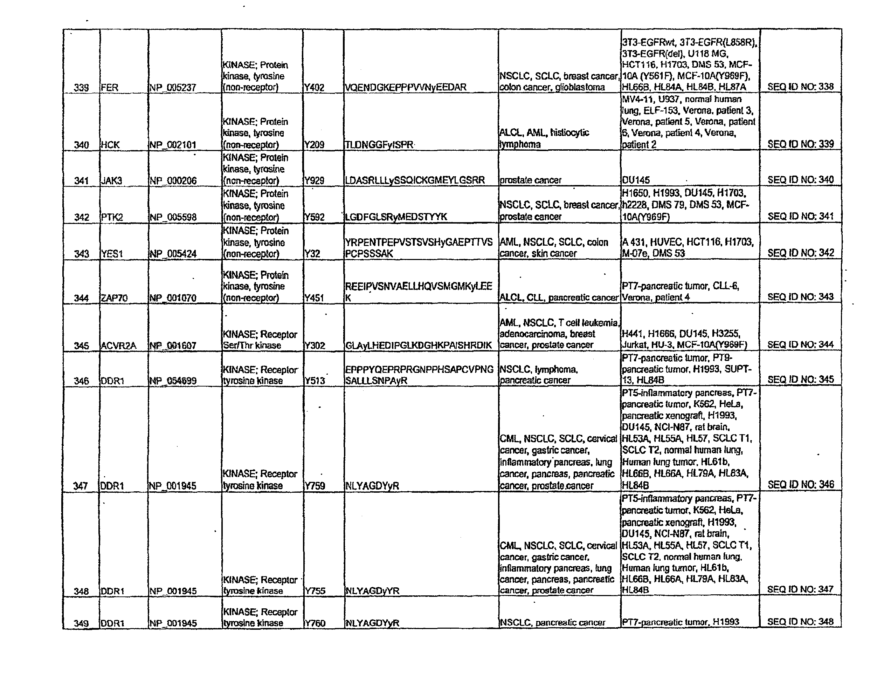

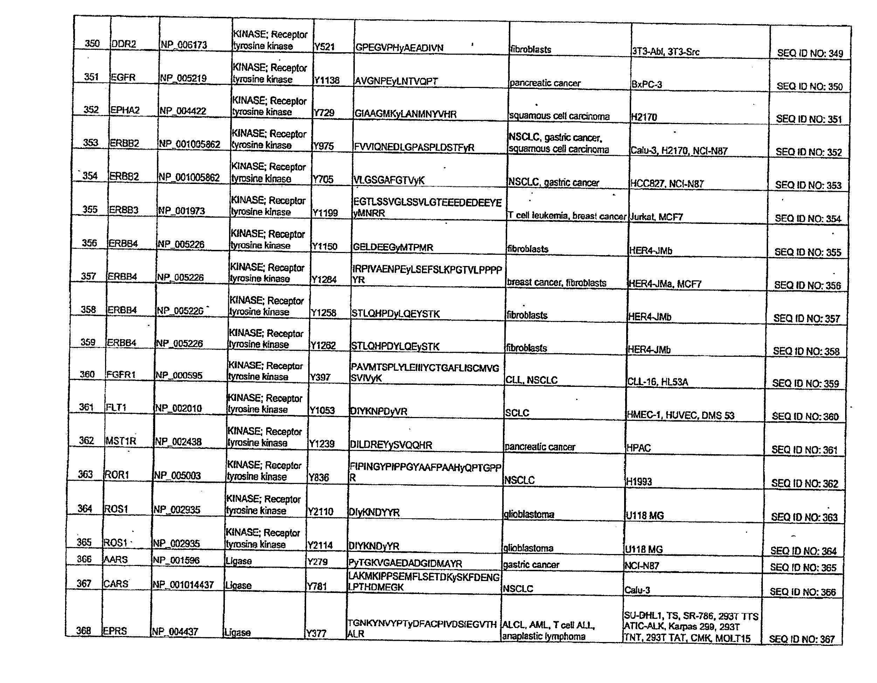

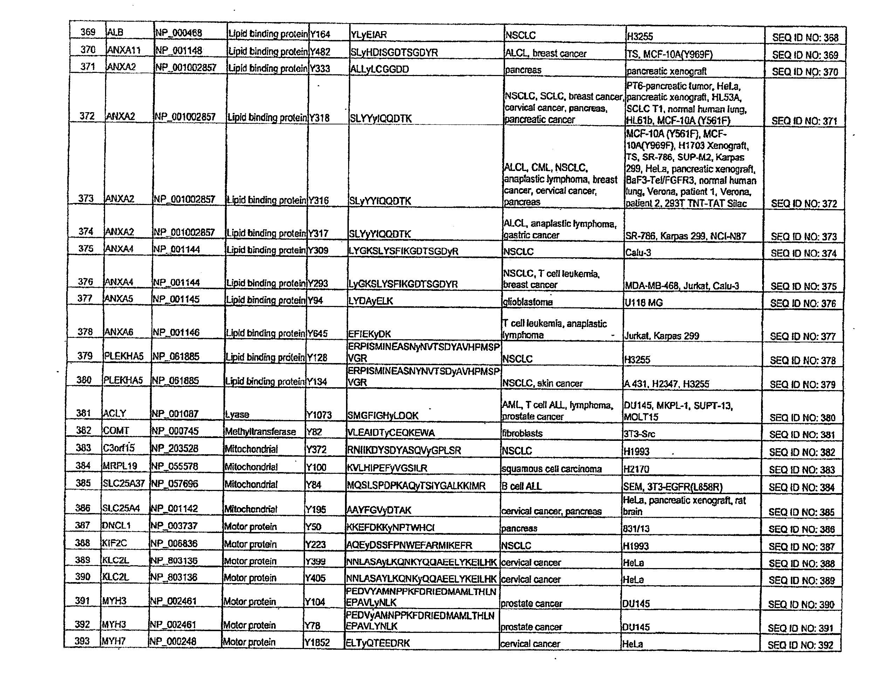

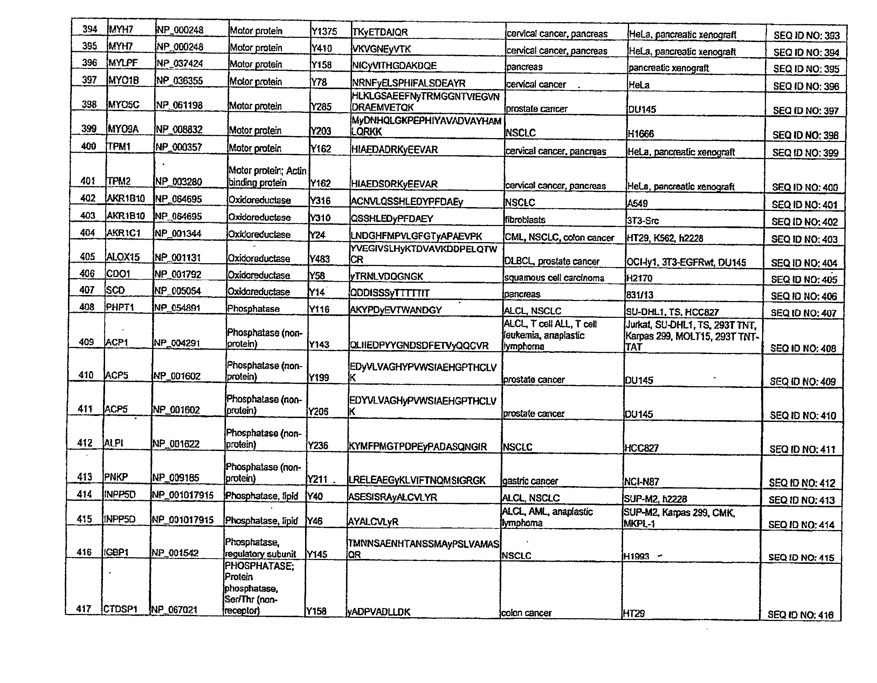

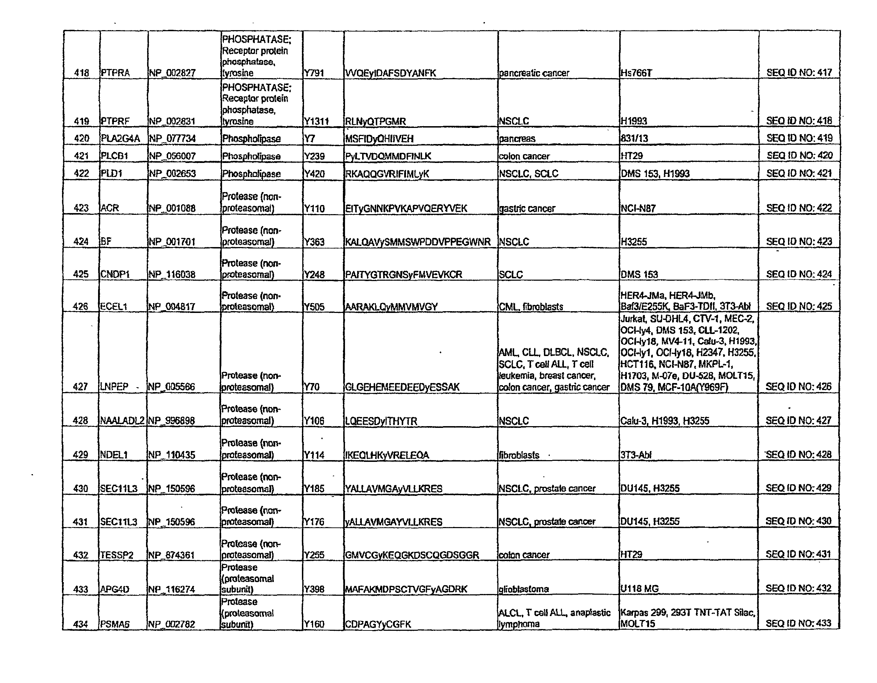

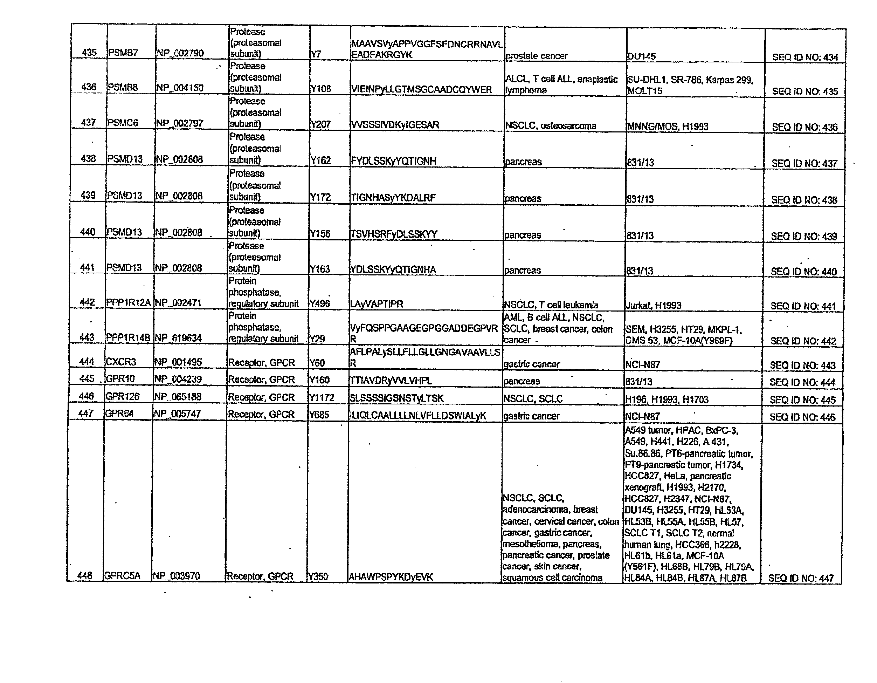

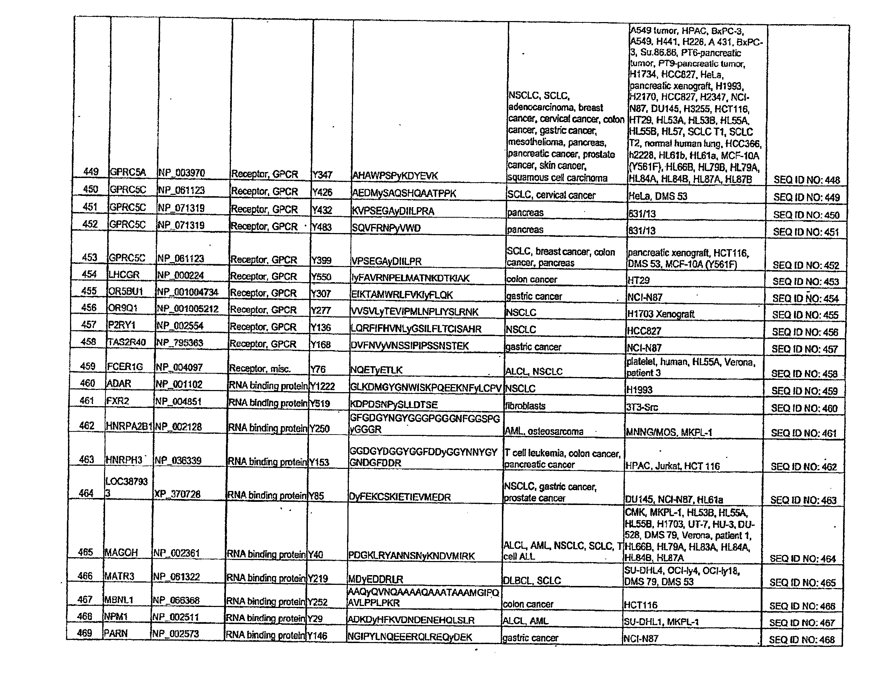

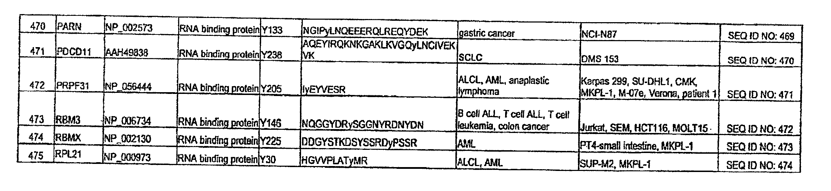

**FIGURE 2** - Is a table (corresponding to Table 1) enumerating the 474 carcinoma signaling protein phosphorylation sites disclosed herein: Column A = the name of the parent protein; Column B = the SwissProt accession number for the protein (human sequence); Column C = the protein type/classification; Column D = the tyrosine residue (in the parent protein amino acid sequence) at which phosphorylation occurs within the phosphorylation site; Column E = the phosphorylation site sequence encompassing the phosphorylatable residue (residue at which phosphorylation occurs (and corresponding to the respective entry in Column D) appears in lowercase; Column F = the type of carcinoma in which the phosphorylation site was discovered; Column G = the cell type(s) in which the phosphorylation site was discovered; and Column H= the SEQ ID NO.

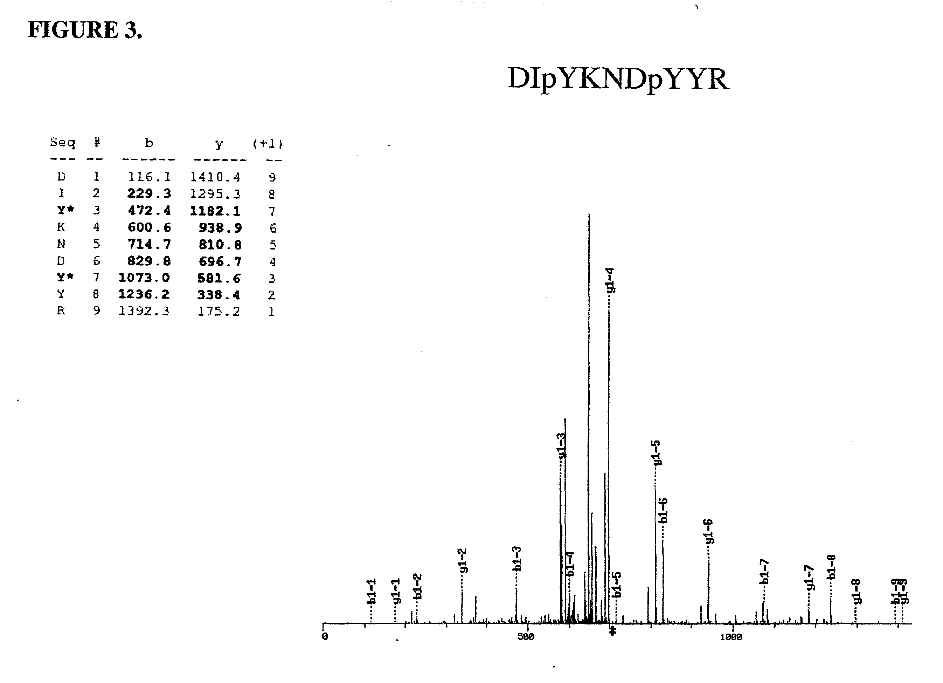

**FIGURE 3** - is an exemplary mass spectrograph depicting the detection of the tyrosine 2110 and 2114 phosphorylation sites in ROS (see Rows 364 and 365 in Figure 2/Table 1), as further described in Example 1 (red and blue indicate ions detected in MS/MS spectrum); Y* (and pY) indicates the phosphorylated tyrosine (shown as lowercase "y" in Figure 2).

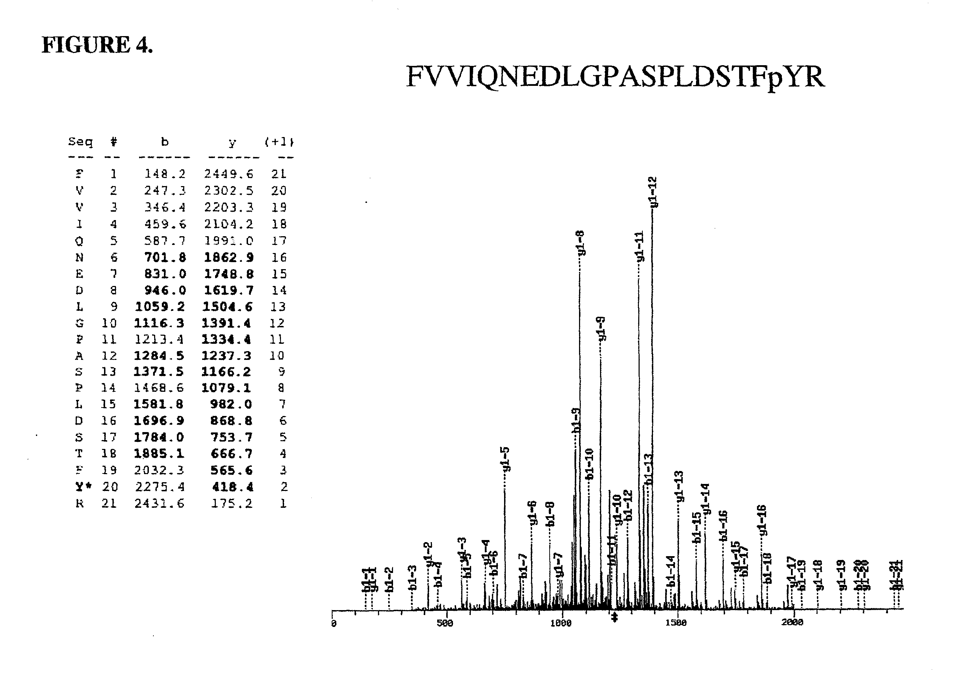

**FIGURE 4** - is an exemplary mass spectrograph depicting the detection of the tyrosine 975 phosphorylation site in ERBB2 (see Row 353 in Figure 2/Table 1), as further described in Example 1 (red and blue indicate ions detected in MS/MS spectrum); Y* (and pY) indicates the phosphorylated tyrosine (shown as lowercase "y" in Figure 2).

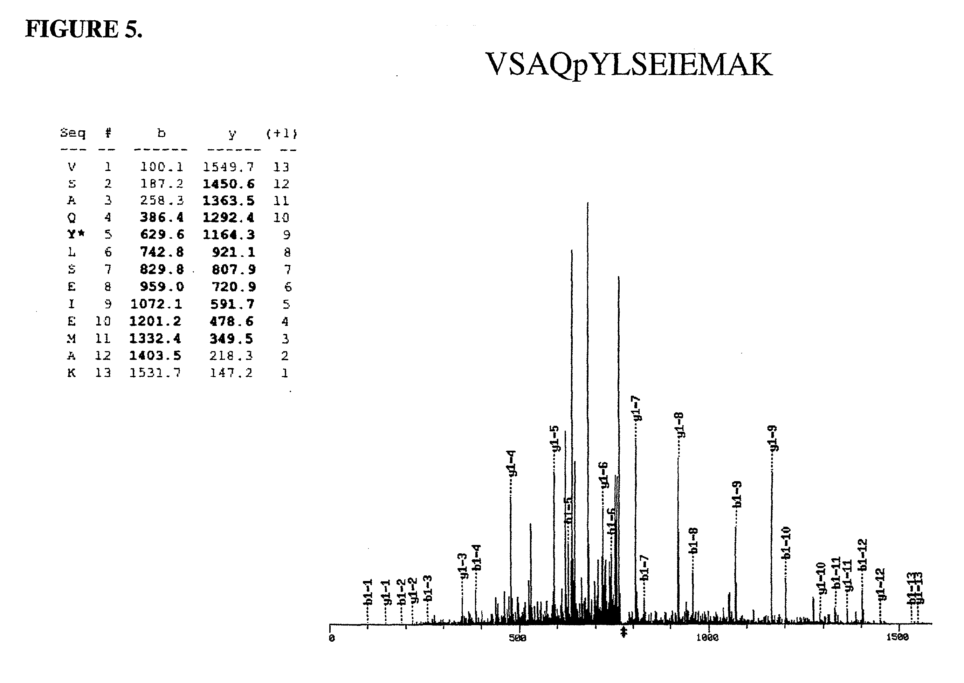

**FIGURE 5** - is an exemplary mass spectrograph depicting the detection of the tyrosine 238 phosphorylation site in FLOT-1 (see Row 49 in Figure 2/Table 1), as further described in Example 1 (red and blue indicate ions detected in MS/MS spectrum); Y* (and pY) indicates the phosphorylated tyrosine (shown as lowercase "y" in Figure 2) and M# (and lowercase "m") indicates an oxidized methionine also detected.

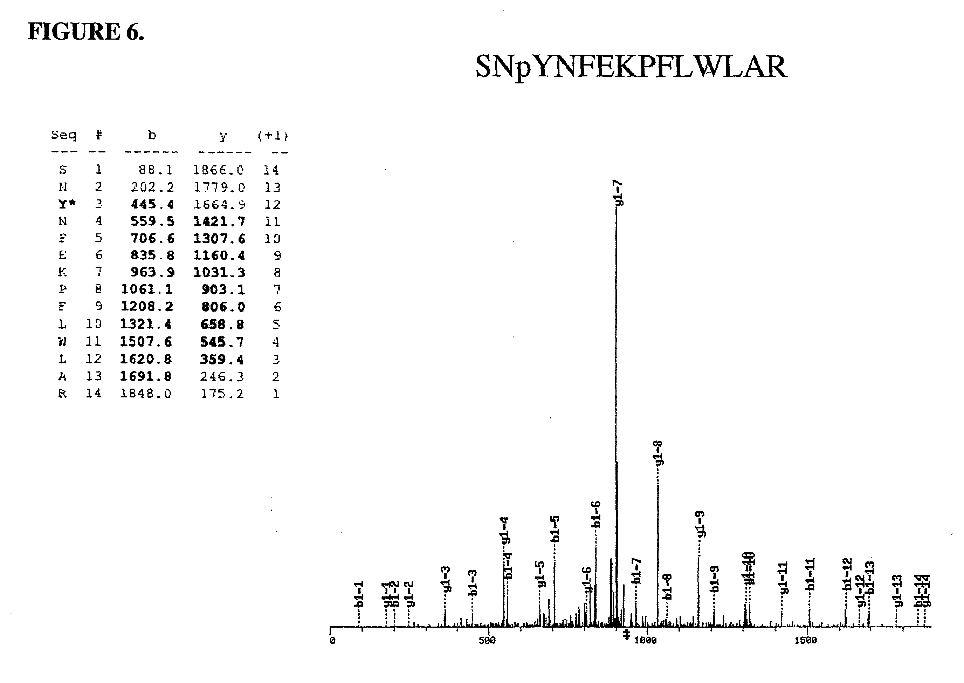

**FIGURE 6** **-** is an exemplary mass spectrograph depicting the detection of the tyrosine 455 phosphorylation site in RAN (see Row 274 in Figure 2/Table 1), as further described in Example 1 (red and blue indicate ions detected in MS/MS spectrum); Y* (and pY) indicates the phosphorylated tyrosine (shown as lowercase "y" in Figure 2).

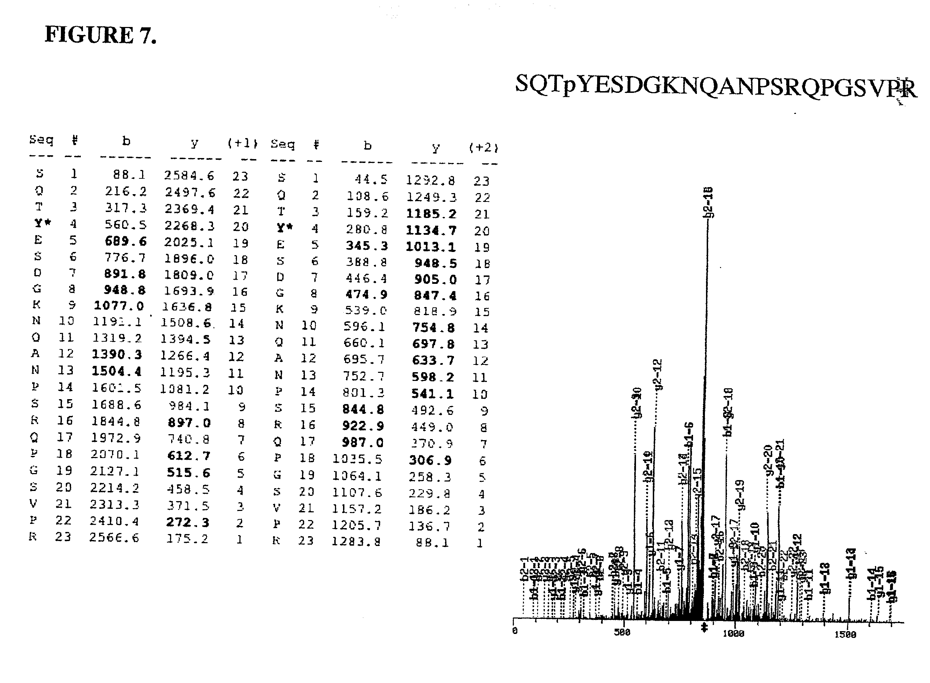

**FIGURE 7** - is an exemplary mass spectrograph depicting the detection of the tyrosine 736 phosphorylation site in ADAM9 (see Row 90 in Figure 2/ Table 1), as further described in Example 1 (red and blue indicate ions detected in MS/MS spectrum); Y* (and pY) indicates the phosphorylated tyrosine (shown as lowercase "y" in Figure 2).

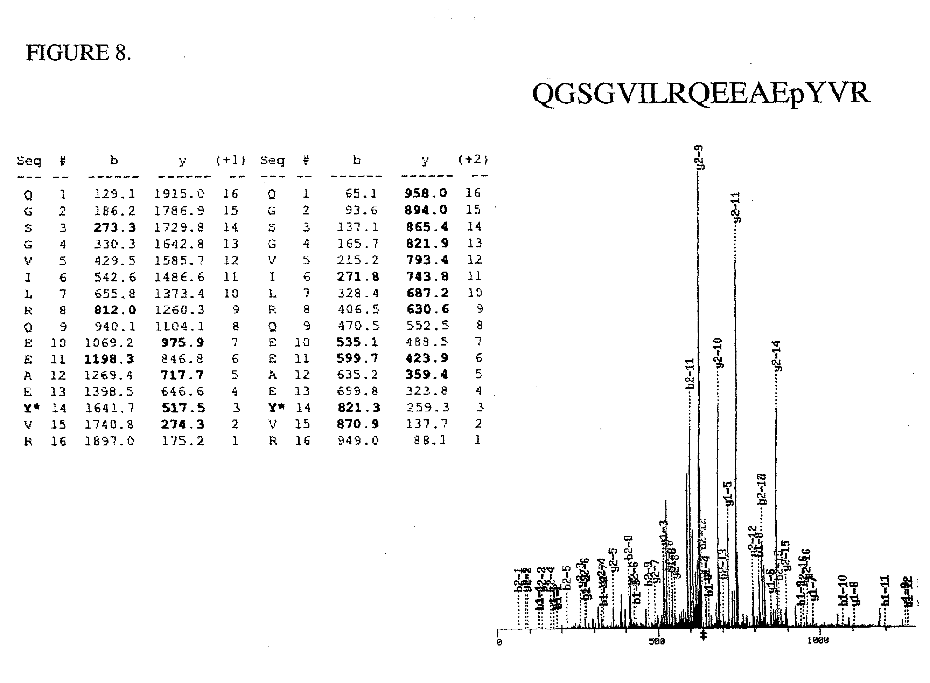

**FIGURE 8** - is an exemplary mass spectrograph depicting the detection of the tyrosine 136 phosphorylation site in CRK (see Row 44 in Figure 2/ Table 1), as further described in Example 1 (red and blue indicate ions detected in MS/MS spectrum); Y* (and pY) indicates the phosphorylated tyrosine (shown as lowercase "y" in Figure 2).

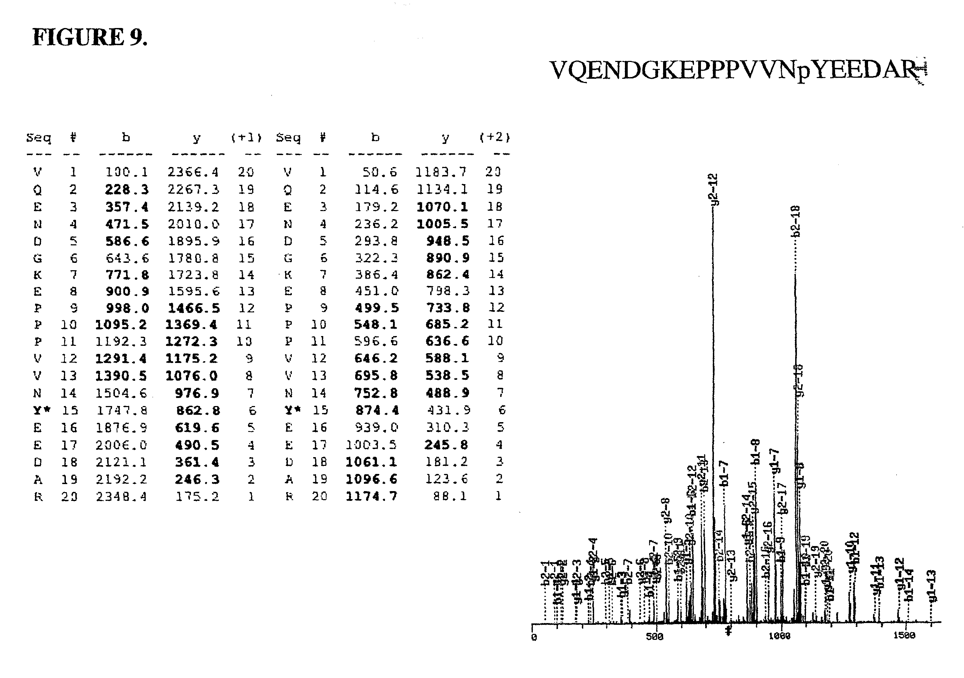

**FIGURE 9** - is an exemplary mass spectrograph depicting the detection of the tyrosine 402 phosphorylation site in FER (see Row 339 in Figure 2/ Table 1), as further described in Example 1 (red and blue indicate ions detected in MS/MS spectrum); Y* (and pY) indicates the phosphorylated tyrosine (shown as lowercase "y" in Figure 2).

### DETAILED DESCRIPTION OF THE INVENTION

In accordance with the present invention, nearly 474 novel protein phosphorylation sites in signaling proteins and pathways underlying carcinoma have now been discovered. These newly described phosphorylation sites were identified by employing the techniques described in "Immunoaffinity Isolation of Modified Peptides From Complex Mixtures," U.S. Patent Publication No. 20030044848, Rush et al., using cellular extracts from a variety of human carcinoma-derived cell lines, such as H69 LS, HT29, MCF10, A431, etc., as further described below. The novel phosphorylation sites (tyrosine), and their corresponding parent proteins, disclosed herein are listed in Table 1.

These phosphorylation sites correspond to numerous different parent proteins (the full sequences of which (human) are all publicly available in SwissProt database and their Accession numbers listed in Column B of Table 1/Figure 2), each of which fall into discrete protein type groups, for example Protein Kinases (Serine/Threonine nonreceptor, Tyrosine receptor, Tyrosine nonreceptor, dual specificity and other), Adaptor/Scaffold proteins, Cytoskeletal proteins, and Cellular Metabolism enzymes, etc. (see Column C of Table 1), the phosphorylation of which is relevant to signal transduction activity underlying carcinomas (*e*.*g*., skin, lung, breast and colon cancer), as disclosed herein.

The discovery of the nearly 474 novel protein phosphorylation sites described herein enables the production, by standard methods, of new reagents, such as phosphorylation site-specific antibodies and AQUA peptides (heavy-isotope labeled peptides), capable of specifically detecting and/or quantifying these phosphorylated sites/proteins. Such reagents are highly useful, *inter alia*, for studying signal transduction events underlying the progression of carcinoma. Accordingly, the invention provides novel reagents -- phospho-specific antibodies and AQUA peptides -- for the specific detection and/or quantification of a Carcinoma-related signaling protein/polypeptide only when phosphorylated (or only when not phosphorylated) at a particular phosphorylation site disclosed herein. The invention also provides methods of detecting and/or quantifying one or more phosphorylated Carcinoma-related signaling proteins using the phosphorylation-site specific antibodies and AQUA peptides of the invention, and methods of obtaining a phosphorylation profile of such proteins (*e*.*g*. Kinases).

In part, the invention provides an isolated phosphorylation site-specific antibody that specifically binds a given Carcinoma-related signaling protein only when phosphorylated (or not phosphorylated, respectively) at a particular tyrosine enumerated in Column D of Table 1/Figure 2 comprised within the phosphorylatable peptide site sequence enumerated in corresponding Column E. In further part, the invention provides a heavy-isotope labeled peptide (AQUA peptide) for the detection and quantification of a given Carcinoma-related signaling protein, the labeled peptide comprising a particular phosphorylatable peptide site/sequence enumerated in Column E of Table 1/Figure 2 herein. For example, among the reagents provided by the invention is an isolated phosphorylation site-specific antibody that specifically binds the RIPK5 kinase (serine/threonine) only when phosphorylated (or only when not phosphorylated) at tyrosine 312 (see Row 310 (and Columns D and E) of Table 1/Figure 2). By way of further example, among the group of reagents provided by the invention is an AQUA peptide for the quantification of phosphorylated RIPK5 kinase, the AQUA peptide comprising the phosphorylatable peptide sequence listed in Column E, Row 310 of Table 1/Figure 2 (which encompasses the phosphorylatable tyrosine at position 312).

In one embodiment, the invention provides an isolated phosphorylation site-specific antibody that specifically binds a human Carcinoma-related signaling protein selected from Column A of Table 1 (Rows 2-475) only when phosphorylated at the tyrosine residue listed in corresponding Column D of Table 1, comprised within the phosphorylatable peptide sequence listed in corresponding Column E of Table 1 (SEQ ID NOs: 1-2, 5-6, 9-11, 13-35, 38-44, 46-49, 51-61, 63-67, 69-80, 83-129, 131, 133-147, 151-188, 191-210, 212-219, 221-240, 242-317, 319-333, 335-344, 346-347, 349, 351-355, 357-400, 402-425, 427-446, 449-451, 453-459, and 461-474), wherein said antibody does not bind said signaling protein when not phosphorylated at said tyrosine. In another embodiment, the invention provides an isolated phosphorylation site-specific antibody that specifically binds a Carcinoma-related signaling protein selected from Column A of Table 1 only when not phosphorylated at the tyrosine residue listed in corresponding Column D of Table 1, comprised within the peptide sequence listed in corresponding Column E of Table 1 (SEQ ID NOs: 1-2, 5-6, 9-11, 13-35, 38-44, 46-49, 51-61, 63-67, 69-80, 83-129, 131, 133-147, 151-188, 191-210, 212-219, 221-240, 242₋317, 319-333, 335-344, 346-347, 349, 351-355, 357-400, 402-425, 427-446, 449-451, 453-459, and 461-474), wherein said antibody does not bind said signaling protein when phosphorylated at said tyrosine. Such reagents enable the specific detection of phosphorylation (or non-phosphorylation) of a novel phosphorylatable site disclosed herein. The invention further provides immortalized cell lines producing such antibodies. In one preferred embodiment, the immortalized cell line is a rabbit or mouse hybridoma.

In another embodiment, the invention provides a heavy-isotope labeled peptide (AQUA peptide) for the quantification of a Carcinoma-related signaling protein selected from Column A of Table 1, said labeled peptide comprising the phosphorylatable peptide sequence listed in corresponding Column E of Table 1 (SEQ ID NOs: 1-2, 5-6, 9-11, 13-35, 38-44, 46-49, 51-61, 63-67, 69-80, 83-129, 131, 133-147, 151-188, 191-210, 212-219, 221-240, 242-317, 319-333, 335-344, 346-347, 349, 351-355, 357-400, 402-425, 427-446, 449-451, 453-459, and 461-474), which sequence comprises the phosphorylatable tyrosine listed in corresponding Column D of Table 1. In certain preferred embodiments, the phosphorylatable tyrosine within the labeled peptide is phosphorylated, while in other preferred embodiments, the phosphorylatable residue within the labeled peptide is not phosphorylated.

Reagents (antibodies and AQUA peptides) provided by the invention may conveniently be grouped by the type of Carcinoma-related signaling protein in which a given phosphorylation site (for which reagents are provided) occurs. The protein types for each respective protein (in which a phosphorylation site has been discovered) are provided in Column C of Table 1/Figure 2, and include: Acetyltransferease, Actin binding proteins, Adaptor/Scaffold proteins, Adenylyl cyclase proteins, Adhesion proteins, Apoptosis proteins, Calcium-binding proteins, Cell Cycle Regulation proteins, Channel proteins, Cell surface proteins, Cellular metabolism proteins, Chaperone proteins, Cytokine proteins, Cytoskeleton proteins, DNA binding proteins, DNA repair proteins, Endoplasmic reticulum proteins, Extracellular Matrix proteins, G proteins regulatory proteins, GTP activating proteins, Guanine nucleotide exchange factor proteins, Hydrolase proteins, Inhibitor proteins, Kinases (Serine/ Threonine, dual specificity, Tyrosine *etc*.), Ligase proteins, Lipid binding proteins, Lyase proteins, Methyltransferase proteins, Mitochondrial proteins, Motor proteins, Oxidoreductase proteins, Phosphatases, Phospholipases, Proteases, Receptor proteins, and RNA binding proteins. Each of these distinct protein groups is considered a preferred subset of Carcinoma-related signal transduction protein phosphorylation sites disclosed herein, and reagents for their detection/quantification may be considered a preferred subset of reagents provided by the invention.

Particularly preferred subsets of the phosphorylation sites (and their corresponding proteins) disclosed herein are those occurring on the following protein types/groups listed in Column C of Table 1/Figure 2: 1) Kinases (including Serine/Threonine dual specificity, and Tyrosine kinases), 2) Adaptor/Scaffold proteins, 3) Phosphatases, 4) G protein regulators, Guanine Nucleotide Exchange factors, GTPase activating proteins, 5) Cytoskeleton proteins, 6) DNA binding proteins, 7) Phospholipase proteins, 8) Receptor proteins, 9) Enzymes, 10) DNA repair/replication proteins, 11) Adhesion proteins, and 12) Proteases. Accordingly, among preferred subsets of reagents provided by the invention are isolated antibodies and AQUA peptides useful for the detection and/or quantification of the foregoing preferred protein/phosphorylation site subsets.

In one subset of preferred embodiments there is provided:
(i) An isolated phosphorylation site-specific antibody that specifically binds a Kinase selected from Column A, Rows 296-365, of Table 1 only when phosphorylated at the tyrosine listed in corresponding Column D, Rows 296-365, of Table 1, comprised within the phosphorylatable peptide sequence listed in corresponding Column E, Rows 296-365, of Table 1 (SEQ ID NOs: 295-317, 319-333, 335-344, 346-347, 349, 351-355, and 357-364), wherein said antibody does not bind said protein when not phosphorylated at said tyrosine.
(ii) An equivalent antibody to (i) above that only binds the Kinase when not phosphorylated at the disclosed site (and does not bind the protein when it is phosphorylated at the site).
(iii) A heavy-isotope labeled peptide (AQUA peptide) for the quantification of a Carcinoma-related signaling protein that is a Kinase selected from Column A, Rows 296-365, said labeled peptide comprising the phosphorylatable peptide sequence listed in corresponding Column E, Rows 296-365, of Table 1 (SEQ ID NOs: 295-317, 319-333, 335-344, 346-347, 349, 351-355, and 357-364), which sequence comprises the phosphorylatable tyrosine listed in corresponding Column D, Rows 296-365, of Table 1.

Among this preferred subset of reagents, antibodies and AQUA peptides for the detection/quantification of the following Kinase phosphorylation sites are particularly preferred: PIK3C2B (Y127), RIPK5 (Y312), CDC2L5 (Y716), PRKCI (Y388), RPS6KA5 (Y423), FER (Y402), JAK3 (Y929), ZAP70 (Y451), DDR1 (Y755), ERBB2 (Y975), FGFR1 (Y397), FLT1 (Y1053), ROR1 (Y836), ROS1 (Y2110), (see SEQ ID NOs: 302, 309, 313, 324, 326, 338, 340, 343, 347, 352, 359, 360, 362, and 363).

In one subset of preferred embodiments, there is provided:
(i) An isolated phosphorylation site-specific antibody that specifically binds an Adaptor/Scaffold protein selected from Column A, Rows 26-85, of Table 1 only when phosphorylated at the tyrosine listed in corresponding Column D, Rows 26-85, of Table 1, comprised within the phosphorylatable peptide sequence listed in corresponding Column E, Rows 26-85, of Table 1 (SEQ ID NOs: 25-35, 38-44, 46-49, 51-61, 63-67, 69-80, and 83-84), wherein said antibody does not bind said protein when not phosphorylated at said tyrosine.
(ii) An equivalent antibody to (i) above that only binds the Adaptor/Scaffold protein when not phosphorylated at the disclosed site (and does not bind the protein when it is phosphorylated at the site).
(iii) A heavy-isotope labeled peptide (AQUA peptide) for the quantification of a Carcinoma-related signaling protein that is an Adaptor/Scaffold protein selected from Column A, Rows 26-85, said labeled peptide comprising the phosphorylatable peptide sequence listed in corresponding Column E, Rows 26-85, of Table 1 (SEQ ID NOs: 25-35, 38-44, 46-49, 51-61, 63-67, 69-80, and 83-84), which sequence comprises the phosphorylatable tyrosine listed in corresponding Column D, Rows 26-85, of Table 1.

Among this preferred subset of reagents, antibodies and AQUA peptides for the detection/quantification of the following Adaptor/Scaffold protein phosphorylation sites are particularly preferred: CRK (Y136), FLOT1 (Y203), GAB2 (Y371), SPRY1 (Y53), (see SEQ ID NOs: 43, 49, 51, and 74).

In a another subset of preferred embodiments there is provided:
(i) An isolated phosphorylation site-specific antibody that specifically binds a Phosphatase protein selected from Column A, Rows 408-419, 442, and 443, of Table 1 only when phosphorylated at the tyrosine listed in corresponding Column D, Rows 408-419, 442, and 443, of Table 1, comprised within the phosphorylatable peptide sequence listed in corresponding Column E, Rows 408-419, 442, and 443, of Table 1 (SEQ ID NOs: 407-418, 441, and 442), wherein said antibody does not bind said protein when not phosphorylated at said tyrosine.
(ii) An equivalent antibody to (i) above that only binds the Phosphatase protein when not phosphorylated at the disclosed site (and does not bind the protein when it is phosphorylated at the site).
(iii) A heavy-isotope labeled peptide (AQUA peptide) for the quantification of a Carcinoma-related signaling protein that is a Phosphatase protein selected from Column A, Rows 408-419, 442, and 443, said labeled peptide comprising the phosphorylatable peptide sequence listed in corresponding Column E, Rows 408-419, 442, and 443, of Table 1 (SEQ ID NOs: 407-418, 441, and 442), which sequence comprises the phosphorylatable tyrosine listed in corresponding Column D, Rows 408-419, 442, and 443, of Table 1.

Among this preferred subset of reagents, antibodies and AQUA peptides for the detection/quantification of the following Phosphatase protein phosphorylation sites are particularly preferred: INPP5D (Y40), PPP1R14B (Y29), (see SEQ ID NOs: 413 and 442).

In still another subset of preferred embodiments, there is provided:
(i) An isolated phosphorylation site-specific antibody that specifically binds a G protein regulator, guanine nucleotide exchange factors, GTPase activating proteins selected from Column A, Rows 270-283, of Table 1 only when phosphorylated at the tyrosine listed in corresponding Column D, Rows 270-283, of Table 1, comprised within the phosphorylatable peptide sequence listed in corresponding Column E, Rows 270-283, of Table 1 (SEQ ID NOs: 269-282), wherein said antibody does not bind said protein when not phosphorylated at said tyrosine.
(ii) An equivalent antibody to (i) above that only binds the G protein regulator, guanine nucleotide exchange factors, or GTPase activating proteins when *not* phosphorylated at the disclosed site (and does not bind the protein when it is phosphorylated at the site).
(iii) A heavy-isotope labeled peptide (AQUA peptide) for the quantification of a Carcinoma-related signaling protein that is a G protein regulator, guanine nucleotide exchange factors, or GTPase activating proteins selected from Column A, Rows 270-283, said labeled peptide comprising the phosphorylatable peptide sequence listed in corresponding Column E, Rows 270-283, of Table 1 (SEQ ID NOs: 269-282), which sequence comprises the phosphorylatable tyrosine listed in corresponding Column D, Rows 270-283, of Table 1.

Among this preferred subset of reagents, antibodies and AQUA peptides for the detection/quantification of the following G protein regulator, guanine nucleotide exchange factors, or GTPase activating proteins phosphorylation sites are particularly preferred: RAN (Y155) and RASA3 (Y757) (see SEQ ID NOs: 273 and 277).

In still another subset of preferred embodiments there is provided:
(i) An isolated phosphorylation site-specific antibody that specifically binds a Cytoskeletal protein selected from Column A, Rows 173-222, of Table 1 only when phosphorylated at the tyrosine listed in corresponding Column D, Rows 173-222, of Table 1, comprised within the phosphorylatable peptide sequence listed in corresponding Column E, Rows 173-222, of Table 1 (SEQ ID NOs: 172-188, 191-210, 212-219, and 221), wherein said antibody does not bind said protein when not phosphorylated at said tyrosine.
(ii) An equivalent antibody to (i) above that only binds the Cytoskeletal protein when not phosphorylated at the disclosed site (and does not bind the protein when it is phosphorylated at the site).
(iii) A heavy-isotope labeled peptide (AQUA peptide) for the quantification of a Carcinoma-related signaling protein that is a Cytoskeletal protein selected from Column A, Rows 173-222, said labeled peptide comprising the phosphorylatable peptide sequence listed in corresponding Column E, Rows 173-222, of Table 1 (SEQ ID NOs: 172-188, 191-210, 212-219, and 221), which sequence comprises the phosphorylatable tyrosine listed in corresponding Column D, Rows 173-222, of Table 1.

Among this preferred subset of reagents, antibodies and AQUA peptides for the detection/quantification of the following Cellular metabolism enzyme phosphorylation sites are particularly preferred: PLEC1 (Y4505), VIM (Y38) (see SEQ ID NOs: 215 and 218).

In still another subset of preferred embodiments there is provided:
(i) An isolated phosphorylation site-specific antibody that specifically binds a DNA binding protein selected from Column A, Rows 223-231, of Table 1 only when phosphorylated at the tyrosine listed in corresponding Column D, Rows 223-231, of Table 1, comprised within the phosphorylatable peptide sequence listed in corresponding Column E, Rows 223-231, of Table 1 (SEQ ID NOs: 222-230), wherein said antibody does not bind said protein when not phosphorylated at said tyrosine.
(ii) An equivalent antibody to (i) above that only binds the DNA binding protein when not phosphorylated at the disclosed site (and does not bind the protein when it is phosphorylated at the site).
(iii) A heavy-isotope labeled peptide (AQUA peptide) for the quantification of a Carcinoma-related signaling protein that is a DNA binding protein selected from Column A, Rows 223-231, said labeled peptide comprising the phosphorylatable peptide sequence listed in corresponding Column E, Rows 223-231, of Table 1 (SEQ ID NOs: 222-230), which sequence comprises the phosphorylatable tyrosine listed in corresponding Column D, Rows 223-231, of Table 1.

In still another subset of preferred embodiments there is provided:
(i) An isolated phosphorylation site-specific antibody that specifically binds a Phospholipase protein selected from Column A, Rows 420-422, of Table 1 only when phosphorylated at the tyrosine listed in corresponding Column D, Rows 420-422, of Table 1, comprised within the phosphorylatable peptide sequence listed in corresponding Column E, Rows 420-422 of Table 1 (SEQ ID NOs: 419-421), wherein said antibody does not bind said protein when not phosphorylated at said tyrosine.
(ii) An equivalent antibody to (i) above that only binds the Phospholipase protein when *not* phosphorylated at the disclosed site (and does not bind the protein when it is phosphorylated at the site).
(iii) A heavy-isotope labeled peptide (AQUA peptide) for the quantification of a Carcinoma-related signaling protein that is a Phospholipase protein selected from Column A, Rows 420-422, said labeled peptide comprising the phosphorylatable peptide sequence listed in corresponding Column E, Rows 420-422, of Table 1 (SEQ ID NOs: 419-421), which sequence comprises the phosphorylatable tyrosine listed in corresponding Column D, Rows 420-422, of Table 1.

Among this preferred subset of reagents, antibodies and AQUA peptides for the detection/quantification of the following Phospholipase protein phosphorylation sites are particularly preferred: PLCB1 (Y239), PLD1 (Y420), (see SEQ ID NOs: 420 and 421).

In still another subset of preferred embodiments, there is provided:
(i) An isolated phosphorylation site-specific antibody that specifically binds an Receptor protein selected from Column A, Rows 444-459, of Table 1 only when phosphorylated at the tyrosine listed in corresponding Column D, Rows 444-459, of Table 1, comprised within the phosphorylatable peptide sequence listed in corresponding Column E, Rows 444-459, of Table 1 (SEQ ID NOs: 443-458), wherein said antibody does not bind said protein when not phosphorylated at said tyrosine.
(ii) An equivalent antibody to (i) above that only binds the Receptor protein when *not* phosphorylated at the disclosed site (and does not bind the protein when it is phosphorylated at the site).
(iii) A heavy-isotope labeled peptide (AQUA peptide) for the quantification of a Carcinoma-related signaling protein that is an Receptor protein selected from Column A, Rows 443-458, said labeled peptide comprising the phosphorylatable peptide sequence listed in corresponding Column E, Rows 444-459, of Table 1 (SEQ ID NOs: 443-458), which sequence comprises the phosphorylatable tyrosine listed in corresponding Column D, Rows 444-459, of Table 1.

Among this preferred subset of reagents, antibodies and AQUA peptides for the detection/quantification of the following Receptor protein phosphorylation sites are particularly preferred: GPRC5A (Y350 and Y347) (see SEQ ID NOs: 447 and 448).

In yet another subset of preferred embodiments, there is provided:
(i) An isolated phosphorylation site-specific antibody that specifically binds an Enzyme selected from Column A, Rows 243-262, of Table 1 only when phosphorylated at the tyrosine listed in corresponding Column D, Rows 243-262, of Table 1, comprised within the phosphorylatable peptide sequence listed in corresponding Column E, Rows 243-262, of Table 1 (SEQ ID NOs: 242-261), wherein said antibody does not bind said protein when not phosphorylated at said tyrosine.
(ii) An equivalent antibody to (i) above that only binds the Enzyme when not phosphorylated at the disclosed site (and does not bind the protein when it is phosphorylated at the site).
(iii) A heavy-isotope labeled peptide (AQUA peptide) for the quantification of a Carcinoma-related signaling protein that is an Enzyme selected from Column A, Rows 243-262, said labeled peptide comprising the phosphorylatable peptide sequence listed in corresponding Column E, Rows 243-262, of Table 1 (SEQ ID NOs: 242-261), which sequence comprises the phosphorylatable tyrosine listed in corresponding Column D, Rows 243-262, of Table 1.

Among this preferred subset of reagents, antibodies and AQUA peptides for the detection/quantification of the following Enzyme phosphorylation sites are particularly preferred: COX11 (Y111), (see SEQ ID NO: 246).

In yet another subset of preferred embodiments, there is provided:
(i) An isolated phosphorylation site-specific antibody specifically binds a DNA repair/DNA replication protein selected from Column A, Rows 232-239, of Table 1 only when phosphorylated at the tyrosine listed in corresponding to Column D, Rows 232-239, of Table 1, comprised within the phosphorylatable peptide sequence listed in corresponding Column E, Rows 232-239, of Table 1 (SEQ ID NOs: 231-238), wherein said antibody does not bind said protein when not phosphorylated at said tyrosine.
(ii) An equivalent antibody to (i) above that only binds the DNA repair/DNA replication protein when not phosphorylated at the disclosed site (and does not bind the protein when it is phosphorylated at the site).
(iii) A heavy-isotope labeled peptide (AQUA peptide) for the quantification of a Carcinoma-related signaling protein that is a DNA repair/DNA replication protein selected from Column A, Rows 232-239, said labeled peptide comprising the phosphorylatable peptide sequence listed in corresponding Column E, Rows 232-239, of Table 1 (SEQ ID NOs: 231-238), which sequence comprises the phosphorylatable tyrosine listed in corresponding Column D, Rows 232-239, of Table 1.

Among this preferred subset of reagents, antibodies and AQUA peptides for the detection/quantification of the following DNA repair/DNA replication protein phosphorylation sites are particularly preferred: PARP1 (Y176), ATRX (Y1667) (see SEQ ID NOs: 231 and 236).

In yet another subset of preferred embodiments, there is provided:
(i) An isolated phosphorylation site-specific antibody that specifically binds a Adhesion protein selected from Column A, Rows 89-137, of Table 1 only when phosphorylated at the tyrosine listed in corresponding Column D, Rows 89-137, of Table 1, comprised within the phosphorylatable peptide sequence listed in corresponding Column E, Rows 89-137, of Table 1 (SEQ ID NOs: 88-129, 131, and 133-136), wherein said antibody does not bind said protein when not phosphorylated at said tyrosine.
(ii) An equivalent antibody to (i) above that only binds the Adhesion protein when not phosphorylated at the disclosed site (and does not bind the protein when it is phosphorylated at the site).
(iii) A heavy-isotope labeled peptide (AQUA peptide) for the quantification of a Carcinoma-related signaling protein that is a Adhesion protein selected from Column A, Rows 89-137, said labeled peptide comprising the phosphorylatable peptide sequence listed in corresponding Column E, Rows 89-137, of Table 1 (SEQ ID NOs: 88-129, 131, and 133-136), which sequence comprises the phosphorylatable tyrosine listed in corresponding Column D, Rows 89-137, of Table 1.

Among this preferred subset of reagents, antibodies and AQUA peptides for the detection/quantification of the following Adhesion protein phosphorylation sites are particularly preferred: ADAM23 (Y375), ADAM9 (Y769), VCL (Y692) (see SEQ ID NOs: 88, 89, and 131).

In still another subset of preferred embodiments, there is provided:
(i) An isolated phosphorylation site-specific antibody that specifically binds a Protease protein selected from Column A, Rows 423-441, of Table 1 only when phosphorylated at the tyrosine listed in corresponding Column D, Rows 423-441, of Table 1, comprised within the phosphorylatable peptide sequence listed in corresponding Column E, Rows 423-441, of Table 1 (SEQ ID NOs: 422-425, and 427-440), wherein said antibody does not bind said protein when not phosphorylated at said tyrosine.
(ii) An equivalent antibody to (i) above that only binds the Protease protein when not phosphorylated at the disclosed site (and does not bind the protein when it is phosphorylated at the site).
(iii) A heavy-isotope labeled peptide (AQUA peptide) for the quantification of a Carcinoma-related signaling protein that is a Protease protein selected from Column A, Rows 423-441, said labeled peptide comprising the phosphorylatable peptide sequence listed in corresponding Column E, Rows 423-441, of Table 1 (SEQ ID NOs: 422-425, and 427-440), which sequence comprises the phosphorylatable tyrosine listed in corresponding Column D, Rows 423-441, of Table 1.

In still another subset of preferred embodiments, there is provided:
(i) An isolated phosphorylation site-specific antibody that specifically binds a protein selected from Column A, Rows 16, 19, and 291, of Table 1 only when phosphorylated at the tyrosine listed in corresponding Column D, Rows 16, 19, and 291, of Table 1, comprised within the phosphorylatable peptide sequence listed in corresponding Column E, Rows 16, 19, and 291, of Table 1 (SEQ ID NOs: 15, 18, and 290), wherein said antibody does not bind said protein when not phosphorylated at said tyrosine.
(ii) An equivalent antibody to (i) above that only binds the protein when not phosphorylated at the disclosed site (and does not bind the protein when it is phosphorylated at the site).
(iii) A heavy-isotope labeled peptide (AQUA peptide) for the quantification of a Carcinoma-related signaling protein that is a protein selected from Column A, Rows 16, 19, and 291, said labeled peptide comprising the phosphorylatable peptide sequence listed in corresponding Column E, Rows 16, 19, and 291, of Table 1 (SEQ ID NOs: 15, 18, and 290), which sequence comprises the phosphorylatable tyrosine listed in corresponding Column D, Rows 16, 19, and 291, of Table 1.

The invention also provides, in part, an immortalized cell line producing an antibody of the invention, for example, a cell line producing an antibody within any of the foregoing preferred subsets of antibodies. In one preferred embodiment, the immortalized cell line is a rabbit hybridoma or a mouse hybridoma.

In certain other preferred embodiments, a heavy-isotope labeled peptide (AQUA peptide) of the invention (for example, an AQUA peptide within any of the foregoing preferred subsets of AQUA peptides) comprises a disclosed site sequence wherein the phosphorylatable tyrosine is phosphorylated. In certain other preferred embodiments, a heavy-isotope labeled peptide of the invention comprises a disclosed site sequence wherein the phosphorylatable tyrosine is *not* phosphorylated.

The foregoing subsets of preferred reagents of the invention should not be construed as limiting the scope of the invention, which, as noted above, includes reagents for the detection and/or quantification of disclosed phosphorylation sites on any of the other protein type/group subsets (each a preferred subset) listed in Column C of Table 1/Figure 2.

Also provided by the invention are methods for detecting or quantifying a Carcinoma-related signaling protein that is tyrosine phosphorylated, said method comprising the step of utilizing one or more of the above-described reagents of the invention to detect or quantify one or more Carcinoma-related signaling protein(s) selected from Column A of Table 1 only when phosphorylated at the tyrosine listed in corresponding Column D of Table 1. In certain preferred embodiments of the methods of the invention, the reagents comprise a subset of preferred reagents as described above.

Also provided by the invention is a method for obtaining a phosphorylation profile of protein kinases that are phosphorylated in Carcinoma signaling pathways, said method comprising the step of utilizing one or more isolated antibody that specifically binds a protein kinase selected from Column A, Rows 296-365, of Table 1 onto when phosphorylated at the tyrosine listed in corresponding Column D, Rows296-365, of Table 1, comprised within the phosphorylation site sequence listed in corresponding Column E, Rows 296-365, of Table 1 (SEQ ID NOs: 295-317, 319-333, 335-344, 346-347, 349, 351-355, and 357-364), to detect the phosphorylation of one or more of said protein kinases, thereby obtaining a phosphorylation profile for said kinases.

The identification of the disclosed nearly 474 novel Carcinoma-related signaling protein phosphorylation sites, and the standard production and use of the reagents provided by the invention are described in further detail below and in the Examples that follow.

All cited references are hereby incorporated herein, in their entirety, by reference. The Examples are provided to further illustrate the invention, and do not in any way limit its scope, except as provided in the claims appended hereto.

**Table 1. Newly Discovered Carcinoma-Related Signaling Protein Phosphorylation Sites.**

| | **Column A** | **Column B** | **Column C** | **Column D** | **Column E** | **Column H** |
|---|---|---|---|---|---|---|
| 1 | **Protein Name** | **Accession No.** | **Protein Type** | **Phospho-Residue** | **Phosphorylation Site Sequence** | **SEQ ID NO:** |
| 2 | ARD1A | NP_003482.1 | Acetyltransferase | Y145 | YYADGEDAyAMKR | SEQ ID NO: 1 |
| 3 | CHAT | NP_065574.1 | Acetyltransferase | Y413 | ALQLLHGGGySKNGANRWYDK | SEQ ID NO: 2 |
| 4 | ANLN | | Actin binding protein | Y671 | SEDRDLLySIDAYRS | SEQ ID NO: 3 |
| 5 | BAIAP2 | | Actin binding protein | Y337 | LSDSySNTLPVR | SEQ ID NO: 4 |
| 6 | BAIAP2 | NP_006331.1 | Actin binding protein | Y310 | MSAQESTPIMNGVTGPDGEDySPWADRK | SEQ ID NO: 5 |
| 7 | BAIAP2 | NP_006331.1 | Actin binding protein | Y353 | NSyATTENKTLPR | SEQ ID NO: 6 |
| 8 | BAIAP2 | | Actin binding protein | Y491 | QRPySVAVPAFSQGLDDYGAR | SEQ ID NO: 7 |
| 9 | BAIAP2 | | Actin binding protein | Y505 | QRPYSVAVPAFSQGLDDyGAR | SEQ ID NO: 8 |
| 10 | BAIAP2 | NP_006331.1 | Actin binding protein | Y164 | YSDKELQyIDAISNK | SEQ 10 NO: 9 |
| 11 | CAPZB | NP_004921.1 | Actin binding protein | Y232 | STLNElyFGK | SEQ ID NO: 10 |
| 12 | CTNNA1 | NP_001894.2 | Actin binding protein | Y177 | NAGNEQDLGiQyK | SEQ ID NO: 11 |
| 13 | CTNNA1 | | Actin binding protein | Y177 | NAGNEQDLGNQyK | SEQ ID NO: 12 |
| 14 | CTNND1 | NP_001322.1 | Actin binding protein | Y193 | DFRKNGNGGPGPyVGQAGTATLPR | SEQ ID NO: 13 |
| 15 | CTNND1 | NP_001322.1 | Actin binding protein | Y600 | EIPQAERyQEAAPNVANNTGPHAASCFGAK | SEQ ID NO: 14 |
| 16 | CTNND1 | AAC39803.1 | Actin binding protein | Y581 | SLDNNySTPNER | SEQ ID NO: 15 |
| 17 | CTNND1 | NP_001322.1 | Actin binding protein | Y859 | SQSSHSyDDSTLPLIDR | SEQ ID NO: 16 |
| 18 | DBN1 | Nip_004386.2 | Actin binding protein | Y163 | LREDENAEPVGTTyQK | SEQ ID NO: 17 |
| 19 | FLNA | NP_001447.1 | Actin binding protein | Y1604 | KTHIQDNHDGTyTVAYVPDVTGR | SEQ ID NO: 18 |
| 20 | FLNA | NP_001447.1 | Actin binding protein | Y2388 | VHSPSGALEECYVTEIDQDKyAVR | SEQ ID NO: 19 |
| 21 | NEBL | NP_006384.1 | Actin binding protein | Y102 | ADLSNSLyKRMPATIDSVFAGEVTQLQSE VAYKQK | SEQ ID NO: 20 |
| 22 | NEBL | NP_006384.1 | Actin binding protein | Y126 | ADLSNSLYKRMPATIDSVFAGEVTQLQSE VAyKQK | SEQ ID NO: 21 |
| 23 | WDR1 | NP_005103.2 | Actin binding protein | Y74 | FSPDGNRFATASADGQIyIYDGK | SEQ ID NO: 22 |
| 24 | WDR1 | NP_005103.2 | Actin binding protein | Y76 | FSPDGNRFATASADGQIYIyDGK | SEQ ID NO: 23 |
| 25 | WDR1 | NP_059830.1 | Actin binding protein | Y72 | YAPSGFyIASGDVSGK | SEQ ID NO: 24 |
| 26 | AFAP | NP_067651.2 | Adaptor/scaffold | Y353 | KKPSTDEQTSSAEEDVPTCGyLNVLSNSR | SEQ ID NO: 25 |
| 27 | AHNAK | NP_001611.1 | Adaptor/scaffold | Y61 | EGDQIVGATIyFDNLQSGEVTQLLNTMGH HTVGLK | SEQ ID NO: 26 |
| 28 | AKAP2 | NP_001004065.2 | Adaptor/scaffold | Y773 | EGSYFSKySEAAELR | SEQ ID NO: 27 |
| 29 | AKAP2 | NP_001004065.2 | Adaptor/scaffold | Y911 | ETRPEGSyFSKYSEA | SEQ ID NO: 28 |
| 30 | ALS2CR19 | NP_689739.3 | Adaptor/scaffold | Y939 | DGHPLSPERDHLEGLyAK | SEQ ID NO: 29 |
| 31 | AMOTL1. | NP_570899.1 | Adaptor/scaffold | Y218 | GQQQQQQQQGAVGHGyYMAGGTSQK | SEQ ID NO: 30 |
| 32 | ANKS1 | NP_056060.1 | Adaptor/scaffold | Y455 | EEDEHPyELLLTAETK | SEQ ID NO: 31 |
| 33 | ARRB1 | NP_004032.2 | Adaptor/scaffold | Y54 | ERRVyVTLTCAFR | SEQ ID NO: 32 |
| 34 | ASB6 | NP_060343.1 | Adaptor/scaffold | Y65 | ILVLTELLERKAHSPFyQEGVSNALLKMAE LGLTR | SEQ ID NO: 33 |
| 35 | AXIN2 | NP_004646.2 | Adaptor/scaffold | Y477 | YSPRSRSPDHHHHHHSQY*HSLLPPGGK | SEQ ID NO: 34 |
| 36 | BCAR1 | NP_055382.2 | Adaptor/scaffold | Y262 | RGLLPSQyGQEVYDT | SEQ ID NO: 35 |
| 37 | BCAR1 | | Adaptor/scaffold | Y372 | TPLVLAAPPPDSPPAEDVYDVPPPAPDLy DVPPGLR | SEQ ID NO: 36 |
| 38 | BCAR1 | | Adaptor/scaffold | Y362 | TPLVLAAPPPDSPPAEDVyDVPPPAPDLY DVPPGLR | SEQ ID NO: 37 |
| 39 | C20orf32 | NP_065089.2 | Adaptor/scaffold | Y329 | GTFPLDEDVSyKVPSSFLIPR | SEQ ID NO: 38 |
| 40 | C20orf32 | NP_065089.2 | Adaptor/scaffold | Y244 | SEWlyDTPVSPGK | SEQ ID NO: 39 |
| 41 | C20orf32 | NP_065089.2 | Adaptor/scaffold | Y131 | SWAEGPQPPTAQVyEFPDPPTSAR | SEQ ID NO: 40 |
| 42 | C20orf32 | NP_065089,2 | Adaptor/scaffold | Y350 | VEQQNTKPNIyDIPK | SEQ ID NO: 41 |
| 43 | CAV1 | NP_001744.2 | Adaptor/scaffold | Y42 | ELSEKQVyDAHTKEI | SEQ ID NO: 42 |
| 44 | CRK | NP_005197.3 | Adaptor/scaffold | Y136 | QGSGVILRQEEAEyVR | SEQ ID NO: 43 |
| 45 | EPS8 | NP_004438.3 | Adaptor/scaffold | Y525 | HIDRNyEPLK | SEQ ID NO: 44 |
| 46 | EPS8 | | Adaptor/scaffold | Y491 | LSTEHSSVSEYHPADGyAFSSNIYTR | SEQ ID NO: 45 |
| 47 | EPS8 | NP_004438.3 | Adaptor/scaffold | Y485 | LSTEHSSVSEyHPADGYAFSSNIYTR | SEQ ID NO: 46 |
| 48 | EPS8 | NP_004438.3 | Adaptor/scaffold | Y774 | VySQITVQK | SEQ ID NO: 47 |
| 49 | FLOT1 | NP_005794.1 | Adaptor/scaffold | Y238 | AQADLAyQLQVAK | SEQ ID NO: 48 |
| 50 | FLOT1 | NP_005794.1 | Adaptor/scaffold | Y203 | VSAQyLSEIEMAK | SEQ ID NO: 49 |
| 51 | G3BP2 | | Adaptor/scaffold | Y175 | QENANSGyYEAHPVT | SEQ ID NO: 50 |
| 52 | GAB2 | NP_036428.1 | Adaptor/scaffold | Y371 | ASSCETyEYPQR | SEQ ID NO: 51 |
| 53 | GAB3 | NP_542179.1 | Adaptor/scaffold | Y560 | SEEQRVDyVQVDEQK | SEQ ID NO: 52 |
| 54 | LRRC17 | NP_005815.1 | Adaptor/scaffoid | Y59 | RGSNPVKRYAPGLPCDVYTyLHEK | SEQ ID NO: 53 |
| 55 | MALT1 | NP_006776.1 | Adaptor/scaffold | Y188 | MNKEIPNGNTSELIFNAVHVKDAGFyVCR | SEQ ID NO: 54 |
| 56 | NRAP | NP_932326.2 | Adaptor/scaffold | Y408 | KFTSDNKyKENYQNH | SEQ ID NO: 55 |
| 57 | NRAP | NP_932326.2 | Adaptor/scaffold | Y420 | QNHMRGRyEGVGMDR | SEQ ID NO: 56 |
| 58 | PARD3 | NP_062565.2 | Adaptor/scaffold | Y1127 | EGHMMDALyAQVK | SEQ ID NO: 57 |
| 59 | PARD3 | NP_062565.2 | Adaptor/scaffold | Y1244 | KNASSVSQDSWEQNySPGEGFQSAK | SEQ ID NO: 58 |
| 60 | PDZK1 | NP_002605.2 | Adaptor/scaffold | Y92 | KSGNSVTLLVLDGDSyEKAVK | SEQ ID NO: 59 |
| 61 | PDZK1IP1 | NP_005755.1 | Adaptor/scaffold | Y99 | SSEHENAyENVPEEEGK | SEQ ID NO: 60 |
| 62 | PPP1R9A | NP_060120.2 | Adaptor/scaffold | Y159+ | SVHESGQNNRySPKKEKAGGSEPQDEW GGSK | SEQ ID NO: 61 |
| 63 | SCAP2 | | Adaptor/scaffold | Y197 | IyQFTAASPK | SEQ ID NO: 62 |
| 64 | SCAP2 | NP_003921.2 | Adaptor/scaffold | Y151 | LSKNFYyYGSDKDK | SEQ ID NO: 63 |
| 65 | SH2D3A | NP_005481.1 | Adaptor/scaffold | Y231 | TPSFELPDASERPPTyCELVPR | SEQ ID NO: 64 |
| 66 | SH3MD1 | NP_055446.2 | Adaptor/scaffold | Y530 | LKYEEPEYDIPAFGF | SEQ ID NO: 65 |
| 67 | SH3MD2 | NP_065921.2 | Adaptor/scaffold | Y253 | IGIFPISyVEFNSAAKQLIEWDK | SEQ ID NO: 66 |
| 68 | SHB | NP_003019.2 | Adaptor/scaffold | Y384 | GIQLyDTPYEPEGQSVDSDSESTVSPR | SEQ ID NO: 67 |
| 69 | SHB | | Adaptor/scaffold | Y201 | LDyCGGSGEPGGVQR | SEQ ID NO: 68 |
| 70 | SHC3 | NP_058544.2 | Adaptor/scaffold | Y269 | QIIANHHMRSISFASGGDPDTTDYVAyVTK | SEQ ID NO: 69 |
| 71 | SHC3 | NP_058544.2 | Adaptor/scaffold | Y266 | QIIANHHMRSISFASGGDPDTTDyVAYVTK | SEQ ID NO: 70 |
| 72 | SLAC2-B | NP_055880.1 | Adaptor/scaffold | Y295 | SPRTSTlyDMYRTRE | SEQ ID NO: 71 |
| 73 | SLAC2-B | NP_055880.1 | Adaptor/scaffold | Y298 | TSTIYDMyRTREPRV | SEQ ID NO: 72 |
| 74 | SOCS7 | NP_055413.1 | Adaptor/scaffold | Y561 | YDPQEEVyLSLKEAQ | SEQ ID NO: 73 |
| 75 | SPRY1 | NP_005832.1 | Adaptor/scaffold | Y53 | GSNEyTEGPSVVK | SEQ ID NO: 74 |
| 76 | TJP1 | NP_003248.2 | Adaptor/scaffold | Y1346 | DIVRSNHyDPEEDEE | SEQ ID NO: 75 |
| 77 | TJP1 | NP_003248.2 | Adaptor/scaffold | Y1059 | DLEQPTyRYESSSYTDQFSR | SEQ ID NO: 76 |
| 78 | TJP2 | NP_004808.2 | Adaptor/scaffold | Y261 | AYDPDyER | SEQ ID NO: 77 |
| 79 | TJP2 | NP_004808.2 | Adaptor/scaffold | Y265 | AYDPDYERAySPEYRR | SEQ ID NO: 78 |
| 80 | TNS1 | NP_072174.3 | Adaptor/scaffold | Y796 | SYSPyDYQPCLAGPNQDFHSK | SEQ ID NO: 79 |
| 81 | TPR | NP_003283.1 | Adaptor/scaffold | Y54 | FKVESEQQyFEIEKR | SEQ ID NO: 80 |
| 82 | TRAF4 | | Adaptor/sraffold | Y204 | YCTKEFVFDTIQSHQ | SEQ ID NO: 81 |
| 83 | TRIP6 | | Adaptor/scaffold | Y55 | VNFCPLPSEQCyQAPGGPEDR | SEQ ID NO: 82 |
| 84 | WASL | NP_003932.3 | Adaptor/scaffold | Y175 | FyGPQVNNISHTK | SEQ ID NO: 83 |
| 85 | WDR45L | NP_062559.1 | Adaptor/scaffold | Y19 | yPPNKVM1WDDLKKKTVIEIEFSTEVK | SEQ ID NO: 84 |
| 86 | CBLB | NP_733762.2 | Adaptor/scaffold, Calcium-binding protein | Y665 | VFSNGHLGSEEyDVPPR | SEQ ID NO: 85 |
| 87 | SPTAN1 | NP_003118.1 | Adaptor/scaffold; Cytoskeletal protein | Y2167 | VASNPyTWFTMEALEETWRNLQK | SEQ ID NO: 86 |
| 88 | ADCY4 | NP_640340.2 | Adenylyl cyclase | Y444 | ELGEPTyLVIDPRAEEEDEKGTAGGLLSSL EGLKMR - | SEQ ID NO: 87 |
| 89 | ADAM23 | NP_003803,1 | Adhesion | Y375 | MLHEFSKyRQRIKQH | SEQ ID NO: 88 |
| 90 | ADAM9 | NP_003807.1 | Adhesion | Y769 | HVSPVTPPREVPIyANR | SEQ ID NO: 89 |
| 91 | ADAM9 | NP_003807,1 | Adhesion | Y736 | KRSQTyESDGKNQANPSR | SEQ ID NO: 90 |
| 92 | ANTXR1 | NP_115584,1 | Adhesion | Y425 | VKMPEQEyEFPEPR | SEQ ID NO: 91 |
| 93 | CDH6 | NP_004923.1 | Adhesion | Y17 | TYRYFLLLFWVGQPyPTLSTPLSK | SEQ ID NO: 92 |
| 94 | CH13L1 | NP_001267.1 | Adhesion | Y189 | VTIDSSyDIAK | SEQ ID NO: 93 |
| 95 | CLDN18 | NP_001002026.1 | Adhesion | Y260 | TEDEVQSYPSKHDyV | SEQ ID NO: 94 |
| 96 | CLDN2 | NP_065917.1 | Adhesion | Y194 | SNyYDAYQAQPLATR | SEQ ID NO: 95 |
| 97 | CLDN7 | NP_001298.2 | Adhesion | Y210 | SYPKSNSSKEyV | SEQ ID NO: 96 |
| 98 | CYFIP2 | NP_055199.2 | Adhesion | Y108 | CNEQPNRVElyEK | SEQ ID NO: 97 |
| 99 | CYFIP2 | NP_055191.2 | Adhesion | Y325 | FFKQLQVVPLFGDMQIELARYIKTSAHyEE NK | SEQ ID NO: 98 |
| 100 | ERBB21P | NP_061165.1 | Adhesion | Y1252 | EQLIDyLMLK | SEQ ID NO: 99 |
| 101 | ERBB21P | NP_061165,1 | Adhesion | Y1229 | MPLSNGQMGQPLRPQANySQIHHPPQAS VAR | SEQ ID N0:100 |
| 102 | ERBB21P | NP_061165.1 | Adhesion | Y1263 | VAHQPPyTQPHCSPR | SEQ ID NO: 101 |
| 103 | ERBB21P | NP_001006600.1 | Adhesion | Y483 | yPTPYPDELKNMVK | SEQ ID NO: 102 |
| 104 | ERBB21P | NP_001006600,1 | Adhesion | Y487 | YPTPyPDELKNMVK | SEQ ID NO: 103 |
| 105 | ITGA3 | NP_002195.1 | Adhesion | Y1051 | SQPSETERLTDDy | SEQ ID NO: 104 |
| 106 | MUCDHL | NP_068743.2 | Adhesion | Y174 | DDILFYTLQEMTAGASDyFSLVSVNRPALR | SEQ ID NO: 105 |
| 107 | MUCDHL | NP_068743.2 | Adhesion | Y844 | GGGPYDAPGGDDSyI | SEQ ID NO: 106 |
| 108 | MUCDHL | NP_068743,2 | Adhesion | Y835 | GGGPyDAPGGDDSYI | SEQ ID NO: 107 |
| 109 | PKP1 | NP_000290.2 | Adhesion | Y120 | FSSySQMENWSR | SEQ ID NO: 108 |
| 110 | PKP1 | NP_000290.2 | Adhesion | Y71 | GSMyDGLADNYNYGTTSR | SEQ ID NO: 109 |
| 111 | PKP1 | NP_000290.2 | Adhesion | Y78 | GSMYDGLADNyNYGTTSR | SEQ ID NO: 110 |
| 112 | PKP1 | NP_000290.2 | Adhesion | Y214 | QDPVyIPPISCNK | SEQ ID NO: 111 |
| 113 | PKP1 | NP_000290.2 | Adhesion | Y160 | SEPDLyCDPR | SEO ID NO: 112 |
| 114 | PKP1 | NP_000290.2 | Adhesion | Y187 | YSFySTCSGQK | SEQ ID NO: 113 |
| 115 | PKP2 | NP_001005242.1 | Adhesion | Y119 | AGTTATyEGRWGR | SEQ ID NO: 114 |
| 116 | PKP2 | NP_001005242.1 | Adhesion | Y130 | AGTTATYEGRWGRGTAQySSQK | SEQ ID NO: 115 |
| 117 | PKP2 | NP_001005242.1 | Adhesion | Y161 | AHyTHSDYQYSQR | SEQ ID NO: 116 |
| 118 | PKP2 | NP_001005242.1 | Adhesion | Y261 | SMGNLLEKENyLTAGLTVGQVRPLVPLQP VTQNR | SEQ ID NO: 117 |
| 119 | PKP2 | NP_001005242.1 | Adhesion | Y108 | SPVPKTyDMLK | SEQ ID NO: 118 |
| 120 | PKP2 | NP_001005242.1 | Adhesion | Y86 | TSSVPEyVYNLHLVENDFVGGR | SEQ ID NO: 119 |
| 121 | PKP2 | NP_001005242.1 | Adhesion | Y615 | VKEQyQDVPMPEEK | SEQ ID NO: 120 |
| 122 | PKP2 | NP_001005242.1 | Adhesion | Y587 | YSQNIYIQNRNIQTDNNK | SEQ ID NO: 121 |
| 123 | PKP2 | NP_001005242.1 | Adhesion | Y582 | ySQNIYIQNRNIQTDNNK | SEQ ID NO: 122 |
| 124 | PKP2 | NP_001005242.1 | Adhesion | Y88 | TSSVPEYVyNLHLVENDFVGGRSPVPK | SEQ ID NO: 123 |
| 125 | PKP4 | NP_001005476.1 | Adhesion | Y1100 | LYLQSPHSYEDPyFDDR | SEQ ID NO: 124 |
| 126 | PKP4 | NP_001005476.1 | Adhesion | Y443 | SPNHGTVELQGSQTALyR | SEQ ID NO: 125 |
| 127 | PKP4 | NP_001005476.1 | Adhesion | Y261 | TSLGSGFGSPSVTDPRPLNPSAySSTTLP AAR | SEQ ID NO: 126 |
| 128 | PLEKHC1 | NP_006823.1 | Adhesion | Y185 | KLDDQSEDEALELEGPLITPGSGSIYSSPG LySK | SEQ ID NO: 127 |
| 129 | SCARF1 | NP_003684.2 | Adhesion | Y818 | QAEEERQEEPEyENVVPISRPPEP | SEQ ID NO: 128 |
| 130 | SIGLEC7 | NP_055200.1 | Adhesion | Y26 | DySLTMQSSVTVQEGMCVHVR | SEQ ID NO: 129 |
| 131 | TNS1 | | Adhesion | Y1323 | HVAYGGySTPEDR | SEQ ID NO: 130 |
| 132 | VCL | NP_003364.1 | Adhesion | Y692 | ILLRNPGNQAAyEHFETMK | SEQ ID NO: 131 |
| 133 | | | Adhesion | Y776 | RPLNPSAySSTTLPA | SEQ ID NO: 132 |
| 134 | CTNN81 | NP_001895.1 | Adhesion; Actin binding protein | Y716 | TEPMAWNETADLGLDIGAQGEPLGYRQD DPSyR | SEQ ID NO: 133 |
| 135 | DSP | NP_001008844.1 | Adhesion; Cytoskeletal protein | Y28 | AESGPDLRyEVTSGGGGTSR | SEQ ID NO: 134 |
| 136 | DSP | NP_001008844.1 | Adhesion; Cytoskeletal protein | Y172 | GGGGyTCQSGSGWDEFTK | SEQ ID NO: 135 |
| 137 | DSP | NP_001008844.1 | Adhesion; Cytoskeletal protein | Y1116 | ITRLTyEIEDEKRR | SEQ ID NO: 136 |
| 138 | BAG3 | NP_004272.2 | Apoptosis | Y457 | TDKKYLMIEEyLTK | SEQ ID NO:137 |
| 139 | BIRC3 | NP_001156.1 | Apoptosis | Y90 | HKKLyPSCR | SEQ ID NO: 138 |
| 140 | CAT | NP_001743.1 | Apoptosis | Y215 | HMNGyGSHTFKLVNANGFAVYCK | SEQ ID N0:139 |
| 141 | QSCN6L1 | NP_859052.2 | Apoptosis | Y469 | RyVHTFFGCKECGEHFEEMAKESMDSVK | SEQ ID NO: 140 |
| 142 | CASQ1 | NP_001222.2 | Calcium-binding protein | Y51 | NyKNVFK | SEQ ID NO: 141 |
| 143 | S100A11 | NP_005611.1 | Calcium-binding protein | Y30 | DGyNYTLSK | SEQ ID NO: 142 |
| 144 | ANAPC7 | NP_057322.1 | Cell cycle regulation | Y247 | SLLRDNVDLLGSLADLyFRAGDNKNSVLK | SEQ ID NO: 143 |
| 145 | ASPM | NP_060606.2 | Cell cycle regulation | Y2497 | TyITFQTWKHASILIQQHYRTYR | SEQ ID NO: 144 |
| 146 | ASPM | NP_060606.2 | Cell cycle regulation | Y2514 | TYITFQTWKHASILIQQHyRTYR | SEQ ID NO:145 |
| 147 | ASPM | NP_060606.2 | Cell cycle regulation | Y2517 | TYITFQTWKHASILlQQHYRTyR | SEQ ID NO:146 |
| 148 | CSPG6 | NP_005436.1 | Cell cycle regulation; DNA repair | Y668 | GALTGGYyDTR | SEQ ID NO:147 |
| 149 | CD34 | | Cell surface | Y339 | ENGGGQGySSGPGTS | SEQ ID NO: 148 |
| 150 | CD34 | | Cell surface | Y329 | ERLGEDPyYTENGGG | SEQ ID NO: 149 |
| 151 | CD34 | | Cell surface | Y328 | GERLGEDpYYTENGG | SEQ ID NO: 150 |
| 152 | M11S1 | NP_005889.3 | Cell surface | Y545 | QNQYQASyNQSFSSQ | SEQ ID NO: 151 |
| 153 | STEAP1 | NP_036581.1 | Cell surface | Y27 | NLEEDDyLHKDTGETSMLK | SEQ ID NO: 152 |
| 154 | TMED7 | NP_861974.1 | Cell surface | Y50 | QCFyEDIAQGTK | SEQ ID NO: 153 |
| 155 | HCN3 | NP_065948,1 | Channel, cation | Y490 | LTDGSyFGEICLLTRGR | SEQ ID NO: 154 |
| 156 | GABRA6 | NP_000802.1 | Channel, chloride | Y420 | APILQSTPVTPPPLPPAFGGTSKIDQySR | SEQ ID NO:155 |
| 157 | GABRA6 | NP_000802.1 | Channel, chloride | Y368 | KAQFAAPPTVTISKATEPLEAEIVLHPDSKy HLK | SEQ ID NO: 156 |
| 158 | GABRB2 | NP_000804.1 | Channel, chloride | Y396 | NEMATSEAVMGLGDPRSTMLAyDASSIQY RK | SEQ ID NO: 157 |
| 159 | GABRB2 | NP_000804.1 | Channel, chloride | Y403 | NEMATSEAVMGLGDPRSTMLAYDASSIQy RK | SEQ ID NO: 158 |
| 160 | GRIA3 | NP_000819.1 | Channel, ligand-gated | Y386 | MVQVQGMTGNIQFDTYGRRTNYTIDVyEM KVSGSR | SEQ ID NO: 159 |
| 161 | RYR2 | NP_001026.1 | Channel, ligand-gated | Y3405 | MVAEVFlyWSKSHNFKR | SEQ ID NO: 160 |
| 162 | VDAC3 | NP_005653.3 | Channel, misc. | Y62 | IDLKTKSCSGVEFSTSGHAYTDTGKASGN LETKyK | SEQ ID NO: 161 |
| 163 | BCS1L | NP_004319.1 | Chaperone | Y181 | TVMYTAVGSEWRPFGyPR | SEQ ID N0: 162 |
| 164 | CCT4 | NP_006421.2 | Chaperone | Y449 | TLSGMESyCVR | SEQ ID NO: 163 |
| 165 | CDC37 | NP_008996.1 | Chaperone | Y155 | TFVEKyEKQIKHFGMLR | SEQ ID NO: 164 |
| 166 | DNAJA1 | NP_001530.1 | Chaperone | Y119 | NVVHQLSVTLEDLyNGATR | SEQ ID NO: 165 |
| 167 | HSP90BB | NP_001014390.1 | Chaperone | Y239 | IKEKylDQEELNK | SEQ 10 NO: 166 |
| 168 | HSPA9B | NP_004125.3 | Chaperone | Y118 | LVGMPAKRQAVTNPNNTFyATKRLIGRR | SEQ ID NO: 167 |
| 169 | HSPB2 | NP_001532.1 | Chaperone | Y16 | SVPHAHPATAEyEFANPSRLGEQR | SEQ ID NO: 168 |
| 170 | HSPD1 | NP_002147.2 | Chaperone | Y243 | CEFQDAyVLLSEK | SEQ ID NO: 169 |
| 171 | CCL28 | NP_683513.1 | Chemokine | Y127 | RNSNRAHQGKHETYGHKTPy | SEQ ID NO: 170 |
| 172 | IL1F6 | NP_055255.1 | Cytokine | Y96 | DIMDLyNQPEPVK | SEQ ID NO: 171 |
| 173 | ACTA1 | NP_001091.1 | Cytoskeletal protein | Y296 | DLyANNVMSGGTTMYPGIADR | SEQ ID NO: 172 |
| 174 | ACTA1 | NP_001091.1 | Cytoskeletal protein | Y200 | GySFVTTAER | SEQ ID NO: 173 |
| 175 | ACTB | NP_001092.1 | Cytoskeletal protein | Y198 | GySFTTTAER | SEQ ID NO: 174 |
| 176 | ACTR8 | NP_075050.3 | Cytoskeletal protein | Y394 | LGDEKLQAPMALFyPATFGIVGQKMTTLQ HR | SEQ ID NO: 175 |
| 177 | ADD3 | NP_001112.2 | Cytoskeletal protein | Y35 | YFDRINENDPEyIR | SEQ ID NO: 176 |
| 178 | ANK3 | NP_001140.2 | Cytoskeletal protein | Y927 | IHGSGHVEEPASPLAAyQK | SEQ lD NO: 1 77 |
| 179 | ANKRA2 | NP_075526.1 | Cytoskeletal protein | Y164 | HRGNEVSTTPLLANSLSVHQLAAQGEMLy LATR | SEQ ID NO:178 |
| 180 | CLDN1 | NP_066924.1 | Cytoskeletal protein | Y210 | KTTSYPTPRPYPKPAPSSGKDyV | SEQ ID NO: 179 |
| 181 | CLDN3 | NP_001297.1 | Cytoskeletal protein | Y219 | STGPGASLGTGYDRKDyV | SEQ ID NO:180 |
| 182 | COR01A | NP_009005.1 | Cytoskeletal protein | Y25 | HVFGQPAKADQCyEDVR | SEQ ID NO: 181 |
| 183 | CTNND2 | NP_001323.1 | Cytoskeletal protein | Y516 | QLQYCPSVESPySK | SEQ ID NO:182 |
| 184 | CTNND2 | NP_001323.1 | Cytoskeletal protein | Y1197 | STGNyVDFYSAARPYSELNYETSHYPASP DSWV | SEQ ID NO:183 |
| 185 | CTTN | NP_612632.1 | Cytoskeletai protein | Y141 | QSAVGFEyQGKTEKH | SEQ ID NO:184 |
| 186 | CTTN | NP_612632.1 | Cytoskeletal protein | Y396 | SFKAELSyRGPVSGT | SEQ ID NO:185 |
| 187 | CTTN | NP_612632.1 | Cytoskeletal protein | Y427 | SSQQGLAyATEAVYE | SEQ ID NO:186 |
| 188 | CYLC2 | NP_001331.1 | Cytoskeletal protein | Y14 | FQRVNFGPyDNYIPVSELSK | SEQ ID NO:187 |
| 189 | DAG1 | NP_004384.1 | Cytoskeletal protein | Y886 | NMTPyRSPPPYVPP | SEQ ID NO:188 |
| 190 | EPB41L2 | | Cytoskeletal protein | Y623 | APHLQLIEGKKNSLRVEGDNIyVR | SEQ ID NO: 189 |
| 191 | EPB41L2 | | Cytoskeletal protein | Y906 | TETKTITyESPQIDG | SEQ ID NO: 190 |
| 192 | EPB41 L4A | NP_071423.3 | Cytoskeletal protein | Y90 | TLAEHKELINTGPPyTLYFGIK | SEQ ID NO: 191 |
| 193 | EPB41 L4A | NP_071423.3 | Cytoskeletal protein | Y93 | TLAEHKELlNTGPPYTLyFGIK | SEQ ID NO: 192 |
| 194 | FKSG30 | NP_001017421.1 | Cytoskeletal protein | Y240 | SyELPDGQVITIGNER | SEQ ID NO: 193 |
| 195 | FRMD3 | NP_777598.2 | Cytoskeletal protein | Y96 | QMKTHPPYTMCFRVKFyPHEPLK | SEQ ID NO: 194 |
| 196 | FRMD3 | NP_777598.2 | Cytoskeletal protein | Y87 | QMKTHPPyTMCFRVKFYPHEPLK | SEQ ID NO: 195 |
| 197 | GAS8 | NP_001472.1 | Cytoskeletal protein | Y98 | HQVEIKVyKQKVKHL | SEQ ID NO: 196 |
| 198 | HRIHF821 22 | NP_008963.3 | Cytoskeletal protein | Y173 | QALDyVELSPLTQASPQR | SEQ ID NO; 197 |
| 199 | JUP | NP_002221.1 | Cytoskeletat protein | Y61 | KTTTyTQGVPPSQGDLEYQMSTTAR | SEQ ID NO: 198 |
| 200 | JUP | NP_002221.1 | Cytoskeletal protein | Y729 | MDMDGDYPIDTySDGLRPPYPT | SEQ ID NO:199 |
| 201 | JUP | NP_002221.1 | Cytoskeletal protein | Y22 | VTEWQQTYTyDSGIHSGANTCVPSVSSK | SEQ ID NO: 200 |
| 202 | K6IRS3 | NP_778238.1 | Cytoskeletal protein | Y32 | GGFSGCSAVLSGGSSSyRAGGKGLSGG FSSR | SEQ ID NO: 201 |
| 203 | KRT8 | NP_002264.1 | Cytoskeletal protein | Y267 | AQyEDIANR | SEQ ID NO: 202 |
| 204 | KRT8 | NP_002264.1 | Cytoskeletal protein | Y204 | DVDEAyMNKVELESR | SEQ ID NO: 203 |
| 205 | KRT9 | AAC60619.1 | Cytoskeletal protein | Y10 | QFSSSyLTSGGGGGGGLGSGGSIR | SEQ ID NO: 204 |
| 206 | MAP1B | NP_005900.1 | Cytoskeletal protein | Y2057 | RTPQASTySYETSDL | SEQ ID NO: 205 |
| 207 | MAP1B | NP_005900.1 | Cytoskeletal protein | Y1337 | SAGHTPYyQSPTDEK | SEQ ID NO: 206 |
| 208 | MAP1B | NP_005900.1 | Cytoskeletal protein | Y1906 | TSDVGGYYyEK | SEQfDNO:207 |
| 209 | NCKIPSD | NP_909119.1 | Cytoskeletal protein | Y161 | QHSLPSSEHLGADGGLyQIPPQPR | SEQ ID NO: 208 |
| 210 | NEB | NP_004534.1 | Cytoskeletal protein | Y4561 | AKRGQKLQSQyLYVELATKER | SEQ ID N0: 209 |
| 211 | NEB | NP_004534.1 | Cytoskeletal protein | Y1381 | KNYENTKTSyHTPGDMVTITAAK | SEQ ID NO: 210 |
| 212 | NEB | | Cytoskeletal protein | Y5194 | AKRGQKLQSQyLYVELATKER | SEQ ID NO: 211 |
| 213 | NEB | NP_004534.1 | Cytoskeletal protein | Y5242 | yTPVPDTPILIRAKR | SEQ ID NO: 212 |
| 214 | NEB | NP_004534.1 | Cytoskeletal protein | Y1412 | TPGDMVTITAAKMAQDVATNVNYKQPLHH | SEQ ID NO: 213 |
| 215 | PLEC1 | | Cytoskeletal protein | Y4408 | GYYSPySVSGSGSTAGSR | SEQ ID NO: 214 |
| 216 | PLEC1 | | Cytoskeletal protein | Y4505 | GYYSPySVSGSGSTAGSR | SEQ ID NO: 215 |
| 217 | SPTBN1 | NP_003119.1 | Cytoskeletal protein | Y2039 | DASVAEAWLLGQEPyLSSR | SEQ ID NO: 216 |
| 218 | TLN1 | NP_006280.2 | Cytoskeletal protein | Y570 | NLTAGDPAETDyTAVGC | SEQ ID NO: 217 |
| 219 | TUBA1 | NP_005991.1 | Cytoskeletal protein | Y103 | QLFHPEOLITGKEDAANNyAR | SEQ ID NO: 218 |
| 220 | VIM | NP_003371.2 | Cytoskeletal protein | Y38 | TySLGSALRPSTSR | SEQ ID NO: 219 |
| 221 | WASF1 | | Cytoskeletal protein | Y235 | ANGPASHfETRPQTY | SEQ ID NO: 220 |
| 222 | VIL2 | NP_003370.2 | Cytoskeletal protein; Cytoskeletal protein | Y483 | SyHVQESLQDEGAEPT | SEQ ID N0: 221 |
| 223 | APLP2 | NP_001633.1 | DNA binding protein | Y755 | MQNHGYENPTyK | SEQ ID NO: 222 |
| 224 | APRIN | NP_055847.1 | DNA binding protein | Y1187 | GRLDSSEMDHSENEDyTMSSPLPGK | SEQ ID NO: 223 |
| 225 | HIST1H2BG | NP_003509.1 | DNA binding protein | Y41 | KESYSVyVYK | SEQ ID NO: 224 |
| 226 | HIST1H2BG | NP_003518.2 | DNA binding protein | Y41 | ESYSIyVYK | SEQ ID NO: 225 |
| 227 | HIST1H41 | NP_003486.1 | DNA binding protein | Y89 | VTAMDVVyALKRQGR | SEQ ID NO: 226 |
| 228 | MECP2 | NP_004983.1 | DNA binding protein | Y141 | VELIAyFEKVGDTSLDPNDFDFTVTGRGSP SR | SEQ ID NO: 227 |
| 229 | NUCB1 | NP_006175.2 | DNA binding protein | Y168 | DLAQyDAAHHEEFKR | SEQ ID N0: 228 |
| 230 | RUVBL2 | NP_006657.1 | DNA binding protein | Y215 | ARDyDAMGSQTK | SEQ ID N0: 229 |
| 231 | FUS | NP_004951.1 | DNA binding protein; RNA binding protein | Y468 | PDGPGGGPGGSHMGGNyGDDRRGGRG GYDR | SEQ ID NO: 230 |
| 232 | PARP1 | NP_001609,1 | DNA repair | Y176 | PEySASQLKGFSLLATEDK | SEQ ID NO: 231 |
| 233 | PAXIP1 | NP_031375.3 | DNA repair | Y115 | CTHLIVPEPKGEKyECALK | SEQ ID NO: 232 |
| 234 | PAXIP1 | NP_031375.3 | DNA repair | Y701 | LMAYLAGAKyTGYLCR | SEQ ID NO: 233 |
| 235 | PAXIP1 | NP_01375.3 | DNA repair | Y704 | LMAYLAGAKYTGyLCR | SEQ ID NO: 234 |
| 236 | POLE | NP_006222.2 | DNA repair | Y718 | AFHELSREEQAKyEK | SEQ ID NO: 235 |
| 237 | ATRX | NP_000480.2 | DNA repair; Helicase | Y1667 | SyMLQRWQEDGGVMIIGYEMYRNLAQGR NVK | SEQ ID NO: 236 |
| 238 | PES1 | NP_055118.1 | DNA replication | Y171 | LTVEFMHyIIAAR | SEQ ID NO: 237 |
| 239 | TERF21P | NP_061848.2 | DNA replication | Y32 | DPNGPTHSSTLFVRDDGSSMSFyVR | SEQ ID NO: 238 |
| 240 | C12orf8 | NP_006808.1 | Endoplasmic reticulum | Y66 | FDTQYPyGEKQDEFK | SEQ ID NO: 239 |
| 241 | DERL2 | NP_057125.2 | Endoplasmic reticulum | Y218 | AIFDTPDEDPNyNPLPEERPGGFAWGEGQ | SEQ ID NO: 240 |
| 242 | Eno1 | | Enzyme, cellular metabolism | Y25 | EIFDSRGNPTVEVDLyTAK | SEQ ID NO: 241 |
| 243 | ADHFE1 | NP_653251.1 | Enzyme, misc. | Y104 | AANLyASSPHSDFLDYVSAPIGK | SEQ ID NO: 242 |
| 244 | AGL | NP_000019.1 | Enzyme, misc. | Y1117 | CWGRDTFIALRGILLITGRyVEAR | SEQ ID NO: 243 |
| 245 | ARSA | NP_000478.2 | Enzyme, misc. | Y63 | FTDFyVPVSLCTPSR | SEQ ID NO: 244 |
| 246 | ARSA | NP_000478.2 | Enzyme, misc. | Y88 | LPVRMGMyPGVLVPSSR | SEQ ID NO: 245 |
| 247 | COX11 | NP_004366.1 | Enzyme, misc. | Y111 | QNKTTLTYVAAVAVGMLGASyAAVPLYR | SEQ ID NO: 246 |
| 248 | CYP2C18 | NP_000763.1 | Enzyme, misc. | Y61 | DMSKSLTNFSKVyGPVFTVYFGLK | SEa 10 NO: 247 |
| 249 | ENTPD1 | NP_001767.3 | Enzyme, misc. | Y63 | YGIVLDAGSSHTSLylYK | SEQ ID NO: 248 |
| 250 | GAST | NP_000796.1 | Enzyme, misc. | Y87 | QGPWLEEEEEAyGWMDFGR | SEQ ID NO: 249 |
| 251 | GYS1 | NP_002094.2 | Enzyme, misc. | Y313 | GHFyGHLDFNLDK | SEQ ID MO: 250 |
| 252 | HYAL4 | NP_036401.1 | Enzyme, misc. | Y132 | ADQDINYyIPAEDFSGLAVIDWEYWR | SEQ ID NO: 251 |
| 253 | HYAL4 | NP_036401.1 | Enzyme, misc. | Y131 | ADQDINyYIPAEDFSGLAVIDWEYWR | SEQ ID NO: 252 |
| 254 | LANGL1 | NP_C06046.1 | Enzyme, misc. | Y21 | SLAEGyFDAAGRLTPEFSQR | SEQ ID N0: 253 |
| 255 | MCCC1 | NP_064551.2 | Enzyme, misc. | Y181 | SIMAAAGVPVVEGyHGEDQSDQCLK | SEQ ID NO: 254 |
| 256 | MOCS2 | NP_004522.1 | Enzyme, misc. | Y170 | AKVPIWKKElyEESSTWK | SEQ ID NO: 255 |
| 257 | NIT2 | NP_064587.1 | Enzyme, misc. | Y49 | IVSLPEOFNSPyGAK | SEQ ID NO: 256 |
| 258 | P4HB | NP_000909.2 | Enzyme, misc. | Y94 | LAKVDATEESDLAQQyGVRGYPTIK | SEQ ID NO: 257 |
| 259 | PDIA5 | NP_006801.1 | Enzyme, misc. | Y113 | VELFHyQDGAFHTEYNR | SEQ ID NO: 258 |
| 260 | POR | NP_000932,2 | Enzyme, misc. | Y262 | VyMGEMGRLKSYENQKPPFDAK | SEQ ID NO: 259 |
| 261 | TPH1 | NP_004170.1 | Enzyme, misc. | Y185 | ELNKLyPTHACREYLK | SEQ ID NO: 260 |
| 262 | XDH | NP_000370,2 | Enzyme, misc. | Y1092 | DLNGQAVyAACQTIL | SEQ ID NO: 261 |
| 263 | ADAMTS15 | NP_620686.1 | Extracellular matrix | Y725 | QRGYKGLlGDDNyLALKNSQGK | SEQ ID NO: 262 |
| 264 | ADAMTS19 | NP _598377,2 | Extracellular matrix | Y293 | RSMEEKVTEKSALHSHyCGIISDKGR | SEQ ID NO: 263 |
| 265 | FRAS1 | NP_079350.4 | Extracellular matrix | Y2710 | GDASSIVSAICyTVPKSAMGSSLYALESGS DFKSR | SEQ ID NO: 264 |
| 266 | HAPLN2 | NP_068589.1 | Extracellular matrix | Y226 | APCGGRGRPGIRSyGPR | SEQ ID NO: 265 |
| 267 | HSPG2 | NP_956472.1 | Extracellular matrix | Y1709 | GPHyFYWSREDGRPVPSGTQQR | SEQ ID NO: 266 |
| 268 | MMP2 | NP_004521.1 | Extracellular matrix | Y182 | IHDGEADIMINFGRWEHGDGyPFDGK | SEQ ID NO: 267 |
| 269 | PCOLCE | NP_002584.1 | Extracellular matrix | Y364 | EPGEGLAVNSLIGAyK | SEQ ID NO: 268 |
| 270 | EPS8L3 | NP_078802.2 | G protein regulator, misc. | Y16 | KEySQNLTSEPTLLQHR | SEQ ID NO: 269 |
| 271 | GPSM1 | NP_056412.2 | G protein regulator, misc. | Y229 | RAySNLGNAHVFLGRFDVAAEYYKK | SEQ ID NO: 270 |
| 272 | RND1 | NP_055285.1 | G protein regulator, misc. | Y50 | VPTVFENyTACLETE | SEQ ID NO: 271 |
| 273 | SPRED2 | NP_861449.1 | G protein regulator, misc. | Y251 | GKYPDPSEDADSSyVR | SEQ ID NO: 272 |
| 274 | RAN | NP_006316.1 | G protein, monomeric (non-Rab) | Y155 | SNyNFEKPFLWLAR | SEQ ID NO: 273 |
| 275 | GNL2 | NP_037417.1 | GTPase activating protein, misc, | Y198 | DRDLVTEDTGVRNEAQEEIyK | SEQ ID NO: 274 |
| 276 | ARHGAP2 1 | NP_065875.2 | GTPase activating protein, Rac/Rho | Y424 | AASQSTTDyNQVVPNR | SEQ ID NO: 275 |
| 277 | RASA1 | NP_002881.1 | GTPase activating protein, Ras | Y239 | IIAMCGDyYIGGR | SEQ ID NO: 276 |
| 278 | RASA3 | NP_031394.2 | GTPase activating protein, Ras | Y757 | ACGSKSVyDGPEQEE | SEQ ID NO: 277 |
| 279 | ARFGEF1 | NP_006412.2 | Guanine nucleotide exchange factor, ARF | Y719 | KPKRGIQyLQEQGML | SEQ ID NO: 278 |
| 280 | ARFGEF2 | NP_006411.1 | Guanine nucleotide exchange factor, ARF | Y1766 | AVLRKFFLRISVVyKIWIPEEPSQVPAALSP VW | SEQ ID NO: 279 |
| 281 | ARHGEF5 | NP_005426.2 | Guanine nucleotide exchange factor, Rac/Rho | Y656 | SGRDySTVSASPTALSTLK | SEQ ID NO: 280 |
| 282 | SWAP70 | NP_055870.2 | Guanine nucleotide exchange factor, Rac/Rho | Y517 | RKQALEQyEEVKKKL | SEQ ID NO: 281 |
| 283 | SOS1 | NP_005624.2 | Guanine nucleotide exchange factor, Ras | Y796 | QLTLLESDLyR | SEQ ID NO: 282 |
| 284 | AMPD2 | NP_004028.3 | Hydrolase, non-esterase | Y69 | yPFKKRASLQASTAAPEAR | SEQ ID NO: 283 |
| 285 | ATIC | NP_004035.2 | Hydrolase, non-esterase | Y293 | VCMVYDLyKTLTPIS | SEQ ID NO: 284 |
| 286 | CACH-1 | NP_570123,1 | Hydrolase, non-esterase | Y314 | yRGAIARKRIRLGR | SEQ ID NO: 285 |
| 287 | GGH | NP_003869.1 | Hydrolase, non-esterase | Y63 | YYIAASYVKyLESAGARWPVR | SEQ ID NO: 286 |
| 288 | META1 | NP_055958.1 | Hydrolase, non-esterase | Y139 | KLVQTTyECLMQAIDAVKPGVR | SEQ ID NO: 287 |
| 289 | NLN | NP_065777.1 | Hydrolase, non-esterase | Y40 | ILLRMTLGREVMSPLQAMSSyTVAGRNVL R | SEQ ID NO: 288 |
| 290 | TH | NP_954987.2 | Hydrolase, non-esterase | Y52 | QAEAIMGAPGPSLTGSPWPGTAAPAASyT PTPR | SEQ ID NO: 289 |
| 291 | THEX1 | NP_699163.2 | Hydrolase, non-esterase | Y66 | FITSSASDFSDPVyKEIAITNGCINR | SEQ ID NO: 290 |
| 292 | CAST | NP_775086.1 | Inhibitor protein | Y100 | yRELLAKPIGPDDAIDALSSDFTCGSPTAA GK | SEQ ID NO: 291 |
| 293 | CSTB | NP_000091.1 | Inhibitor protein | Y97 | AKHDELTyF | SEQ ID NO: 292 |
| 294 | ENSA | NP_004427.1 | Inhibitor protein | Y41 | LKAKyPSLGQKPGGSDFLMK | SEQ ID NO: 293 |
| 295 | ENSA | NP_004427.1 | Inhibitor protein | Y70 | YFDSGDyNMAK | SEQ ID NO: 294 |
| 296 | AK7 | NP_689540.1 | Kinase (non-protein) | Y359 | WAAQTGFVENINTILKEyKQSR | SEQ ID NO: 295 |
| 297 | ALDH18A1 | NP_001017423,1 | Kinase (non-protein) | Y585 | AAKGIPVMGHSEGICHMyVDSEASVDK | SEQ ID NO: 296 |
| 298 | C9orf12 | NP_073592.1 | Kinase (non-protein) | Y445 | PyESIPHQYKLDGK | SEQ ID NO: 297 |
| 299 | CKM | NP_001815.2 | Kinase (non-protein) | Y125 | GGDDLDPNyVLSSR | SEQ ID NO: 298 |
| 300 | MPP1 | NP_002427.1 | Kinase (non-protein) | Y48 | SRPEAVSHPLNTVTEDMyTNGSPAPGSPA QVK | SEQ ID NO: 299 |
| 301 | NME7 | NP_037462.1 | Kinase (non-protein) | Y82 | VNUFSRQLVLIDYGDQyTARQLGSRK | SEQ ID NO: 300 |
| 302 | NME7 | NP_037462.1 | Kinase (non-protein) | Y78 | VNVFSRQLVLIDyGDQYTARQLGSRK | SEQ ID NO: 301 |
| 303 | PIK3C2B | NP_002637.2 | Kinase, lipid | Y127 | GSLSGDyLYIFDGSDGGVSSSPGPGDIEG SCK | SEQ ID NO: 302 |
| 304 | PIK3R3 | AAC39696.1 | Kinase, lipid | Y282 | NEDADENyFINEEDENLPHYDEK | SEQ ID NO: 303 |
| 305 | PIPK-1 gamma | NP_003548.1 | Kinase, lipid | Y129 | FKTyAPVAFR | SEQ ID NO: 304 |
| 306 | PIK3CG | NP_002640.2 | Kinase, lipid | Y480 | FLLRRGEyVLHMWQISGK | SEQ ID NO: 305 |
| 307 | CLK2 | NP_003984.2 | KINASE; Protein kinase, dual-specificity | Y258 | DNNyLPYPIHQVR | SEQ ID NO: 306 |
| 308 | DYRK1A | NP_001387.2 | KINASE; Protein kinase, dual-specificity | Y319 | IyQYIQSR | SEQ ID NO: 307 |
| 309 | DYRK1B | NP_006475.1 | KINASE; Protein kinase, dual-specificity | Y386 | LQEDLVLRMLEyEPAAR | SEQ ID NO: 308 |
| 310 | RIPK5 | NP_056190.1 | KINASE; Protein kinase, dual-specificity | Y312 | QLIDLGyLSSSHWNCGAPGQDTKAQSML VEQSEK | SEQ ID NO: 309 |
| 311 | ANKK1 | NP_848605.1 | KINASE; Protein kinase, Ser/Thr (non-receptor) | Y67 | WRTEYAIKCAPCLPPDAASSDVNyLIEEAA KMK | SEQ ID NO: 310 |
| 312 | ANKK1 | NP_848605.1 | KINASE; Protein kinase, Ser/Chr (non-receptor) | Y48 | WRTEyAIKCAPCLPPDAASSDVNYLIEEAA KMK | SEQ ID NO: 311 |
| 313 | ARAF | NP_001645,1 | KINASE; Protein kinase, Ser/Thr (non-receptor) | Y526 | GyLSPDLSKISSNCPK | SEQ ID NO: 312 |
| 314 | CDC2L5 | NP_003709.2 | KINASE; Protein kinase, Ser/Thr (non-receptor) | Y716 | FDIIGIIGEGTyGQVYKARDKDTGEMVALK K | SEQ ID NO: 313 |
| 315 | CDC428PB | NP_06026.2 | KINASE; Protein receptor) | Y1638 | NKPylSWPSSGGSEPSVTVPLR | SEQ ID NO: 314 |
| 316 | DKFZp761 P0423 | XP_291277.2 | KINASE; Protein kinase, Ser/Thr (non-receptor), predicted | Y253 | CSPSGDSEGGEyCSILDCCPGSPVAK | SEQ ID NO: 315 |
| 317 | HUNK | NP_055401.1 | KINASE; Protein kinase, Ser/Thr (non-receptor) | Y388 | KLERyLSGKSDIQDSLCYK | SEQ ID NO: 316 |
| 318 | MAP4K1 | NP_009112.1 | KINASE; Protein kinase, Ser/Thr (non-receptor) | Y28 | LGGGTyGEVFKARDKVSGDLVALK | SEQ ID NO: 317 |
| 319 | MARK3 | | KINASE; Protein kinase, Ser/Thr (non-receptor) | Y418 | VQRSVSSSQKQRRySDHAGPAIPSVVAY PK | SEQ ID NO: 318 |
| 320 | MINK1 | NP_056531.1 | KINASE; Protein kinase, Ser/Thr (non-receptor) | Y1223 | IIKDVVLQWGEMPTSVAyICSNQIMGWGE K | SEQ ID NO: 319 |
| 321 | NEK2 | NP_002488.1 | KINASE; Protein kinase, Ser/Thr (non-receptor) | Y240 | RIPYRySDELNEIITRMLNLKDYHR | SEQ ID NO: 320 |
| 322 | PLK1 | NP_005021.2 | KINASE; Protein kinase, Ser/Thr (non-receptor) | Y268 | NEySIPKHINPVAASLIQKMLQTDPTAR | SEQ ID NO: 321 |
| 323 | PLK3 | NP_004064.2 | KINASE; Protein kinase, Ser/Thr (non-receptor) | Y164 | yYLRQILSGLKYLHQR | SEQ ID NO: 322 |
| 324 | PLK3 | NP_004064.2 | KINASE; Protein kinase, Ser/Thr (non-receptor) | Y165 | YyLRQILSGLKYLHQR | SEQ ID NO: 323 |
| 325 | PRKCI | NP_002731.3 | KINASE; Protein kinase, Ser/Thr (non-receptor) | Y388 | GIIYRDLKLDNVLLDSEGHIKLTDyGMCK | SEQ ID NO: 324 |
| 326 | RIPK2 | NP_003812.1 | KINASE; Protein kinase, Ser/Thr (non-receptor) | Y381 | KAQDCyFMK | SEQ ID NO: 325 |
| 327 | RPS6KA5 | NP_004746.2 | KINASE; Protein kinase, Ser/Thr (non-receptor) | Y423 | PGVTNVARSAMMKDSPFYQHyDLDLKDK | SEQ ID NO: 326 |
| 328 | RPS6KA5 | NP_004746.2 | KINASE; Protein kinase, Ser/Thr (non-receptor) | Y420 | PGVTNVARSAMMKDSPFyQHYDLDLKDK | SEQ ID NO: 327 |
| 329 | SLK | NP_085535.2 | KINASE; Protein kinase, Ser/Thr (non-receptor) | Y21 | QyEHVKRDLNPEDFWEIIGELGDGAFGKV YK | SEQ ID NO: 328 |
| 330 | SLK | NP_055535.2 | KINASE; Protein kinase, Ser/Thr (non-receptor) | Y49 | QYEHVKRDLNPEDFWEIIGELGDGAFGKV yK | SEQ ID NO: 329 |
| 331 | TNIK | NP_055843.1 | KINASE; Protein kinase, Ser/Thr (non-receptor) | Y963 | VSTHSQEMDSGTEyGMGSSTK | SEQ ID NO: 330 |
| 332 | TRIB2 | NP_067675.1 | KINASE; Protein kinase, Ser/Thr (non-receptor) | Y14 | STPITIARyGRSRNKTQDFEELSSIR | SEQ ID NO: 331 |
| 333 | TSSK1 | NP_114417.1 | KINASE; Protein kinase, Ser/Thr (non-receptor) | Y23 | RGYLLGINLGEGSyAKVK | SEQ ID NO: 332 |
| 334 | TTN | NP_003310.3 | KINASE; Protein kinase, Ser/Thr (non-receptor) | Y22419 | PMYDGGTDIVGyVLEMQEK | SEQ ID NO: 333 |
| 335 | TTN | | KINASE; Protein kinase, Ser/Thr (non-receptor) | Y22879 | PMYDGGTDIVGyVLEMQEK | SEQ ID NO: 334 |
| 336 | TTN | NP_003310.3 | KINASE; Protein kinase, Ser/Thr (non-receptor) | Y15525 | VENLTEGAIYyFR | SEQ ID NO: 335 |
| 337 | TTN | NP_003310.3 | KINASE; Protein kinase, Ser/Thr (non-receptor) | Y21240 | VTGLVEGLEYQFRTyALNAAGVSKASEASF SEQ | ID NO: 336 |
| 338 | TTN | NP_003310.3 | KINASE; Protein kinase, Ser/Thr (non-receptor) | Y17689 | yGVSQPLVSSIIVAK | SEQ ID NO: 337 |
| 339 | FER | NP_005237.1 | KINASE; Protein kinase, tyrosine (non-receptor) | Y402 | VQENDGKEPPPWNyEEDAR | SEQ ID NO: 338 |
| 340 | HCK | NP_002101.2 | KINASE; Protein kinase, tyrosine (non-receptor) | Y209 | TLDNGGFyISPR | SEQ ID NO: 339 |
| 341 | JAK3 | NP_000206.2 | KINASE; Protein kinase, tyrosine (non-receptor) | Y929 | LDASRLLLySSQICKGMEYLGSRR | SEQ ID NO: 340 |
| 342 | PTK2 | NP_005598.3 | KINASE; Protein kinase, tyrosine (non-receptor) | Y592 | LGDFGLSRyMEDSTYYK | SEQ ID NO: 341 |
| 343 | YES1 | NP_005424.1 | KINASE; Protein kinase, tyrosine (non-receptor) | Y32 | YRPENTPEPVSTSVSHyGAEPTTVSPCPS SSAK | SEQ ID NO: 342 |
| 344 | ZAP70 | NP_001070.2 | KINASE; Protein kinase, tyrosine (non-receptor) | Y451 | REEIPVSNVAELLHQVSMGMKyLEEK | SEQ ID NO: 343 |
| 345 | ACVR2A | NP_001607.1 | KINASE; Receptor Ser/Thr kinase | Y302 | GLAyLHEDIPGLKDGHKPAISHRDIK | SEQ ID NO: 344 |
| 346 | DDR1 | | KINASE; Receptor tyrosine kinase | Y513 | EPPPYQEPRPRGNPPHSAPCVPNGSALL LSNPAyR | SEQ ID NO: 345 |
| 347 | DDR1 | NP_001945.3 | KINASE; Receptor tyrosine kinase | Y759 | NLYAGDYyR | SEQ ID NO: 346 |
| 348 | DDR1 | NP_001945.3 | KINASE; Receptor tyrosine kinase | Y755 | NLYAGDyYR | SEQ ID NO: 347, |
| 349 | DDR1 | | KINASE; Receptor tyrosine kinase | Y760 | NLYAGDYyR | SEQ ID NO: 348 |
| 350 | DDR2 | NP_006113.2 | KINASE; Receptor tyrosine kinase | Y521 | GPEGVPHyAEADIVN | SEQ ID NO: 349 |
| 351 | EGFR | | KINASE; Receptor tyrosine kinase | Y1138 | AVGNPEyLNTVQPT | SEQ ID NO: 350 |
| 352 | EPHA2 | NP_004422.2 | KINASE; Receptor tyrosine kinase | Y729 | GIAAGMKyLANMNYVHR | SEQ ID NO: 351 |
| 353 | ERBB2 | NP_001005862.1 | KINASE; Receptor tyrosine kinase | Y975 | FVVIQNEDLGPASPLDSTFyR | SEQ ID NO: 352 |
| 354 | ERBB2 | NP_001005862.1 | KINASE; Receptor tyrosine kinase | Y705 | VLGSGAFGTVyK | SEQ ID NO: 353 |
| 355 | ERBB3 | NP_001973.2 | KINASE; Receptor tyrosine kinase | Y1199 | EGTLSSVGLSSVLGTEEEDEDEEYEyMN RR | SEQ ID NO: 354 |
| 356 | ERBB4 | NP_005226.1 | KINASE; Receptor tyrosine kinase | Y1150 | GELDEEGyMTPMR | SEQ ID NO: 355 |
| 357 | ERBB4 | | KINASE; Receptor tyrosine kinase | Y1284 | IRPIVAENPEyLSEFSLKPGTVLPPPPYR | SEQ ID NO: 356 |
| 358 | ERBB4 | NP_005226.1 | KINASE; Receptor tyrosine kinase | Y1258 | STLQHPDyLQEYSTK | SEQ ID NO: 357 |
| 359 | ERBB4 | NP_005226.1 | KINASE; Receptor tyrosine kinase | Y1262 | STLQHPDYLQEySTK | SEQ ID NO: 358 |
| 360 | FGFR1 | NP_056934.2 | KINASE; Receptor tyrosine kinase | Y397 | PAVMTSPLYLEIIIYCTGAFLISCMVGSVIV yK | SEQ ID NO: 359 |
| 361 | FLT1 | NP_002010.1 | KINASE; Receptor tyrosine kinase | Y1053 | DIYKNPDyVR | SEQ ID NO: 360 |
| 362 | MST1R | NP_002438.1 | KINASE; Receptor tyrosine kinase | Y1239 | DILDREYySVQQHR | SEQ ID NO: 361 |
| 363 | ROR1 | NP_005003.1 | KINASE; Receptor tyrosine kinase | Y836 | FIPINGYPIPPGYAAFPAAHyQPTGPPR | SEQ ID NO: 362 |
| 364 | ROS1 | NP_002935.2 | KINASE; Receptor tyrosine kinase | Y2110 | DIyKNDYYR | SEQ ID NO: 363 |
| 365 | ROS1 | NP_002935.2 | KINASE; Receptor tyrosine kinase | Y2114 | DIYKNDyYR | SEQ ID NO: 364 |
| 366 | AARS | NP_001596.2 | Ligase | Y279 | PyTGKVGAEDADGIDMAYR | SEQ ID NO: 365 |
| 367 | CARS | NP_001014437.1 | Ligase | Y781 | LAKMKIPPSEMFLSETDKySKFDENGLPTH DMEGK | SEQ ID NO: 366 |
| 368 | EPRS | NP_004437.2 | Ligase | Y377 | TGNKYNVYPTyDFACPIVDSIEGUTHALR | SEQ ID NO: 367 |
| 369 | ALB | NP_000468.1 | Lipid binding protein | Y164 | YLyEIAR | SEQ ID NO: 368 |
| 370 | ANXA11 | NP_001148.1 | Lipid binding protein | Y482 | SLyHDISGDTSGDYR | SEQ ID NO: 369 |
| 371 | ANXA2 | NP_001002857.1 | Lipid binding protein | Y333 | ALLyLCGGDD | SEQ ID NO: 370 |
| 372 | ANXA2 | NP_001002857.1 | Lipid binding protein | Y318 | SLYYyIQQDTK | SEQ ID NO: 371 |
| 373 | ANXA2 | NP_001002857.1 | Lipid binding protein | Y316 | SLyYYIQQDTK | SEQ ID NO: 372 |
| 374 | ANXA2 | NP_001002857.1 | Lipid binding protein | Y317 | SLYyYIQQDTK | SEQ ID NO: 373 |
| 375 | ANXA4 | NP_001144.1 | Lipid binding protein | Y309 | LYGKSLYSFIKGDTSGDyR | SEQ ID NO: 374 |
| 376 | ANXA4 | NP_001144.1 | Lipid binding protein | Y293 | LyGKSLYSFIKGDTSGDYR | SEQ ID NO: 375 |
| 377 | ANXA5 | NP_001145.1 | Lipid binding protein | Y94 | LYDAyELK | SEQ ID NO: 376 |
| 378 | ANXA6 | NP_001146.2 | Lipid binding protein | Y645 | EFIEKyDK | SEQ ID NO: 377 |
| 379 | PLEKHA5 | NP_061885.2 | Lipid binding protein | Y128 | ERPISMINEASNyNVTSDYAVHPMSPVGR | SEQ ID NO: 378 |
| 380 | PLEKHA5 | NP_061885.2 | Lipid binding protein | Y134 | ERPISMINEASNYNVTSDyAVHPMSPVGR | SEQ ID NO: 379 |
| 381 | ACLY | NP_001087.2 | Lyase | Y1073 | SMGFIGHyLDQK | SEQ ID NO: 380 |
| 382 | COMT | NP_000745.1 | Methyltransferase | Y82 | VLEAIDTyCEQKEWA | SEQ ID NO: 381 |
| 383 | C3orf15 | NP_203528.2 | Mitochondrial | Y372 | RNIIKDYSDYASQVyGPLSR | SEQ ID NO: 382 |
| 384 | MRPL19 | NP_055578.2 | Mitochondrial | Y100 | KVLHIPEFyVGSILR | SEQ ID NO: 383 |
| 385 | SLC25A37 | NP_057696.2 | Mitochondrial | Y84 | MQSLSPDPKAQyTSIYGALKKIMR | SEQ ID NO: 384 |
| 386 | SLC25A4 | NP_001142.2 | Mitochondrial | Y195 | AAYFGVyDTAK | SEQ ID NO: 385 |
| 387 | DNCL1 | NP_003737.1 | Motor protein | Y50 | KKEFDKKyNPTWHCI | SEQ ID NO: 386 |
| 388 | KIF2C | NP_006836.1 | Motor protein | Y223 | AQEyDSSFPNWEFARMIKEFR | SEQ ID NO: 387 |
| 389 | KLC2L | NP_803136.2 | Motor protein | Y399 | NNLASAyLKQNKYQQAEELYKEILHK | SEQ ID NO: 388 |
| 389 | KLC2L | NP_803136.2 | Motor protein | Y399 | NNLASAyLKQNKYQQAEELYKEILHK | SEQ ID NO: 388 |
| 390 | KLC2L | NP_803136.2 | Motor protein | Y405 | NNLASAYLKQNKyQQAEELYKEILHK | SEQ ID NO: 389 |
| 391 | MYH3 | NP_002461.2 | Motor protein | Y104 | PEDVYAMNPPKFDRIEDMAMLTHLNEPAV LyNLK | SEQ ID NO: 390 |
| 392 | MYH3 | NP_002461.2 | Motor protein | Y78 | PEDVyAMNPPKFDRIEDMAMLTHLNEPAV LYNLK | SEQ ID NO: 391 |
| 393 | MYH1 | NP_005954.2 | Motor protein | Y1852 | ELTyQTEEDRK | SEQ ID NO: 392 |
| 394 | MYH1 | NP_005954.2 | Motor protein | Y1375 | TKyETDAIQR | SEQ ID NO: 393 |
| 395 | MYH1 | NP_005954.2 | Motor protein | Y410 | VKVGNEyVTK | SEQ ID NO: 394 |
| 396 | MYLPF | NP_037424.2 | Motor protein | Y158 | NICyVITHGDAKDQE | SEQ ID NO: 395 |
| 397 | MYO1B | NP_036355.2 | Motor protein | Y78 | NRNFyELSPHIFALSDEAYR | SEQ ID NO: 396 |
| 398 | MYO5C | NP_061198.1 | Motor protein | Y285 | HLKLGSAEEFNyTRMGGNTVIEGVNDRAE MVETQK | SEQ ID NO: 397 |
| 399 | MYO9A | NP_008832.1 | Motor protein | Y203 | MyDNHQLGKPEPHIYAVADVAYHAMLQR KK -- | SEQ ID NO: 398 |
| 400 | TPM1 | NP_000357.3 | Motor protein | Y162 | HIAEDADRKyEEVAR | SEQ ID NO: 399 |
| 401 | TPM2 | NP_003280.2 | Motor protein; Actin binding protein | Y162 | HIAEDSDRKyEEVAR | SEQ ID NO: 400 |
| 402 | AKR1B10 | | Oxidoreductase | Y316 | ACNVLQSSHLEDYPFDAEy | SEQ ID NO: 401 |
| 403 | AKR1B10 | NP_064695.2 | Oxidoreductase | Y310 | QSSHLEDyPFDAEY | SEQ ID NO: 402 |
| 404 | AKR1C1 | NP_001344.2 | Oxidoreductase | Y24 | LNDGHFMPVLGFGTyAPAEVPK | SEQ ID NO: 403 |
| 405 | ALOX15 | NP_001131.3 | Oxidoreductase | Y483 | YVEGIVSLHyKTDVAVKDDPELQTWCR | SEQ ID NO: 404 |
| 406 | CDO1 | NP_001792.2 | Oxidoreductase | Y58 | yTRNLVDQGNGK | SEQ ID NO: 405 |
| 407 | SCD | NP_005054.3 | Oxidoreductase | Y14 | QDDISSSyTTTTTIT | SEQ ID NO: 406 |
| 408 | PHPT1 | NP_054891.2 | Phosphatase | Y116 | AKYPDyEVTWANDGY | SEQ ID NO: 407 |
| 409 | ACP1 | NP_004291.1 | Phosphatase (non-protein) | Y143 | QLIIEDPYYGNDSDFETVyQQCVR | SEQ ID NO: 408 |
| 410 | ACP5 | NP_001602.1 | Phosphatase (non-protein) | Y199 | EDyVLVAGHYPVWSIAEHGPTHCLVK | SEQ ID NO: 409 |
| 411 | ACP5 | NP_001602.1 | Phosphatase (non-protein) | Y206 | EDYVLVAGHyPVWSIAEHGPTHCLVK | SEQ ID NO: 410 |
| 412 | ALPI | NP_001622.1 | Phosphatase (non-protein) | Y236 | KYMFPMGTPDPEyPADASQNGIR | SEQ ID NO: 411 |
| 413 | PNKP | NP_009185.2 | Phosphatase (non-protein) | Y211 | LRELEAEGyKLVIFTNQMSIGRGK | SEQ ID NO: 412 |
| 414 | INPP5D | NP_001017915.1 | Phosphatase, lipid | Y40 | ASESISRAyALCVLYR | SEQ ID NO: 413 |
| 415 | INPP5D | NP_001017915.1 | Phosphatase, lipid | Y46 | AYALCVLyR | SEQ ID NO: 414 |
| 416 | IGBP1 | NP_001542.1 | Phosphatase, regulatory subunit | Y145 | TMNNSAENHTANSSMAyPSLVAMASQR | SEQ ID NO: 415 |
| 417 | CTDSP1 | NP_067021.1 | PHOSPHATASE; Protein phosphatase, Ser/Thr (non-receptor) | Y158 | yADPVADLLDK | SEQ ID NO: 416 |
| 418 | PTPRA | NP_002827.1 | PHOSPHATASE; Receptor protein phosphatase, tyrosine | Y791 | VVQEyIDAFSDYANFK | SEQ ID NO: 417 |
| 419 | PTPRF | NP_002831.2 | PHOSPHATASE; Receptor protein phosphatase, tyrosine | Y1311 | RLNyQTPGMR | SEQ ID NO: 418 |
| 420 | PLA2G4A | NP_077734.1 | Phospholipase | Y7 | MSFIDyQHIIVEH | SEQ ID NO: 419 |
| 421 | PLCB1 | NP_056007.1 | Phospholipase | Y239 | PyLTVDQMMDFINLK | SEQ ID NO: 420 |
| 422 | PLD1 | NP_002653.1 | Phospholipase | Y420 | RKAQQGVRIFIMLyK | SEQ ID NO: 421 |
| 423 | ACR | NP_001088.1 | Protease (non-proteasomal) | Y110 | EITyGNNKPVKAPVQERYVEK | SEQ ID NO: 422 |
| 424 | BF | NP_001701.2 | Protease (non-proteasomal) | Y363 | KALQAVySMMSWPDDVPPEGWNR | SEQ ID NO: 423 |
| 425 | CNDP1 | NP_116038.4 | Protease (non-proteasomal) | Y248 | PAITYGTRGNSyFMVEVKCR | SEQ ID NO: 424 |
| 426 | ECEL1 | NP_004817.1 | Protease (non-proteasomal) | Y505 | AARAKLQyMMVMVGY | SEQ ID NO: 425 |
| 427 | LNPEP | | Protease (non-proteasomal) | Y70 | GLGEHEMEEDEEDyESSAK | SEQ ID NO: 426 |
| 428 | NAALADL2 | NP_996898.1 | Protease (non-proteasomal) | Y106 | LQEESDyITNYTR | SEQ ID NO: 427 |
| 429 | NDEL1 | NP_110435.1 | Protease (non-proteasomal) | Y114 | IKEQLHKyVRELEQA | SEQ ID NO: 428 |
| 430 | SEC11L3 | NP_150596.1 | Protease (non-proteasomal) | Y185 | YALLAVMGAyVLLKRES | SEQ ID NO: 429 |
| 431 | SEC11L3 | NP_150596.1 | Protease (non-proteasomal) | Y176 | yALLAVMGAYVLLKRES | SEQ ID NO: 430 |
| 432 | TESSP2 | NP_874361.1 | Protease (non-proteasomal) | Y255 | GMVCGyKEQGKDSCQGDSGGR | SEQ ID NO: 431 |
| 433 | APG4D | NP_116274.3 | Protease (proteasomal subunit) Y398 | | MAFAKMDPSCTVGFyAGDRK | SEQ ID NO: 432 |
| 434 | PSMA6 | NP_002782.1 | Protease (proteasomal subunit) | Y160 | CDPAGYyCGFK | SEQ ID NO: 433 |
| 435 | PSMB7 | NP_002790.1 | Protease (proteasomal subunit) | Y7 | MAAVSVyAPPVGGFSFDNCRRNAVLEAD FAKRGYK | SEQ ID NO: 434 |
| 436 | PSMB8 | NP_004150.1 | Protease (proteasomal subunit) | Y108 | VIEINPyLLGTMSGCAADCQYWER | SEQ ID NO: 435 |
| 437 | PSMC6 | NP_002797.2 | Protease (proteasomal subunit) Y207 | | VVSSSIVDKyIGESAR | SEQ ID NO: 436 |
| 438 | PSMD13 | NP_002808.2 | Protease (proteasomal subunit) | Y162 | FYDLSSKyYQTIGNH | SEQ ID NO: 437 |
| 439 | PSMD13 | NP.002808.2 | Protease (proteasomal subunit) Y172 | | TIGNHASyYKDALRF | SEQ ID NO: 438 |
| 440 | PSMD13 | NP_002808.2 | Protease (proteasomal subunit) Y156 | | TSVHSRFyDLSSKYY | SEQ ID NO: 439 |
| 441 | PSMD13 | NP_002808.2 | Protease (proteasomal subunit) Y163 | | YDLSSKYyQTIGNHA | SEQ ID NO: 440 |
| 442 | PPP1R12A | NP_002471.1 | Protein phosphatase, regulatory subunit | Y496 | LAyVAPTIPR | SEQ ID NO: 441 |
| 443 | PPP1R14B | NP_619634.1 | Protein phosphatase, regulatory subunit | Y29 | VyFQSPPGAAGEGPGGADDEGPVRR | SEQ ID NO: 442 |
| 444 | CXCR3 | NP_001495.1 | Receptor, GPCR | Y60 | AFLPALySLLFLLGLLGNGAVAAVLLSR | SEQ ID NO: 443 |
| 445 | GPR10 | NP_004239.1 | Receptor, GPCR | Y160 | TTIAVDRyVVLVHPL | SEQ ID NO: 444 |
| 446 | GPR126 | NP_055188.4 | Receptor, GPCR | Y1172 | SLSSSSIGSNSTyLTSK | SEQ ID NO. 445 |
| 447 | GPR64 | NP_005747.1 | Receptor, GPCR | Y685 | ILIQLCAALLLLNLVFLLDSWIALyK | SEQ ID NO: 446 |
| 448 | GPRC5A | | Receptor, GPCR | Y350 | AHAWPSPYKDyEVK | SEQ ID NO: 447 |
| 449 | GPRC5A | | Receptor, GPCR | Y347 | AHAWPSPyKDYEVK | SEQ ID NO: 448 |
| 450 | GPRC5C | NP_061123.3 | Receptor, GPCR | Y426 | AEDMySAQSHQAATPPK | SEQ ID NO: 449 |
| 451 | GPRC5C | NP_071319.2 | Receptor, GPCR | Y432 | KVPSEGAyDIILPRA | SEQ ID NO: 450 |
| 452 | GPRC5C | NP_071319.2 | Receptor, GPCR | Y483 | SQVFRNPyVWD | SEQ ID NO: 451 |
| 453 | GPRC5C | | Receptor, GPCR | Y399 | VPSEGAyDIILPR | SEQ ID NO: 452 |
| 454 | LHCGR | NP_000224.2 | Receptor, GPCR | Y550 | IyFAVRNPELMATNKDTKIAK | SEQ ID NO: 453 |
| 455 | OR5BU1 | NP_001004734.1 | Receptor, GPCR | Y307 | EIKTAMWRLFVKIyFLQK | SEQ ID NO: 454 |
| 456 | OR9Q1 | NP_001005212.1 | Receptor, GPCR | Y277 | VVSVLyTEVIPMLNPLIYSLRNK | SEQ ID NO: 455 |
| 457 | P2RY1 | NP_002554.1 | Receptor, GPCR | Y136 | LQRFIFHVNLyGSILFLTCISAHR | SEQ ID NO: 456 |
| 458 | TAS2R40 | NP_795363.1 | Receptor, GPCR | Y168 | DVFNVyVNSSIPIPSSNSTEK | SEQ ID NO: 457 |
| 459 | FCER1G | NP_004097.1 | Receptor, misc. | Y76 | NQETyETLK | SEQ ID NO: 458 |
| 460 | ADAR | NP_001102.2 | RNA binding protein | Y1222 | GLKDMGYGNWISKPQEEKNFyLCPV | SEQ ID NO: 459 |
| 461 | FXR2 | | RNA binding protein | Y519 | KDPDSNPySLLDTSE | SEQ ID NO: 460 |
| 462 | HNRPA2B1 | NP_002128.1 | RNA binding protein | Y250 | GFGDGYNGYGGGPGGGNFGGSPGyGG GR | SEQ ID NO: 461 |
| 463 | HNRPH3 | NP_036339.1 | RNA binding protein | Y153 | GGDGYDGGYGGFDDyGGYNNYGYGNDG FDDR | SEQ ID NO: 462 |
| 464 | LOC38793 3 | CAI12730.1 | RNA binding protein | Y85 | DyFEKCSKIETIEVMEDR | SEQ ID NO: 463 |
| 465 | MAGOH | NP_002361.1 | RNA binding protein | Y40 | PDGKLRYANNSNyKNDVMIRK | SEQ ID NO: 464 |
| 466 | MATR3 | NP_061322.2 | RNA binding protein | Y219 | MDyEDDRLR | SEQ ID NO: 465 |
| 467 | MBNL1 | NP_066368.2 | RNA binding protein | Y252 | AAQyQVNQAAAAQAAATAAAMGIPQAVLP PLPKR | SEQ ID NO: 466 |
| 468 | NPM1 | NP_002511.1 | RNA binding protein | Y29 | ADKDyHFKVDNDENEHQLSLR | SEQ ID NO: 467 |
| 469 | PARN | NP_002573.1 | RNA binding protein | Y146 | NGIPYLNQEEERQLREQyDEK | SEQ ID NO: 468 |
| 470 | PARN | NP_002573.1 | RNA binding protein | Y133 | NGIPyLNQEEERQLREQYDEK | SEQ ID NO: 469 |
| 471 | PDCD11 | NP_055791.1 | RNA binding protein | Y238 | AQEYIRQKNKGAKLKVGQyLNCIVEKVK | SEQ ID NO: 470 |
| 472 | PRPF31 | NP_056444.2 | RNA binding protein | Y205 | IyEYVESR | SEQ ID NO: 471 |
| 473 | RBM3 | NP_006734.1 | RNA binding protein | Y146 | NQGGYDRySGGNYRDNYDN | SEQ ID NO: 472 |
| 474 | RBMX | NP_002130.2 | RNA binding protein | Y225 | DDGYSTKDSYSSRDyPSSR | SEQ ID NO: 473 |
| 475 | RPL21 | NP_000973.2 | RNA binding protein | Y30 | HGVVPLATyMR | SEQ ID NO: 474 |

The short name for each protein in which a phosphorylation site has presently been identified is provided in Column A, and its SwissProt accession number (human) is provided Column B. The protein type/group into which each protein falls is provided in Column C. The identified tyrosine residue at which phosphorylation occurs in a given protein is identified in Column D, and the amino acid sequence of the phosphorylation site encompassing the tyrosine residue is provided in Column E (lower case y = the tyrosine (identified in Column D)) at which phosphorylation occurs. Table 1 above is identical to Figure 2, except that the latter includes the disease and cell type(s) in which the particular phosphorylation site was identified (Columns F and G).

The identification of these 474 phosphorylation sites is described in more detail in Part A below and in Example 1.

### Definitions.

As used herein, the following terms have the meanings indicated:
"Antibody" or "antibodies" refers to all types of immunoglobulins, including IgG, IgM, IgA, IgD, and IgE, including F_{ab} or antigen-recognition fragments thereof, including chimeric, polyclonal, and monoclonal antibodies. The term "does not bind" with respect to an antibody's binding to one phospho-form of a sequence means does not substantially react with as compared to the antibody's binding to the other phospho-form of the sequence for which the antibody is specific.
"Carcinoma-related signaling protein" means any protein (or polypeptide derived therefrom) enumerated in Column A of Table 1/Figure 2, which is disclosed herein as being phosphorylated in one or more human carcinoma cell line(s). Carcinoma-related signaling proteins may be protein kinases, or direct substrates of such kinases, or may be indirect substrates downstream of such kinases in signaling pathways. A Carcinoma-related signaling protein may also be phosphorylated in other cell lines (non-carcinomic) harboring activated kinase activity.
"Heavy-isotope labeled peptide" (used interchangeably with AQUA peptide) means a peptide comprising at least one heavy-isotope label, which is suitable for absolute quantification or detection of a protein as described in WO/03016861, "Absolute Quantification of Proteins and Modified Forms Thereof by Multistage Mass Spectrometry" (Gygi *et al*.), further discussed below.
"Protein" is used interchangeably with polypeptide, and includes protein fragments and domains as well as whole protein.
"Phosphorylatable amino acid" means any amino acid that is capable of being modified by addition of a phosphate group, and includes both forms of such amino acid.
"Phosphorylatable peptide sequence" means a peptide sequence comprising a phosphorylatable amino acid.
"Phosphorylation site-specific antibody" means an antibody that specifically binds a phosphorylatable peptide sequence/epitope only when phosphorylated, or only when not phosphorylated, respectively. The term is used interchangeably with "phospho-specific" antibody.

### A. Identification of Novel Carcinoma-related Signaling Protein Phosphorylation Sites.

The nearly 474 novel Carcinoma-related signaling protein phosphorylation sites disclosed herein and listed in Table 1/Figure 2 were discovered by employing the modified peptide isolation and characterization techniques described in "Immunoaffinity Isolation of Modified Peptides From Complex Mixtures," U.S. Patent Publication No. 20030044848, Rush et al. (the teaching of which is hereby incorporated herein by reference, in its entirety) using cellular extracts from the human carcinoma derived cell lines and patient samples indicated in Column G of Table 1/Figure 2. Exemplary cell lines used include Su-DHL1, MOLT15, H1703, 3T3-src, 3T3, Abl, A431, pancreatic xenograft, H1993, HCC827, 3T3-EGFRwt, 3T3-EGFR(L858R), HCT 116, HT29, NCI-N87, HT29, CTV-1, Karpas 299, MCF-10A (Y561F), MCF-10A (Y969F), Calu-3, H2347, H3255, H2170, U118MG, H1703, HCC366, H2228, HL61b, jurkat, SUPT-13, Verona patient 4, PT9, DU145, DMS79, MDA-MB-468, A549, H1666, H1650, 831/13, K562, HL53B, HL66B, HL84B, HL87A, HPAC, H441, SEM, Sor4, SorA, SEM, TgOVA, UT-7, MKPL-1, H69 LS, A431, DMS153 NS, SW620, HT116, MDA-MB-468, MCF10, HPAC, and HT29. The isolation and identification of phosphopeptides from these cell lines, using an immobilized general phosphotyrosine-specific antibody, is described in detail in Example 1 below. In addition to the nearly 474 previously unknown protein phosphorylation sites (tyrosine) discovered, many known phosphorylation sites were also identified (not described herein).

The immunoaffinity/mass spectrometric technique described in the '848 Patent Publication (the "IAP" method) -- and employed as described in detail in the Examples -- is briefly summarized below.

The IAP method employed generally comprises the following steps: (a) a proteinaceous preparation (*e*.*g*. a digested cell extract) comprising phosphopeptides from two or more different proteins is obtained from an organism; (b) the preparation is contacted with at least one immobilized general phosphotyrosine-specific antibody; (c) at least one phosphopeptide specifically bound by the immobilized antibody in step (b) is isolated; and (d) the modified peptide isolated in step (c) is characterized by mass spectrometry (MS) and/or tandem mass spectrometry (MS-MS). Subsequently, (e) a search program (*e*.*g*. Sequest) may be utilized to substantially match the spectra obtained for the isolated, modified peptide during the characterization of step (d) with the spectra for a known peptide sequence. A quantification step employing, *e*.*g*. SILAC or AQUA, may also be employed to quantify isolated peptides in order to compare peptide levels in a sample to a baseline.

In the IAP method as employed herein, a general phosphotyrosine-specific monoclonal antibody (commercially available from Cell Signaling Technology, Inc., Beverly, MA, Cat #9411 (p-Tyr-100)) was used in the immunoaffinity step to isolate the widest possible number of phosphotyrosine containing peptides from the cell extracts. Extracts from the human carcinoma cell lines described above were employed.

As described in more detail in the Examples, lysates were prepared from these cells line and digested with trypsin after treatment with DTT and iodoacetamide to alkylate cysteine residues. Before the immunoaffinity step, peptides were pre-fractionated by reversed-phase solid phase extraction using Sep-Pak C₁₈ columns to separate peptides from other cellular components. The solid phase extraction cartridges were eluted with varying steps of acetonitrile. Each lyophilized peptide fraction was redissolved in IAP buffer and treated with phosphotyrosine-specific antibody (P-Tyr-100, CST #9411) immobilized on protein Agarose. Immunoaffinity-purified peptides were eluted with 0.1% TFA and a portion of this fraction was concentrated with Stage or Zip tips and analyzed by LC-MS/MS, using a ThermoFinnigan LCQ Deca XP Plus ion trap mass spectrometer. Peptides were eluted from a 10 cm x 75 µm reversed-phase column with a 45-min linear gradient of acetonitrile. MS/MS spectra were evaluated using the program Sequest with the NCBI human protein database.

This revealed a total of 474 novel tyrosine phosphorylation sites in signaling pathways affected by kinase activation or active in carcinoma cells. The identified phosphorylation sites and their parent proteins are enumerated in Table 1/Figure 2. The tyrosine (human sequence) at which phosphorylation occurs is provided in Column D, and the peptide sequence encompassing the phosphorylatable tyrosine residue at the site is provided in Column E. Figure 2 also shows the particular type of carcinoma (see Column G) and cell line(s) (see Column F) in which a particular phosphorylation site was discovered.

As a result of the discovery of these phosphorylation sites, phospho-specific antibodies and AQUA peptides for the detection of and quantification of these sites and their parent proteins may now be produced by standard methods, described below. These new reagents will prove highly useful in, *e*.*g*., studying the signaling pathways and events underlying the progression of carcinomas and the identification of new biomarkers and targets for diagnosis and treatment of such diseases.

### B. Antibodies and Cell Lines

Isolated phosphorylation site-specific antibodies that specifically bind a Carcinoma-related signaling protein disclosed in Column A of Table 1 only when phosphorylated (or only when not phosphorylated) at the corresponding amino acid and phosphorylation site listed in Columns D and E of Table 1/Figure 2 may now be produced by standard antibody production methods, such as anti-peptide antibody methods, using the phosphorylation site sequence information provided in Column E of Table 1. For example, previously unknown Ser/Thr kinase phosphorylation site (tyrosine 388) (see Row 325 of Table 1/Figure 2) is presently disclosed. Thus, antibodies that specifically bind this novel Ser/Thr kinase site can now be produced, *e*.*g*. by immunizing an animal with a peptide antigen comprising all or part of the amino acid sequence encompassing the respective phosphorylated residue (*e*.*g*. a peptide antigen comprising the sequence set forth in Rows 325 of Column E, of Table 1 (SEQ ID NO: 324) (which encompasses the phosphorylated tyrosine at positions 388 of the Ser/Thr kinase), to produce an antibody that only binds Ser/Thr kinase when phosphorylated at that site.

Polyclonal antibodies of the invention may be produced according to standard techniques by immunizing a suitable animal (*e*.*g*., rabbit, goat, etc.) with a peptide antigen corresponding to the Carcinoma-related phosphorylation site of interest (*i*.*e*. a phosphorylation site enumerated in Column E of Table 1, which comprises the corresponding phosphorylatable amino acid listed in Column D of Table 1), collecting immune serum from the animal, and separating the polyclonal antibodies from the immune serum, in accordance with known procedures. For example, a peptide antigen corresponding to all or part of the novel Receptor tyrosine kinase phosphorylation site disclosed herein (SEQ ID NO: 352 = FVVIQNEDLGPASPLDSTFyR, encompassing phosphorylated tyrosine 975 (lowercase y; see Row 353 of Table 1)) may be used to produce antibodies that only bind Receptor tyrosine kinase phosphorylation when phosphorylated at tyr975. Similarly, a peptide comprising all or part of any one of the phosphorylation site sequences provided in Column E of Table 1 may employed as an antigen to produce an antibody that only binds the corresponding protein listed in Column A of Table 1 when phosphorylated (or when not phosphorylated) at the corresponding residue listed in Column D. If an antibody that only binds the protein when phosphorylated at the disclosed site is desired, the peptide antigen includes the phosphorylated form of the amino acid. Conversely, if an antibody that only binds the protein when not phosphorylated at the disclosed site is desired, the peptide antigen includes the non-phosphorylated form of the amino acid.

Peptide antigens suitable for producing antibodies of the invention may be designed, constructed and employed in accordance with well-known techniques. *See, e.g.,* ANTIBODIES: A LABORATORY MANUAL, Chapter 5, p. 75-76, Harlow & Lane Eds., Cold Spring Harbor Laboratory (1988); Czernik, Methods In Enzymology, 201: 264-283 (1991); Merrifield, J. Am. Chem. Soc. 85: 21-49 (1962)).

It will be appreciated by those of skill in the art that longer or shorter phosphopeptide antigens may be employed. *See Id.* For example, a peptide antigen may comprise the full sequence disclosed in Column E of Table 1/Figure 2, or it may comprise additional amino acids flanking such disclosed sequence, or may comprise of only a portion of the disclosed sequence immediately flanking the phosphorylatable amino acid (indicated in Column E by lowercase "y"). Typically, a desirable peptide antigen will comprise four or more amino acids flanking each side of the phosphorylatable amino acid and encompassing it. Polyclonal antibodies produced as described herein may be screened as further described below.

Monoclonal antibodies of the invention may be produced in a hybridoma cell line according to the well-known technique of Kohler and Milstein, See Nature 265: 495-97 (1975); Kohler and Milstein, Eur. J. Immunol. 6: 511 (1976); *see also,* CURRENT PROTOCOLS IN MOLECULAR BIOLOGY, Ausubel et al. Eds. (1989). Monoclonal antibodies so produced are highly specific, and improve the selectivity and specificity of diagnostic assay methods provided by the invention. For example, a solution containing the appropriate antigen may be injected into a mouse or other species and, after a sufficient time (in keeping with conventional techniques), the animal is sacrificed and spleen cells obtained. The spleen cells are then immortalized by fusing them with myeloma cells, typically in the presence of polyethylene glycol, to produce hybridoma cells. Rabbit fusion hybridomas, for example, may be produced as described in U.S Patent No. 5,675,063, C. Knight, Issued October 7, 1997. The hybridoma cells are then grown in a suitable selection media, such as hypoxanthine-aminopterin-thymidine (HAT), and the supernatant screened for monoclonal antibodies having the desired specificity, as described below. The secreted antibody may be recovered from tissue culture supernatant by conventional methods such as precipitation, ion exchange or affinity chromatography, or the like.

Monoclonal Fab fragments may also be produced in *Escherichia coli* by recombinant techniques known to those skilled in the art. See, *e.g.,* W. Huse, Science 246: 1275-81 (1989); Mullinax et al., Proc. Nat'l Acad. Sci. 87: 8095 (1990). If monoclonal antibodies of one isotype are preferred for a particular application, particular isotypes can be prepared directly, by selecting from the initial fusion, or prepared secondarily, from a parental hybridoma secreting a monoclonal antibody of different isotype by using the sib selection technique to isolate class-switch variants (Steplewski, et al., Proc. Nat'l. Acad. Sci., 82: 8653 (1985); Spira et al., J. Immunol. Methods, 74: 307 (1984)).

The preferred epitope of a phosphorylation-site specific antibody of the invention is a peptide fragment consisting essentially of about 8 to 17 amino acids including the phosphorylatable tyrosine, wherein about 3 to 8 amino acids are positioned on each side of the phosphorylatable tyrosine (for example, the FLOT1 tyrosine 238 phosphorylation site sequence disclosed in Row 49, Column E of Table 1), and antibodies of the invention thus specifically bind a target Carcinoma-related signaling polypeptide comprising such epitopic sequence. Particularly preferred epitopes bound by the antibodies of the invention comprise all or part of a phosphorylatable site sequence listed in Column E of Table 1, including the phosphorylatable amino acid.

Included in the scope of the invention are equivalent non-antibody molecules, such as protein binding domains or nucleic acid aptamers, which bind, in a phospho-specific manner, to essentially the same phosphorylatable epitope to which the phospho-specific antibodies of the invention bind. *See, e.g.,* Neuberger et al., Nature 312: 604 (1984). Such equivalent non-antibody reagents may be suitably employed in the methods of the invention further described below.

Antibodies provided by the invention may be any type of immunoglobulins, including IgG, IgM, IgA, IgD, and IgE, including F_{ab} or antigen-recognition fragments thereof. The antibodies may be monoclonal or polyclonal and may be of any species of origin, including (for example) mouse, rat, rabbit, horse, or human, or may be chimeric antibodies. *See, e.g.,* M. Walker et al., Molec. Immunol. 26: 403-11 (1989); Morrision et al., Proc. Nat'l. Acad. Sci. 81: 6851 (1984); Neuberger et al., Nature 312: 604 (1984)). The antibodies may be recombinant monoclonal antibodies produced according to the methods disclosed in U.S. Pat. No. 4,474,893 (Reading) or U.S. Pat. No. 4,816,567 (Cabilly et al.) The antibodies may also be chemically constructed by specific antibodies made according to the method disclosed in U.S. Pat. No. 4,676,980 (Segel et al.)

The invention also provides immortalized cell lines that produce an antibody of the invention. For example, hybridoma clones, constructed as described above, that produce monoclonal antibodies to the Carcinoma-related signaling protein phosphorylation sties disclosed herein are also provided. Similarly, the invention includes recombinant cells producing an antibody of the invention, which cells may be constructed by well known techniques; for example the antigen combining site of the monoclonal antibody can be cloned by PCR and single-chain antibodies produced as phage-displayed recombinant antibodies or soluble antibodies in *E. coli* (*see*, *e.g*., ANTIBODY ENGINEERING PROTOCOLS, 1995, Humana Press, Sudhir Paul editor.)

Phosphorylation site-specific antibodies of the invention, whether polyclonal or monoclonal, may be screened for epitope and phospho-specificity according to standard techniques. *See, e.g.* Czemik et al., Methods in Enzymology, 201: 264-283 (1991). For example, the antibodies may be screened against the phospho and non-phospho peptide library by ELISA to ensure specificity for both the desired antigen (*i.e.* that epitope including a phosphorylation site sequence enumerated in Column E of Table 1) and for reactivity only with the phosphorylated (or non-phosphorylated) form of the antigen. Peptide competition assays may be carried out to confirm lack of reactivity with other phosphoepitopes on the given Carcinoma-related signaling protein. The antibodies may also be tested by Western blotting against cell preparations containing the signaling protein, *e.g.* cell lines overexpressing the target protein, to confirm reactivity with the desired phosphorylated epitope/target.

Specificity against the desired phosphorylated epitope may also be examined by constructing mutants lacking phosphorylatable residues at positions outside the desired epitope that are known to be phosphorylated, or by mutating the desired phospho-epitope and confirming lack of reactivity. Phosphorylation-site specific antibodies of the invention may exhibit some limited cross-reactivity to related epitopes in non-target proteins. This is not unexpected as most antibodies exhibit some degree of cross-reactivity, and anti-peptide antibodies will often cross-react with epitopes having high homology to the immunizing peptide. *See, e.g., Czernik, supra.* Cross-reactivity with non-target proteins is readily characterized by Western blotting alongside markers of known molecular weight. Amino acid sequences of cross-reacting proteins may be examined to identify sites highly homologous to the Carcinoma-related signaling protein epitope for which the antibody of the invention is specific.

In certain cases, polyclonal antisera may exhibit some undesirable general cross-reactivity to phosphotyrosine itself, which may be removed by further purification of antisera, *e.g.* over a phosphotyramine column. Antibodies of the invention specifically bind their target protein (*i.e*. a protein listed in Column A of Table 1) only when phosphorylated (or only when not phosphorylated, as the case may be) at the site disclosed in corresponding Columns D/E, and do not (substantially) bind to the other form (as compared to the form for which the antibody is specific).

Antibodies may be further characterized via immunohistochemical (IHC) staining using normal and diseased tissues to examine Carcinoma-related phosphorylation and activation status in diseased tissue. IHC may be carried out according to well-known techniques. *See, e.g.,* ANTIBODIES: A LABORATORY MANUAL, Chapter 10, Harlow & Lane Eds., Cold Spring Harbor Laboratory (1988). Briefly, paraffin-embedded tissue (*e.g.* tumor tissue) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody and secondary antibody; and finally detecting using ABC avidin/biotin method according to manufacturer's instructions.

Antibodies may be further characterized by flow cytometry carried out according to standard methods. *See* Chow et al., Cytometry (Communications in Clinical Cytometry) 46: 72-78 (2001). Briefly and by way of example, the following protocol for cytometric analysis may be employed: samples may be centrifuged on Ficoll gradients to remove erythrocytes, and cells may then be fixed with 2% paraformaldehyde for 10 minutes at 37 °C followed by permeabilization in 90% methanol for 30 minutes on ice. Cells may then be stained with the primary phosphorylation-site specific antibody of the invention (which detects a Carcinoma-related signal transduction protein enumerated in Table 1), washed and labeled with a fluorescent-labeled secondary antibody. Additional fluorochrome-conjugated marker antibodies (*e.g*. CD45, CD34) may also be added at this time to aid in the subsequent identification of specific hematopoietic cell types. The cells would then be analyzed on a flow cytometer (*e.g.* a Beckman Coulter FC500) according to the specific protocols of the instrument used.

Antibodies of the invention may also be advantageously conjugated to fluorescent dyes (*e.g.* Alexa488, PE) for use in multiparametric analyses along with other signal transduction (phospho-CrkL, phospho-Erk 1/2) and/or cell marker (CD34) antibodies.

Phosphorylation-site specific antibodies of the invention specifically bind to a human Carcinoma-related signal transduction protein or polypeptide only when phosphorylated at a disclosed site, but are not limited only to binding the human species, *per se.* The invention includes antibodies that also bind conserved and highly homologous or identical phosphorylation sites in respective Carcinoma-related proteins from other species (*e.g*. mouse, rat, monkey, yeast), in addition to binding the human phosphorylation site. Highly homologous or identical sites conserved in other species can readily be identified by standard sequence comparisons, such as using BLAST, with the human Carcinoma-related signal transduction protein phosphorylation sites disclosed herein.

### C. Heavy-Isotope Labeled Peptides (AQUA Peptides).

The novel Carcinoma-related signaling protein phosphorylation sites disclosed herein now enable the production of corresponding heavy-isotope labeled peptides for the absolute quantification of such signaling proteins (both phosphorylated and not phosphorylated at a disclosed site) in biological samples. The production and use of AQUA peptides for the absolute quantification of proteins (AQUA) in complex mixtures has been described. *See* WO/03016861, "Absolute Quantification of Proteins and Modified Forms Thereof by Multistage Mass Spectrometry," Gygi *et al. and also* Gerber et al. Proc. Natl. Acad. Sci. U.S.A. 100: 6940-5 (2003) (the teachings of which are hereby incorporated herein by reference, in their entirety).

The AQUA methodology employs the introduction of a known quantity of at least one heavy-isotope labeled peptide standard (which has a unique signature detectable by LC-SRM chromatography) into a digested biological sample in order to determine, by comparison to the peptide standard, the absolute quantity of a peptide with the same sequence and protein modification in the biological sample. Briefly, the AQUA methodology has two stages: peptide internal standard selection and validation and method development; and implementation using validated peptide internal standards to detect and quantify a target protein in sample. The method is a powerful technique for detecting and quantifying a given peptide/protein within a complex biological mixture, such as a cell lysate, and may be employed, *e.g.,* to quantify change in protein phosphorylation as a result of drug treatment, or to quantify differences in the level of a protein in different biological states.

Generally, to develop a suitable internal standard, a particular peptide (or modified peptide) within a target protein sequence is chosen based on its amino acid sequence and the particular protease to be used to digest. The peptide is then generated by solid-phase peptide synthesis such that one residue is replaced with that same residue containing stable isotopes (¹³C, ¹⁵N). The result is a peptide that is chemically identical to its native counterpart formed by proteolysis, but is easily distinguishable by MS via a mass shift. A newly synthesized AQUA internal standard peptide is then evaluated by LC-MS/MS. This process provides qualitative information about peptide retention by reverse-phase chromatography, ionization efficiency, and fragmentation via collision-induced dissociation. Informative and abundant fragment ions for sets of native and internal standard peptides are chosen and then specifically monitored in rapid succession as a function of chromatographic retention to form a selected reaction monitoring (LC-SRM) method based on the unique profile of the peptide standard.

The second stage of the AQUA strategy is its implementation to measure the amount of a protein or modified protein from complex mixtures. Whole cell lysates are typically fractionated by SDS-PAGE gel electrophoresis, and regions of the gel consistent with protein migration are excised. This process is followed by in-gel proteolysis in the presence of the AQUA peptides and LC-SRM analysis. (*See* Gerber *et al. supra.*) AQUA peptides are spiked in to the complex peptide mixture obtained by digestion of the whole cell lysate with a proteolytic enzyme and subjected to immunoaffinity purification as described above. The retention time and fragmentation pattern of the native peptide formed by digestion (e.g. trypsinization) is identical to that of the AQUA internal standard peptide determined previously; thus, LC-MS/MS analysis using an SRM experiment results in the highly specific and sensitive measurement of both internal standard and analyte directly from extremely complex peptide mixtures. Because an absolute amount of the AQUA peptide is added (*e.g.* 250 fmol), the ratio of the areas under the curve can be used to determine the precise expression levels of a protein or phosphorylated form of a protein in the original cell lysate. In addition, the internal standard is present during in-gel digestion as native peptides are formed, such that peptide extraction efficiency from gel pieces, absolute losses during sample handling (including vacuum centrifugation), and variability during introduction into the LC-MS system do not affect the determined ratio of native and AQUA peptide abundances.

An AQUA peptide standard is developed for a known phosphorylation site sequence previously identified by the IAP-LC-MS/MS method within a target protein. One AQUA peptide incorporating the phosphorylated form of the particular residue within the site may be developed, and a second AQUA peptide incorporating the non-phosphorylated form of the residue developed. In this way, the two standards may be used to detect and quantify both the phosphorylated and non-phosphorylated forms of the site in a biological sample.

Peptide internal standards may also be generated by examining the primary amino acid sequence of a protein and determining the boundaries of peptides produced by protease cleavage. Alternatively, a protein may actually be digested with a protease and a particular peptide fragment produced can then sequenced. Suitable proteases include, but are not limited to, serine proteases (*e.g.* trypsin, hepsin), metallo proteases (*e.g.* PUMP1), chymotrypsin, cathepsin, pepsin, thermolysin, carboxypeptidases, etc.

A peptide sequence within a target protein is selected according to one or more criteria to optimize the use of the peptide as an internal standard. Preferably, the size of the peptide is selected to minimize the chances that the peptide sequence will be repeated elsewhere in other non-target proteins. Thus, a peptide is preferably at least about 6 amino acids. The size of the peptide is also optimized to maximize ionization frequency. Thus, peptides longer than about 20 amino acids are not preferred. The preferred ranged is about 7 to 15 amino acids. A peptide sequence is also selected that is not likely to be chemically reactive during mass spectrometry, thus sequences comprising cysteine, tryptophan, or methionine are avoided.

A peptide sequence that does not include a modified region of the target region may be selected so that the peptide internal standard can be used to determine the quantity of all forms of the protein. Alternatively, a peptide internal standard encompassing a modified amino acid may be desirable to detect and quantify only the modified form of the target protein. Peptide standards for both modified and unmodified regions can be used together, to determine the extent of a modification in a particular sample (*i.e*. to determine what fraction of the total amount of protein is represented by the modified form). For example, peptide standards for both the phosphorylated and unphosphorylated form of a protein known to be phosphorylated at a particular site can be used to quantify the amount of phosphorylated form in a sample.

The peptide is labeled using one or more labeled amino acids (*i.e*. the label is an actual part of the peptide) or less preferably, labels may be attached after synthesis according to standard methods. Preferably, the label is a mass-altering label selected based on the following considerations: The mass should be unique to shift fragment masses produced by MS analysis to regions of the spectrum with low background; the ion mass signature component is the portion of the labeling moiety that preferably exhibits a unique ion mass signature in MS analysis; the sum of the masses of the constituent atoms of the label is preferably uniquely different than the fragments of all the possible amino acids. As a result, the labeled amino acids and peptides are readily distinguished from unlabeled ones by the ion/mass pattern in the resulting mass spectrum. Preferably, the ion mass signature component imparts a mass to a protein fragment that does not match the residue mass for any of the 20 natural amino acids.

The label should be robust under the fragmentation conditions of MS and not undergo unfavorable fragmentation. Labeling chemistry should be efficient under a range of conditions, particularly denaturing conditions, and the labeled tag preferably remains soluble in the MS buffer system of choice. The label preferably does not suppress the ionization efficiency of the protein and is not chemically reactive. The label may contain a mixture of two or more isotopically distinct species to generate a unique mass spectrometric pattern at each labeled fragment position. Stable isotopes, such as ¹³C, ¹⁵N, ¹⁷O, ¹⁸O, or ³⁴S, are among preferred labels. Pairs of peptide internal standards that incorporate a different isotope label may also be prepared. Preferred amino acid residues into which a heavy isotope label may be incorporated include leucine, proline, valine, and phenylalanine.

Peptide internal standards are characterized according to their mass-to-charge (m/z) ratio, and preferably, also according to their retention time on a chromatographic column (*e.g.* an HPLC column). Internal standards that co-elute with unlabeled peptides of identical sequence are selected as optimal internal standards. The internal standard is then analyzed by fragmenting the peptide by any suitable means, for example by collision-induced dissociation (CID) using, e.g., argon or helium as a collision gas. The fragments are then analyzed, for example by multi-stage mass spectrometry (MSⁿ) to obtain a fragment ion spectrum, to obtain a peptide fragmentation signature. Preferably, peptide fragments have significant differences in m/z ratios to enable peaks corresponding to each fragment to be well separated, and a signature that is unique for the target peptide is obtained. If a suitable fragment signature is not obtained at the first stage, additional stages of MS are performed until a unique signature is obtained.

Fragment ions in the MS/MS and MS³ spectra are typically highly specific for the peptide of interest, and, in conjunction with LC methods, allow a highly selective means of detecting and quantifying a target peptide/protein in a complex protein mixture, such as a cell lysate, containing many thousands or tens of thousands of proteins. Any biological sample potentially containing a target protein/peptide of interest may be assayed. Crude or partially purified cell extracts are preferably employed. Generally, the sample has at least 0.01 mg of protein, typically a concentration of 0.1-10 mg/mL, and may be adjusted to a desired buffer concentration and pH.

A known amount of a labeled peptide internal standard, preferably about 10 femtomoles, corresponding to a target protein to be detected/quantified is then added to a biological sample, such as a cell lysate. The spiked sample is then digested with one or more protease(s) for a suitable time period to allow digestion. A separation is then performed (e.g. by HPLC, reverse-phase HPLC, capillary electrophoresis, ion exchange chromatography, etc.) to isolate the labeled internal standard and its corresponding target peptide from other peptides in the sample. Microcapillary LC is a preferred method.

Each isolated peptide is then examined by monitoring of a selected reaction in the MS. This involves using the prior knowledge gained by the characterization of the peptide internal standard and then requiring the MS to continuously monitor a specific ion in the MS/MS or MSⁿ spectrum for both the peptide of interest and the internal standard. After elution, the area under the curve (AUC) for both peptide standard and target peptide peaks are calculated. The ratio of the two areas provides the absolute quantification that can be normalized for the number of cells used in the analysis and the protein's molecular weight, to provide the precise number of copies of the protein per cell. Further details of the AQUA methodology are described in Gygi *et al.,* and Gerber *et al. supra.*

In accordance with the present invention, AQUA internal peptide standards (heavy-isotope labeled peptides) may now be produced, as described above, for any of the nearly 474 novel Carcinoma-related signaling protein phosphorylation sites disclosed herein (see Table 1/Figure 2). Peptide standards for a given phosphorylation site (*e.g.* the tyrosine 40 site in INPP5D kinase- see Row 414 of Table 1) may be produced for both the phosphorylated and non-phosphorylated forms of the site (*e.g*. see INPP5D site sequence in Column E, Row 414 of Table 1 (SEQ ID NO: 413)) and such standards employed in the AQUA methodology to detect and quantify both forms of such phosphorylation site in a biological sample.

AQUA peptides of the invention may comprise all, or part of, a phosphorylation site peptide sequence disclosed herein (see Column E of Table 1/Figure 2). In a preferred embodiment, an AQUA peptide of the invention consists of, or comprises, a phosphorylation site sequence disclosed herein in Table 1/Figure 2. For example, an AQUA peptide of the invention for detection/quantification of FGFR1 kinase when phosphorylated at tyrosine 397 may consist of, or comprise, the sequence PAVMTSPLYLEIIIYCTGAFLISCMVGSVIVyK (y=phosphotyrosine), which comprises phosphorylatable tyrosine 397 (see Row 360, Column E; (SEQ ID NO: 359)). Heavy-isotope labeled equivalents of the peptides enumerated in Table 1/Figure 2 (both in phosphorylated and unphosphorylated form) can be readily synthesized and their unique MS and LC-SRM signature determined, so that the peptides are validated as AQUA peptides and ready for use in quantification experiments.

The phosphorylation site peptide sequences disclosed herein (see Column E of Table 1/Figure 2) are particularly well suited for development of corresponding AQUA peptides, since the IAP method by which they were identified (see Part A above and Example 1) inherently confirmed that such peptides are in fact produced by enzymatic digestion (trypsinization) and are in fact suitably fractionated/ionized in MS/MS. Thus, heavy-isotope labeled equivalents of these peptides (both in phosphorylated and unphosphorylated form) can be readily synthesized and their unique MS and LC-SRM signature determined, so that the peptides are validated as AQUA peptides and ready for use in quantification experiments.

Accordingly, the invention provides heavy-isotope labeled peptides (AQUA peptides) for the detection and/or quantification of any of the Carcinoma-related phosphorylation sites disclosed in Table 1/Figure 2 (see Column E) and/or their corresponding parent proteins/polypeptides (see Column A). A phosphopeptide sequence consisting of, or comprising, any of the phosphorylation sequences listed in Table 1 may be considered a preferred AQUA peptide of the invention. For example, an AQUA peptide comprising the sequence LGGGTyGEVFKARDKVSGDL**V**ALK (SEQ ID NO: 317) (where y may be either phosphotyrosine or tyrosine, and where **V** = labeled valine (*e.g.* ¹⁴C)) is provided for the quantification of phosphorylated (or non-phosphorylated) kinase (Tyr 28) in a biological sample (see Row 318 of Table 1, tyrosine 28 being the phosphorylatable residue within the site). However, it will be appreciated that a larger AQUA peptide comprising a disclosed phosphorylation site sequence (and additional residues downstream or upstream of it) may also be constructed. Similarly, a smaller AQUA peptide comprising less than all of the residues of a disclosed phosphorylation site sequence (but still comprising the phosphorylatable residue enumerated in Column D of Table 1/Figure 2) may alternatively be constructed. Such larger or shorter AQUA peptides are within the scope of the present invention, and the selection and production of preferred AQUA peptides may be carried out as described above (see Gygi *et al.,* Gerber *et al. supra.*).

Certain particularly preferred subsets of AQUA peptides provided by the invention are described above (corresponding to particular protein types/groups in Table 1, for example, Kinases or Adaptor/Scaffold proteins). Example 4 is provided to further illustrate the construction and use, by standard methods described above, of exemplary AQUA peptides provided by the invention. For example, the above-described AQUA peptides corresponding to the both the phosphorylated and non-phosphorylated forms of the disclosed MAP4K1 kinase tyrosine 28 phosphorylation site (see Row 318 of Table 1/Figure 2) may be used to quantify the amount of phosphorylated MAP4K1 (Tyr 28) in a biological sample, *e.g.* a tumor cell sample (or a sample before or after treatment with a test drug).

AQUA peptides of the invention may also be employed within a kit that comprises one or multiple AQUA peptide(s) provided herein (for the quantification of a Carcinoma-related signal transduction protein disclosed in Table 1/Figure 2), and, optionally, a second detecting reagent conjugated to a detectable group. For example, a kit may include AQUA peptides for both the phosphorylated and non-phosphorylated form of a phosphorylation site disclosed herein. The reagents may also include ancillary agents such as buffering agents and protein stabilizing agents, *e.g.,* polysaccharides and the like. The kit may further include, where necessary, other members of the signal-producing system of which system the detectable group is a member (*e.g.,* enzyme substrates), agents for reducing background interference in a test, control reagents, apparatus for conducting a test, and the like. The test kit may be packaged in any suitable manner, typically with all elements in a single container along with a sheet of printed instructions for carrying out the test.

AQUA peptides provided by the invention will be highly useful in the further study of signal transduction anomalies underlying cancer, including carcinomas, and in identifying diagnostic/bio-markers of these diseases, new potential drug targets, and/or in monitoring the effects of test compounds on Carcinoma-related signal transduction proteins and pathways.

### D. Immunoassay Formats

Antibodies provided by the invention may be advantageously employed in a variety of standard immunological assays (the use of AQUA peptides provided by the invention is described separately above). Assays may be homogeneous assays or heterogeneous assays. In a homogeneous assay the immunological reaction usually involves a phosphorylation-site specific antibody of the invention), a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels that may be employed include free radicals, radioisotopes, fluorescent dyes, enzymes, bacteriophages, coenzymes, and so forth.

In a heterogeneous assay approach, the reagents are usually the specimen, a phosphorylation-site specific antibody of the invention, and suitable means for producing a detectable signal. Similar specimens as described above may be used. The antibody is generally immobilized on a support, such as a bead, plate or slide, and contacted with the specimen suspected of containing the antigen in a liquid phase. The support is then separated from the liquid phase and either the support phase or the liquid phase is examined for a detectable signal employing means for producing such signal. The signal is related to the presence of the analyte in the specimen. Means for producing a detectable signal include the use of radioactive labels, fluorescent labels, enzyme labels, and so forth. For example, if the antigen to be detected contains a second binding site, an antibody which binds to that site can be conjugated to a detectable group and added to the liquid phase reaction solution before the separation step. The presence of the detectable group on the solid support indicates the presence of the antigen in the test sample. Examples of suitable immunoassays are the radioimmunoassay, immunofluorescence methods, enzyme-linked immunoassays, and the like.

Immunoassay formats and variations thereof that may be useful for carrying out the methods disclosed herein are well known in the art. *See generally* E. Maggio, Enzyme-Immunoassay, (1980) (CRC Press, Inc., Boca Raton, Fla.); *see also, e.g.,* U.S. Pat. No. 4,727,022 (Skold et al.*,* "Methods for Modulating Ligand-Receptor Interactions and their Application"); U.S. Pat. No. 4,659,678 (Forrest et al.*,* "Immunoassay of Antigens"); U.S. Pat. No. 4,376,110 (David et al.*,* "Immunometric Assays Using Monoclonal Antibodies"). Conditions suitable for the formation of antigen-antibody complexes are well described. *See id.* Monoclonal antibodies of the invention may be used in a "two-site" or "sandwich" assay, with a single cell line serving as a source for both the labeled monoclonal antibody and the bound monoclonal antibody. Such assays are described in U.S. Pat. No. 4,376,110. The concentration of detectable reagent should be sufficient such that the binding of a target Carcinoma-related signal transduction protein is detectable compared to background.

Phosphorylation site-specific antibodies disclosed herein may be conjugated to a solid support suitable for a diagnostic assay (*e.g.,* beads, plates, slides or wells formed from materials such as latex or polystyrene) in accordance with known techniques, such as precipitation. Antibodies, or other target protein or target site-binding reagents, may likewise be conjugated to detectable groups such as radiolabels (*e.g*., ³⁵S, ¹²⁵I, ¹³¹I), enzyme labels (*e.g.,* horseradish peroxidase, alkaline phosphatase), and fluorescent labels (*e.g.*, fluorescein) in accordance with known techniques.

Antibodies of the invention may also be optimized for use in a flow cytometry (FC) assay to determine the activation/phosphorylation status of a target Carcinoma-related signal transduction protein in patients before, during, and after treatment with a drug targeted at inhibiting phosphorylation at such a protein at the phosphorylation site disclosed herein. For example, bone marrow cells or peripheral blood cells from patients may be analyzed by flow cytometry for target Carcinoma-related signal transduction protein phosphorylation, as well as for markers identifying various hematopoietic cell types. In this manner, activation status of the malignant cells may be specifically characterized. Flow cytometry may be carried out according to standard methods. *See, e.g.* Chow et al., Cytometry (Communications in Clinical Cytometry) 46: 72-78 (2001). Briefly and by way of example, the following protocol for cytometric analysis may be employed: fixation of the cells with 1% paraformaldehyde for 10 minutes at 37 °C followed by permeabilization in 90% methanol for 30 minutes on ice. Cells may then be stained with the primary antibody (a phospho-specific antibody of the invention), washed and labeled with a fluorescent-labeled secondary antibody. Alternatively, the cells may be stained with a fluorescent-labeled primary antibody. The cells would then be analyzed on a flow cytometer (*e.g*. a Beckman Coulter EPICS-XL) according to the specific protocols of the instrument used. Such an analysis would identify the presence of activated Carcinoma-related signal transduction protein(s) in the malignant cells and reveal the drug response on the targeted protein.

Alternatively, antibodies of the invention may be employed in immunohistochemical (IHC) staining to detect differences in signal transduction or protein activity using normal and diseased tissues. IHC may be carried out according to well-known techniques. *See, e*.*g*., ANTIBODIES: A LABORATORY MANUAL, supra. Briefly, paraffin-embedded tissue (*e.g.* tumor tissue) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody and secondary antibody; and finally detecting using ABC avidin/biotin method according to manufacturer's instructions.

Antibodies of the invention may be also be optimized for use in other clinically-suitable applications, for example bead-based multiplex-type assays, such as IGEN, Luminex™ and/or Bioplex™ assay formats, or otherwise optimized for antibody arrays formats, such as reversed-phase array applications (*see, e.g.* Paweletz et al., Oncogene 20(16): 1981-89 (2001)). Accordingly, in another embodiment, the invention provides a method for the multiplex detection of Carcinoma-related protein phosphorylation in a biological sample, the method comprising utilizing two or more antibodies or AQUA peptides of the invention to detect the presence of two or more phosphorylated Carcinoma-related signaling proteins enumerated in Column A of Table 1/Figure 2. In one preferred embodiment, two to five antibodies or AQUA peptides of the invention are employed in the method. In another preferred embodiment, six to ten antibodies or AQUA peptides of the invention are employed, while in another preferred embodiment eleven to twenty such reagents are employed.

Antibodies and/or AQUA peptides of the invention may also be employed within a kit that comprises at least one phosphorylation site-specific antibody or AQUA peptide of the invention (which binds to or detects a Carcinoma-related signal transduction protein disclosed in Table 1/Figure 2), and, optionally, a second antibody conjugated to a detectable group. In some embodies, the kit is suitable for multiplex assays and comprises two or more antibodies or AQUA peptides of the invention, and in some embodiments, comprises two to five, six to ten, or eleven to twenty reagents of the invention. The kit may also include ancillary agents such as buffering agents and protein stabilizing agents, *e.g.*, polysaccharides and the like. The kit may further include, where necessary, other members of the signal-producing system of which system the detectable group is a member (*e.g.*, enzyme substrates), agents for reducing background interference in a test, control reagents, apparatus for conducting a test, and the like. The test kit may be packaged in any suitable manner, typically with all elements in a single container along with a sheet of printed instructions for carrying out the test.

The following Examples are provided only to further illustrate the invention, and are not intended to limit its scope, except as provided in the claims appended hereto. The present invention encompasses modifications and variations of the methods taught herein which would be obvious to one of ordinary skill in the art.

### EXAMPLE 1

### Isolation of Phosphotyrosine-Containing Peptides from Extracts of Carcinoma Cell Lines and Identification of Novel Phosphorylation Sites.

In order to discover previously unknown Carcinoma-related signal transduction protein phosphorylation sites, IAP isolation techniques were employed to identify phosphotyrosine-containing peptides in cell extracts from human carcinoma cell lines and patient cell lines identified in Column G of Table 1 including Su-DHL1, MOLT15, H1703, 3T3-src, 3T3, Abl, A431, pancreatic xenograft, H1993, HCC827, 3T3-EGFRwt, 3T3-EGFR(L858R), HCT 116; HT29, NCl-N87, HT29, CTV-1, Karpas 299, MCF-10A (Y561F), MCF-10A (Y969F), Calu-3, H2347, H3255, H2170, U118MG, H1703, HCC366, H2228, HL61 b, jurkat, SUPT-13, Verona patient 4, PT9, DU145, DMS79, MDA-MB-468, A549, H1666, H1650, 831/13, K562, HL53B, HL66B, HL84B, HL87A, HPAC, H441, SEM, Sor4, SorA, SEM, TgOVA, UT-7, MKPL-1, H69 LS, A431, DMS153 NS, SW620, HT116, MDA-MB-468, MCF10, HPAC, and HT29. Tryptic phosphotyrosine-containing peptides were purified and analyzed from extracts of each of the cell lines mentioned above, as follows. Cells were cultured in DMEM medium or RPMI 1640 medium supplemented with 10% fetal bovine serum and penicillin/streptomycin.

Suspension cells were harvested by low speed centrifugation. After complete aspiration of medium, cells were resuspended in 1 mL lysis buffer per 1.25 x 10⁸ cells (20 mM HEPES pH 8.0, 9 M urea, 1 mM sodium vanadate, supplemented or not with 2.5 mM sodium pyrophosphate, 1 mM β-glycerol-phosphate) and sonicated.

Adherent cells at about 80% confluency were starved in medium without serum overnight and stimulated, with ligand depending on the cell type or not stimulated. After complete aspiration of medium from the plates, cells were scraped off the plate in 10 ml lysis buffer per 2 x 10⁸ cells (20 mM HEPES pH 8.0, 9 M urea, 1 mM sodium vanadate, supplemented with 2.5 mM sodium pyrophosphate, 1 mM β-glycerol-phosphate) and sonicated.

Sonicated cell lysates were cleared by centrifugation at 20,000 x g, and proteins were reduced with DTT at a final concentration of 4.1 mM and alkylated with iodoacetamide at 8.3 mM. For digestion with trypsin, protein extracts were diluted in 20 mM HEPES pH 8.0 to a final concentration of 2 M urea and soluble TLCK-trypsin (Worthington) was added at 10-20 µg/mL. Digestion was performed for 1-2 days at room temperature.

Trifluoroacetic acid (TFA) was added to protein digests to a final concentration of 1%, precipitate was removed by centrifugation, and digests were loaded onto Sep-Pak C₁₈ columns (Waters) equilibrated with 0.1 % TFA. A column volume of 0.7-1.0 ml was used per 2 x 10⁸ cells. Columns were washed with 15 volumes of 0.1% TFA, followed by 4 volumes of 5% acetonitrile (MeCN) in 0.1% TFA. Peptide fraction I was obtained by eluting columns with 2 volumes each of 8, 12, and 15% MeCN in 0.1 % TFA and combining the eluates. Fractions II and III were a combination of eluates after eluting columns with 18, 22, 25% MeCN in 0.1% TFA and with 30, 35, 40% MeCN in 0.1% TFA, respectively. All peptide fractions were lyophilized.

Peptides from each fraction corresponding to 2 x 10⁸ cells were dissolved in 1 ml of IAP buffer (20 mM Tris/HCl or 50 mM MOPS pH 7.2, 10 mM sodium phosphate, 50 mM NaCl) and insoluble matter (mainly in peptide fractions III) was removed by centrifugation. IAP was performed on each peptide fraction separately. The phosphotyrosine monoclonal antibody P-Tyr-100 (Cell Signaling Technology, Inc., catalog number 9411) was coupled at 4 mg/ml beads to protein G (Roche), respectively. Immobilized antibody (15 µl, 60 µg) was added as 1:1 slurry in IAP buffer to 1 ml of each peptide fraction, and the mixture was incubated overnight at 4° C with gentle rotation. The immobilized antibody beads were washed three times with 1 ml IAP buffer and twice with 1 ml water, all at 4° C. Peptides were eluted from beads by incubation with 75 µl of 0.1 % TFA at room temperature for 10 minutes.

Alternatively, one single peptide fraction was obtained from Sep-Pak C18 columns by elution with 2 volumes each of 10%, 15%, 20 %, 25 %, 30 %, 35 % and 40 % acetonitirile in 0.1% TFA and combination of all eluates. IAP on this peptide fraction was performed as follows: After lyophilization, peptide was dissolved in 50 ml IAP buffer (MOPS pH 7.2, 10 mM sodium phosphate, 50 mM NaCl) and insoluble matter was removed by centrifugation. Immobilized antibody (40 µl, 160 µg) was added as 1:1 slurry in IAP buffer, and the mixture was incubated overnight at 4° C with gentle shaking. The immobilized antibody beads were washed three times with 1 ml IAP buffer and twice with 1 ml water, all at 4° C. Peptides were eluted from beads by incubation with 55 µl of 0.15% TFA at room temperature for 10 min (eluate 1), followed by a wash of the beads (eluate 2) with 45 µl of 0.15% TFA. Both eluates were combined.

### Analysis by LC-MS/MS Mass Spectrometry.

40 µl or more of IAP eluate were purified by 0.2 µl StageTips or ZipTips. Peptides were eluted from the microcolumns with 1 µl of 40% MeCN, 0.1% TFA (fractions I and II) or 1 µl of 60% MeCN, 0.1% TFA (fraction III) into 7.6-9.0 µl of 0.4% acetic acid/0.005% heptafluorobutyric acid. For single fraction analysis, 1 µl of 60% MeCN, 0.1% TFA, was used for elution from the microcolumns. This sample was loaded onto a 10 cm x 75 µm PicoFrit capillary column (New Objective) packed with Magic C18 AQ reversed-phase resin (Michrom Bioresources) using a Famos autosampler with an inert sample injection valve (Dionex). The column was then developed with a 45-min linear gradient of acetonitrile delivered at 200 nl/min (Ultimate, Dionex), and tandem mass spectra were collected in a data-dependent manner with an LTQ ion trap mass spectrometer essentially as described by Gygi *et al., supra.*

### Database Analysis & Assignments.

MS/MS spectra were evaluated using TurboSequest in the Sequest Browser package (v. 27, rev. 12) supplied as part of BioWorks 3.0 (ThermoFinnigan). Individual MS/MS spectra were extracted from the raw data file using the Sequest Browser program CreateDta, with the following settings: bottom MW, 700; top MW, 4,500; minimum number of ions, 20; minimum TIC, 4 x 10⁵; and precursor charge state, unspecified. Spectra were extracted from the beginning of the raw data file before sample injection to the end of the eluting gradient. The IonQuest and VuDta programs were not used to further select MS/MS spectra for Sequest analysis. MS/MS spectra were evaluated with the following TurboSequest parameters: peptide mass tolerance, 2.5; fragment ion tolerance, 0.0; maximum number of differential amino acids per modification, 4; mass type parent, average; mass type fragment, average; maximum number of internal cleavage sites, 10; neutral losses of water and ammonia from b and y ions were considered in the correlation analysis. Proteolytic enzyme was specified except for spectra collected from elastase digests.

Searches were performed against the NCBI human protein database (NCBI RefSeq protein release #11; 8 May 2005; 1,826,611 proteins, including 47,859 human proteins. Peptides that did not match RefSeq were compared to NCBI GenPept release #148; 15 June 2005 release date; 2,479,172 proteins, including 196,054 human proteins.). Cysteine carboxamidomethylation was specified as a static modification, and phosphorylation was allowed as a variable modification on serine, threonine, and tyrosine residues or on tyrosine residues alone. It was determined that restricting phosphorylation to tyrosine residues had little effect on the number of phosphorylation sites assigned. Furthermore, it should be noted that certain peptides were originally isolated in mouse and later normalized to human sequences as shown by Table1/Figure2.

In proteomics research, it is desirable to validate protein identifications based solely on the observation of a single peptide in one experimental result, in order to indicate that the protein is, in fact, present in a sample. This has led to the development of statistical methods for validating peptide assignments, which are not yet universally accepted, and guidelines for the publication of protein and peptide identification results (*see* Carr et al., Mol. Cell Proteomics 3: 531-533 (2004)), which were followed in this Example. However, because the immunoaffinity strategy separates phosphorylated peptides from unphosphorylated peptides, observing just one phosphopeptide from a protein is a common result, since many phosphorylated proteins have only one tyrosine-phosphorylated site. For this reason, it is appropriate to use additional criteria to validate phosphopeptide assignments. Assignments are likely to be correct if any of these additional criteria are met: (i) the same sequence is assigned to co-eluting ions with different charge states, since the MS/MS spectrum changes markedly with charge state; (ii) the site is found in more than one peptide sequence context due to sequence overlaps from incomplete proteolysis or use of proteases other than trypsin; (iii) the site is found in more than one peptide sequence context due to homologous but not identical protein isoforms; (iv) the site is found in more than one peptide sequence context due to homologous but not identical proteins among species; and (v) sites validated by MS/MS analysis of synthetic phosphopeptides corresponding to assigned sequences, since the ion trap mass spectrometer produces highly reproducible MS/MS spectra. The last criterion is routinely employed to confirm novel site assignments of particular interest.

All spectra and all sequence assignments made by Sequest were imported into a relational database. The following Sequest scoring thresholds were used to select phosphopeptide assignments that are likely to be correct: RSp < 6, XCorr ≥ 2.2, and DeltaCN > 0.099. Further, the sequence assignments could be accepted or rejected with respect to accuracy by using the following conservative, two-step process.

In the first step, a subset of high-scoring sequence assignments should be selected by filtering for XCorr values of at least 1.5 for a charge state of +1, 2.2 for +2, and 3.3 for +3, allowing a maximum RSp value of 10. Assignments in this subset should be rejected if any of the following criteria are satisfied: (i) the spectrum contains at least one major peak (at least 10% as intense as the most intense ion in the spectrum) that can not be mapped to the assigned sequence as an *a*, *b*, or *y* ion, as an ion arising from neutral-loss of water or ammonia from a b or y ion, or as a multiply protonated ion; (ii) the spectrum does not contain a series of b or y ions equivalent to at least six uninterrupted residues; or (iii) the sequence is not observed at least five times in all the studies conducted (except for overlapping sequences due to incomplete proteolysis or use of proteases other than trypsin).

In the second step, assignments with below-threshold scores should be accepted if the low-scoring spectrum shows a high degree of similarity to a high-scoring spectrum collected in another study, which simulates a true reference library-searching strategy.

### EXAMPLE 2

### Production of Phospho-specific Polyclonal Antibodies for the Detection of Carcinoma-related Signaling Protein Phosphorylation

Polyclonal antibodies that specifically bind a Carcinoma-related signal transduction protein only when phosphorylated at the respective phosphorylation site disclosed herein (see Table 1/Figure 2) are produced according to standard methods by first constructing a synthetic peptide antigen comprising the phosphorylation site sequence and then immunizing an animal to raise antibodies against the antigen, as further described below. Production of exemplary polyclonal antibodies is provided below.

### A. JAK3 (tyrosine 929).

A 24 amino acid phospho-peptide antigen, LDASRLLLy*SSQICKGMEYLGSRR (where y*= phosphotyrosine) that corresponds to the sequence encompassing the tyrosine 929 phosphorylation site in human JAK3 kinase (see Row 341 of Table 1; SEQ ID NO: 340), plus cysteine on the C-terminal for coupling, is constructed according to standard synthesis techniques using, *e.g.*, a Rainin/Protein Technologies, Inc., Symphony peptide synthesizer. See ANTIBODIES: A LABORATORY MANUAL, *supra.;* Merrifield, *supra.* This peptide is then' coupled to KLH and used to immunize animals to produce (and subsequently screen) phospho-specific JAK3 (tyr 929) polyclonal antibodies as described in Immunization/Screening below.

### B. SPRY1 (tyrosine 53).

A 13 amino acid phospho-peptide antigen, GSNEy*TEGPSVVK (where y*= phosphotyrosine) that corresponds to the sequence encompassing the tyrosine 53 phosphorylation site in human SPRY1 (see Row 75 of Table 1 (SEQ ID NO: 74)), plus cysteine on the C-terminal for coupling, is constructed according to standard synthesis techniques using, *e.g.*, a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals to produce (and subsequently screen) phospho-specific SPRY1 (tyr 53) polyclonal antibodies as described in Immunization/Screening below.

### C. INPP5D (tyrosine 40).

A 16 amino acid phospho-peptide antigen, ASESISRAy*ALCVLYR (where y*= phosphotyrosine) that corresponds to the sequence encompassing the tyrosine 40 phosphorylation site in human INPP5D protein (*see* Row 414 of Table 1 (SEQ ID NO: 413), plus cysteine on the C-terminal for coupling, is constructed according to standard synthesis techniques using, *e.g.*, a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals to produce (and subsequently screen) phospho-specific INPP5D (tyr 40) antibodies as described in Immunization/Screening below.

### Immunization/Screening.

A synthetic phospho-peptide antigen as described in A-C above is coupled to KLH, and rabbits are injected intradermally (ID) on the back with antigen in complete Freunds adjuvant (500 µg antigen per rabbit). The rabbits are boosted with same antigen in incomplete Freund adjuvant (250 µg antigen per rabbit) every three weeks. After the fifth boost, bleeds are collected. The sera are purified by Protein A-affinity chromatography by standard methods (see ANTIBODIES: A LABORATORY MANUAL, Cold Spring Harbor, *supra.).* The eluted immunoglobulins are further loaded onto a non-phosphorylated synthetic peptide antigen-resin Knotes column to pull out antibodies that bind the non-phosphorylated form of the phosphorylation site. The flow through fraction is collected and applied onto a phospho-synthetic peptide antigen-resin column to isolate antibodies that bind the phosphorylated form of the site. After washing the column extensively, the bound antibodies (*i.e*. antibodies that bind a phosphorylated peptide described in A-C above, but do not bind the non-phosphorylated form of the peptide) are eluted and kept in antibody storage buffer.

The isolated antibody is then tested for phospho-specificity using Western blot assay using an appropriate cell line that expresses (or overexpresses) target phospho-protein (*i.e*. phosphorylated JAK3, SPRY1 or INPP5D), for example, A431, and A549, respectively. Cells are cultured in DMEM or RPMI supplemented with 10% FCS. Cell are collected, washed with PBS and directly lysed in cell lysis buffer. The protein concentration of cell lysates is then measured. The loading buffer is added into cell lysate and the mixture is boiled at 100 °C for 5 minutes. 20 µl (10 µg protein) of sample is then added onto 7.5% SDS-PAGE gel.

A standard Western blot may be performed according to the Immunoblotting Protocol set out in the CELL SIGNALING TECHNOLOGY, INC. 2003-04 Catalogue, p. 390. The isolated phospho-specific antibody is used at dilution 1:1000. Phosphorylation-site specificity of the antibody will be shown by binding of only the phosphorylated form of the target protein. Isolated phospho-specific polyclonal antibody does not (substantially) recognize the target protein when not phosphorylated at the appropriate phosphorylation site in the non-stimulated cells (*e.g.* JAK3 is not bound when not phosphorylated at tyrosine 929).

In order to confirm the specificity of the isolated antibody, different cell lysates containing various phosphorylated signal transduction proteins other than the target protein are prepared. The Western blot assay is performed again using these cell lysates. The phospho-specific polyclonal antibody isolated as described above is used (1:1000 dilution) to test reactivity with the different phosphorylated non-target proteins on Western blot membrane. The phospho-specific antibody does not significantly cross-react with other phosphorylated signal transduction proteins, although occasionally slight binding with a highly homologous phosphorylation-site on another protein may be observed. In such case the antibody may be further purified using affinity chromatography, or the specific immunoreactivity cloned by rabbit hybridoma technology.

### EXAMPLE 3

### Production of Phospho-specific Monoclonal Antibodies for the Detection of Carcinoma-related Signaling Protein Phosphorylation

Monoclonal antibodies that specifically bind a Carcinoma-related signal transduction protein only when phosphorylated at the respective phosphorylation site disclosed herein (see Table 1/Figure 2) are produced according to standard methods by first constructing a synthetic peptide antigen comprising the phosphorylation site sequence and then immunizing an animal to raise antibodies against the antigen, and harvesting spleen cells from such animals to produce fusion hybridomas, as further described below. Production of exemplary monoclonal antibodies is provided below.

### A. RAN (tyrosine 155).

An 14 amino acid phospho-peptide antigen, SNy*NFEKPFLWLAR (where y*= phosphotyrosine) that corresponds to the sequence encompassing the tyrosine 155 phosphorylation site in human RAN phosphatase (see Row 274 of Table 1 (SEQ ID NO: 273)), plus cysteine on the C-terminal for coupling, is constructed according to standard synthesis techniques using, *e.g.*, a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal RAN (tyr 155) antibodies as described in Immunization/Fusion/Screening below.

### B. PLEC1 (tyrosine 4505).

A 18 amino acid phospho-peptide antigen, GYYSPy*SVSGSGSTAGSR (where y*= phosphotyrosine) that corresponds to the sequence encompassing the tyrosine 4505 phosphorylation site in human PLEC1 (see Row 216 of Table 1 (SEQ ID NO: 215)), plus cysteine on the C-terminal for coupling, is constructed according to standard synthesis techniques using, *e.g.,* a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal PLEC1 (tyr 4505) antibodies as described in Immunization/Fusion/Screening below.

### C. PLCB1 (tyrosine 239).

A 15 amino acid phospho-peptide antigen, Py*LTVDQMMDFINLK (where y*= phosphotyrosines) that corresponds to the sequence encompassing the tyrosine 239 phosphorylation site in human PLCB1 protein (see Row 421 of Table 1 (SEQ ID NO: 420)), plus cysteine on the C-terminal for coupling, is constructed according to standard synthesis techniques using, *e.g.,* a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal PLCB1 (tyr 239) antibodies as described in Immunization/Fusion/Screening below.

### Immunization/Fusion/Screening.

A synthetic phospho-peptide antigen as described in A-C above is coupled to KLH, and BALB/C mice are injected intradermally (ID) on the back with antigen in complete Freunds adjuvant (*e.g.* 50 µg antigen per mouse). The mice are boosted with same antigen in incomplete Freund adjuvant (*e.g.* 25 µg antigen per mouse) every three weeks. After the fifth boost, the animals are sacrificed and spleens are harvested.

Harvested spleen cells are fused to SP2/0 mouse myeloma fusion partner cells according to the standard protocol of Kohler and Milstein (1975). Colonies originating from the fusion are screened by ELISA for reactivity to the phospho-peptide and non-phospho-peptide forms of the antigen and by Western blot analysis (as described in Example 1 above). Colonies found to be positive by ELISA to the phospho-peptide while negative to the non-phospho-peptide are further characterized by Western blot analysis. Colonies found to be positive by Western blot analysis are subcloned by limited dilution. Mouse ascites are produced from a single clone obtained from subcloning, and tested for phospho-specificity (against the RAN, PLEC1, or PLCB1) phospho-peptide antigen, as the case may be) on ELISA. Clones identified as positive on Western blot analysis using cell culture supernatant as having phospho-specificity, as indicated by a strong band in the induced lane and a weak band in the uninduced lane of the blot, are isolated and subcloned as clones producing monoclonal antibodies with the desired specificity.

Ascites fluid from isolated clones may be further tested by Western blot analysis. The ascites fluid should produce similar results on Western blot analysis as observed previously with the cell culture supernatant, indicating phospho-specificity against the phosphorylated target *(e.g.* PLCB1 phosphorylated at tyrosine 239).

### EXAMPLE 4

### Production and Use of AQUA Peptides for the Quantification of Carcinoma-related Signaling Protein Phosphorylation

Heavy-isotope labeled peptides (AQUA peptides (internal standards)) for the detection and quantification of a Carcinoma-related signal transduction protein only when phosphorylated at the respective phosphorylation site disclosed herein (*see* Table 1/Figure 2) are produced according to the standard AQUA methodology (*see* Gygi *et al.,* Gerber *et al., supra.*) methods by first constructing a synthetic peptide standard corresponding to the phosphorylation site sequence and incorporating a heavy-isotope label. Subsequently, the MSⁿ and LC-SRM signature of the peptide standard is validated, and the AQUA peptide is used to quantify native peptide in a biological sample, such as a digested cell extract. Production and use of exemplary AQUA peptides is provided below.

### A. PIK3C2B (tyrosine 127).

An AQUA peptide comprising the sequence, GSLSGDy*LYIFDGSDGGVSSSPGPGDIEGSCK (y*= phosphotyrosine; sequence incorporating ¹⁴C/¹⁵N-labeled valine (indicated by bold **V**), which corresponds to the tyrosine 127 phosphorylation site in human PIK3C2B kinase (see Row 303 in Table 1 (SEQ ID NO: 302)), is constructed according to standard synthesis techniques using, *e.g.*, a Rainin/Protein Technologies, Inc., Symphony peptide synthesizer (see Merrifield, *supra.)* as further described below in Synthesis & MS/MS Signature. The Met (tyr 835) AQUA peptide is then spiked into a biological sample to quantify the amount of phosphorylated PIK3C2B (tyr 127) in the sample, as further described below in Analysis & Quantification.

### B. GAB2 (tyrosine 371).

An AQUA peptide comprising the sequence ASSCETyEY**P**QR (y*= phosphotyrosine; sequence incorporating ¹⁴C/¹⁵N-labeled proline (indicated by bold **P**), which corresponds to the tyrosine 371 Phosphorylation site in human GAB2 protein (see Row 52 in Table 1 (SEQ ID NO: 51)), is constructed according to standard synthesis techniques using, *e.g.*, a Rainin/Protein Technologies, Inc., Symphony peptide synthesizer (see Merrifield, *supra.*) as further described below in Synthesis & MS/MS Signature. The GAB2 (tyr 287) AQUA peptide is then spiked into a biological sample to quantify the amount of phosphorylated GAB2 (tyr 371) in the sample, as further described below in Analysis & Quantification.

### C. VIM (tyrosine 38).

An AQUA peptide comprising the sequence, Ty*SLGSALRPSTSR (y*= phosphotyrosine; sequence incorporating ¹⁴C/¹⁵N-labeled Leucine (indicated by bold L), which corresponds to the tyrosine 38 phosphorylation site in human VIMprotein (*see* Row 220 in Table 1 (SEQ ID NO: 219)), is constructed according to standard synthesis techniques using, *e.g.,* a Rainin/Protein Technologies, Inc., Symphony peptide synthesizer (*see* Merrifield, *supra.*) as further described below in Synthesis & MS/MS Signature. The VIM (tyr 38) AQUA peptide is then spiked into a biological sample to quantify the amount of phosphorylated VIM (tyr 38) in the sample, as further described below in Analysis & Quantification.

### D. GPRC5A (tyrosine 350).

An AQUA peptide comprising the sequence AHAWPS**P**YKDyEVK (y*= phosphotyrosine; sequence incorporating ¹⁴C/¹⁵N-labeled proline (indicated by bold **P**), which corresponds to the tyrosine 350 phosphorylation site in human GPRC5A protein (*see* Row 448 in Table 1 (SEQ ID NO: 447)), is constructed according to standard synthesis techniques using, *e.g.,* a Rainin/Protein Technologies, Inc., Symphony peptide synthesizer (*see* Merrifield, *supra.*) as further described below in Synthesis & MS/MS Signature. The GPRC5A (tyr 350) AQUA peptide is then spiked into a biological sample to quantify the amount of phosphorylated GPRC5A (tyr 350) in the sample, as further described below in Analysis & Quantification.

### Synthesis & MS/MS Spectra.

Fluorenylmethoxycarbonyl (Fmoc)-derivatized amino acid monomers may be obtained from AnaSpec (San Jose, CA). Fmoc-derivatized stable-isotope monomers containing one ¹⁵N and five to nine ¹³C atoms may be obtained from Cambridge Isotope Laboratories (Andover, MA). Preloaded Wang resins may be obtained from Applied Biosystems. Synthesis scales may vary from 5 to 25 µmol. Amino acids are activated *in situ* with 1-H-benzotriazolium, 1-bis(dimethylamino) methylene]-hexafluorophosphate (1-),3-oxide:1-hydroxybenzotriazole hydrate and coupled at a 5-fold molar excess over peptide. Each coupling cycle is followed by capping with acetic anhydride to avoid accumulation of one-residue deletion peptide byproducts. After synthesis peptide-resins are treated with a standard scavenger-containing trifluoroacetic acid (TFA)-water cleavage solution, and the peptides are precipitated by addition to cold ether. Peptides (*i.e*. a desired AQUA peptide described in A-D above) are purified by reversed-phase C18 HPLC using standard TFA/acetonitrile gradients and characterized by matrix-assisted laser desorption ionization-time of flight (Biflex III, Bruker Daltonics, Billerica, MA) and ion-trap (ThermoFinnigan, LCQ DecaXP) MS.

MS/MS spectra for each AQUA peptide should exhibit a strong *y*-type ion peak as the most intense fragment ion that is suitable for use in an SRM monitoring/analysis. Reverse-phase microcapillary columns (0.1 Å∼ 150-220 mm) are prepared according to standard methods. An Agilent 1100 liquid chromatograph may be used to develop and deliver a solvent gradient [0.4% acetic acid/0.005% heptafluorobutyric acid (HFBA)/7% methanol and 0.4% acetic acid/0.005% HFBA/65% methanol/35% acetonitrile] to the microcapillary column by means of a flow splitter. Samples are then directly loaded onto the microcapillary column by using a FAMOS inert capillary autosampler (LC Packings, San Francisco) after the flow split. Peptides are reconstituted in 6% acetic acid/0.01% TFA before injection.

### Analysis & Quantification.

Target protein (*e.g.* a phosphorylated protein of A-D above) in a biological sample is quantified using a validated AQUA peptide (as described above). The IAP method is then applied to the complex mixture of peptides derived from proteolytic cleavage of crude cell extracts to which the AQUA peptides have been spiked in.

LC-SRM of the entire sample is then carried out. MS/MS may be performed by using a ThermoFinnigan (San Jose, CA) mass spectrometer (LCQ DecaXP ion trap or TSQ Quantum triple quadrupole). On the DecaXP, parent ions are isolated at 1.6 m/z width, the ion injection time being limited to 150 ms per microscan, with two microscans per peptide averaged, and with an AGC setting of 1 x 10⁸; on the Quantum, Q1 is kept at 0.4 and Q3 at 0.8 m/z with a scan time of 200 ms per peptide. On both instruments, analyte and internal standard are analyzed in alternation within a previously known reverse-phase retention window; well-resolved pairs of internal standard and analyte are analyzed in separate retention segments to improve duty cycle. Data are processed by integrating the appropriate peaks in an extracted ion chromatogram (60.15 m/z from the fragment monitored) for the native and internal standard, followed by calculation of the ratio of peak areas multiplied by the absolute amount of internal standard (*e.g.*, 500 fmol).

## Claims

1. An isolated phosphorylation site-specific antibody that specifically binds a human Carcinoma-related signaling protein selected from Column A of Table 1 only when phosphorylated at the tyrosine listed in corresponding Column D of Table 1, comprised within the phosphorylatable peptide sequence of SEQ ID NO: 360, wherein said antibody does not bind said signaling protein when not phosphorylated at said tyrosine.

2. An isolated phosphorylation site-specific antibody that specifically binds a human Carcinoma-related signaling protein selected from Column A of Table 1 only when not phosphorylated at the tyrosine listed in corresponding Column D of Table 1, comprised within the phosphorylatable peptide sequence of SEQ ID NO: 360, wherein said antibody does not bind said signaling protein when phosphorylated at said tyrosine.

3. A method selected from the group consisting of:
(a) a method for detecting a human leukemia-related signaling protein selected from Column A of Table 1, wherein said human leukemia-related signaling protein is phosphorylated at the tyrosine listed in corresponding Column D of Table 1, comprised within the phosphorylatable peptide sequence of SEQ ID NO: 360, comprising the step of adding an isolated phosphorylation-specific antibody according to claim 2, to a sample comprising said human leukemia-related signaling protein under conditions that permit the binding of said antibody to said human leukemia-related signaling protein, and detecting bound antibody;
(b) a method for quantifying the amount of a human leukemia-related signaling protein listed in Column A of Table 1 that is phosphorylated at the corresponding tyrosine listed in Column D of Table 1, comprised within the phosphorylatable peptide sequence of SEQ ID NO: 360, in a sample using a heavy-isotope labeled peptide (AQUA™ peptide), said labeled peptide comprising a phosphorylated tyrosine at said corresponding tyrosine listed Column D of Table 1, comprised within the phosphorylatable peptide sequence listed in corresponding Column E of Table 1 as an internal standard; and
(c) a method comprising step (a) followed by step (b).


SEQUENCE LISTING

<110> Cell Signalling Technology, Inc

<120> REAGENTS FOR THE DETECTION OF PROTEIN PHOSPHORYLATION
      IN CARCINOMA SIGNALING PATHWAYS

<130> 602188PEP

<140> EP 06814000

<141> 2005 08 31

<150> US 60/713,041

<151> 2005 08 31


<160> 474

<170> PatentIn version 3.3

<210> 1
<211> 13
<212> PRT
<213> Artificial Sequence

<220>
<223> Description of Artificial Sequence: Synthetic peptide


<220>
<221> MOD_RES
<222> (9)..(9)
<223> Phosphorylated Tyr

<400> 1
Tyr Tyr Ala Asp Gly Glu Asp Ala Xaa Ala Met Lys Arg
1               5                   10


<210> 2
<211> 21
<212> PRT
<213> Artificial Sequence

<220>
<223> Description of Artificial Sequence: Synthetic peptide


<220>
<221> MOD_RES
<222> (10)..(10)
<223> Phosphorylated Tyr

<400> 2
Ala Leu Gln Leu Leu His Gly Gly Gly Xaa Ser Lys Asn Gly Ala Asn
1               5                   10                  15


Arg Trp Tyr Asp Lys

            20

<210> 3
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 3
Ser Glu Asp Arg Asp Leu Leu Xaa Ser Ile Asp Ala Tyr Arg Ser
1               5                   10                  15


<210> 4
<211> 12
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 4
Leu Ser Asp Ser Xaa Ser Asn Thr Leu Pro Val Arg
1               5                   10


<210> 5
<211> 28
<212> PRT
<213> Artificial Sequence

<220>
<223> Description of Artificial Sequence: Synthetic peptide


<220>
<221> MOD_RES
<222> (21)..(21)
<223> Phosphorylated Tyr

<400> 5
Met Ser Ala Gln Glu Ser Thr Pro Ile Met Asn Gly Val Thr Gly Pro
1               5                   10                  15


Asp Gly Glu Asp Xaa Ser Pro Trp Ala Asp Arg Lys

            20                  25

<210> 6
<211> 13
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 6
Asn Ser Xaa Ala Thr Thr Glu Asn Lys Thr Leu Pro Arg
1               5                   10

<210> 7
<211> 21
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 7
Gln Arg Pro Xaa Ser Val Ala Val Pro Ala Phe Ser Gln Gly Leu Asp
1               5                   10                  15

Asp Tyr Gly Ala Arg
            20

<210> 8
<211> 21
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 8
Gln Arg Pro Tyr Ser Val Ala Val Pro Ala Phe Ser Gln Gly Leu Asp

1               5                   10                  15

Asp Xaa Gly Ala Arg
            20

<210> 9
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 9
Tyr Ser Asp Lys Glu Leu Gln Xaa Ile Asp Ala Ile Ser Asn Lys
1               5                   10                  15

<210> 10
<211> 10
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 10
Ser Thr Leu Asn Glu Ile Xaa Phe Gly Lys
1               5                   10

<210> 11
<211> 13
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 11
Asn Ala Gly Asn Glu Gln Asp Leu Gly Ile Gln Xaa Lys

1                5                   10

<210> 12
<211> 13
<212> PRT
<213> Artificial Sequence

<220>
<223> Description of Artificial Sequence: Synthetic peptide


<220>
<221> MOD_RES
<222> (12)..(12)
<223> Phosphorylated Tyr

<400> 12
Asn Ala Gly Asn Glu Gln Asp Leu Gly Asn Gln Xaa Lys
1               5                   10


<210> 13
<211> 24
<212> PRT
<213> Artificial Sequence

<220>
<223> Description of Artificial Sequence: Synthetic peptide


<220>
<221> MOD_RES
<222> (13)..(13)
<223> Phosphorylated Tyr

<400> 13
Asp Phe Arg Lys Asn Gly Asn Gly Gly Pro Gly Pro Xaa Val Gly Gln
1               5                   10                  15


Ala Gly Thr Ala Thr Leu Pro Arg
            20


<210> 14
<211> 30
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 14
Glu Ile Pro Gln Ala Glu Arg Xaa Gln Glu Ala Ala Pro Asn Val Ala

1                   5                   10                  15

Asn Asn Thr Gly Pro His Ala Ala Ser Cys Phe Gly Ala Lys
            20                  25                  30

<210> 15
<211> 12
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 15
Ser Leu Asp Asn Asn Xaa Ser Thr Pro Asn Glu Arg
1               5                   10

<210> 16
<211> 17
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 16
Ser Gln Ser Ser His Ser Xaa Asp Asp Ser Thr Leu Pro Leu Ile Asp
1               5                   10                  15

Arg

<210> 17
<211> 16
<212> PRT
<213> Artificial Sequence

<220>
<223> Description of Artificial Sequence: Synthetic peptide

<220>
<221> MOD_RES
<222> (14)..(14)

<223> Phosphorylated Tyr

<400> 17
Leu Arg Glu Asp Glu Asn Ala Glu Pro Val Gly Thr Thr Xaa Gln Lys
1               5                   10                  15


<210> 18
<211> 23
<212> PRT
<213> Artificial Sequence

<220>
<223> Description of Artificial Sequence: Synthetic peptide


<220>
<221> MOD_RES
<222> (12)..(12)
<223> Phosphorylated Tyr

<400> 18
Lys Thr His Ile Gln Asp Asn His Asp Gly Thr Xaa Thr Val Ala Tyr
1               5                   10                  15


Val Pro Asp Val Thr Gly Arg
            20


<210> 19
<211> 24
<212> PRT
<213> Artificial Sequence

<220>
<223> Description of Artificial Sequence: Synthetic peptide


<220>
<221> MOD_RES
<222> (21)..(21)
<223> Phosphorylated Tyr

<400> 19
Val His Ser Pro Ser Gly Ala Leu Glu Glu Cys Tyr Val Thr Glu Ile
1               5                   10                  15


Asp Gln Asp Lys Xaa Ala Val Arg
            20


<210> 20
<211> 35
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 20
Ala Asp Leu Ser Asn Ser Leu Xaa Lys Arg Met Pro Ala Thr Ile Asp
1               5                   10                  15

Ser Val Phe Ala Gly Glu Val Thr Gln Leu Gln Ser Glu Val Ala Tyr
            20                  25                  30

Lys Gln Lys
        35

<210> 21
<211> 35
<212> PRT
<213> Artificial Sequence

<220>
<223> Description of Artificial Sequence: Synthetic peptide

<220>
<221> MOD_RES
<222> (32)..(32)
<223> Phosphorylated Tyr

<400> 21
Ala Asp Leu Ser Asn Ser Leu Tyr Lys Arg Met Pro Ala Thr Ile Asp
1               5                   10                  15

Ser Val Phe Ala Gly Glu Val Thr Gln Leu Gln Ser Glu Val Ala Xaa
            20                  25                  30

Lys Gln Lys
        35

<210> 22
<211> 23
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 22

Phe Ser Pro Asp Gly Asn Arg Phe Ala Thr Ala Ser Ala Asp Gly Gln
1               5                   10                  15

Ile Xaa Ile Tyr Asp Gly Lys
            20

<210> 23
<211> 23
<212> PRT
<213> Artificial Sequence

<220>
<223> Description of Artificial Sequence: Synthetic peptide

<220>
<221> MOD_RES
<222> (20)..(20)
<223> Phosphorylated Tyr

<400> 23
Phe Ser Pro Asp Gly Asn Arg Phe Ala Thr Ala Ser Ala Asp Gly Gln
1               5                   10                  15

Ile Tyr Ile Xaa Asp Gly Lys
            20

<210> 24
<211> 16
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 24
Tyr Ala Pro Ser Gly Phe Xaa Ile Ala Ser Gly Asp Val Ser Gly Lys
1               5                   10                  15

<210> 25
<211> 29
<212> PRT
<213> Artificial Sequence

<220>
<223> Description of Artificial Sequence: Synthetic peptide

<220>
<221> MOD_RES

<222> (21)..(21)
<223> Phosphorylated Tyr

<400> 25
Lys Lys Pro Ser Thr Asp Glu Gln Thr Ser Ser Ala Glu Glu Asp Val
1               5                   10                  15

Pro Thr Cys Gly Xaa Leu Asn Val Leu Ser Asn Ser Arg
            20                  25

<210> 26
<211> 35
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 26
Glu Gly Asp Gln Ile Val Gly Ala Thr Ile Xaa Phe Asp Asn Leu Gln
1               5                   10                  15

Ser Gly Glu Val Thr Gln Leu Leu Asn Thr Met Gly His His Thr Val
            20                  25                  30

Gly Leu Lys
        35

<210> 27
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 27
Glu Gly Ser Tyr Phe Ser Lys Xaa Ser Glu Ala Ala Glu Leu Arg
1               5                   10                  15

<210> 28
<211> 15
<212> PRT

<213> Artificial Sequence

<220>
<223> Description of Artificial Sequence: Synthetic peptide


<220>
<221> MOD_RES
<222> (8)..(8)
<223> Phosphorylated Tyr

<400> 28
Glu Thr Arg Pro Glu Gly Ser Xaa Phe Ser Lys Tyr Ser Glu Ala
1               5                   10                  15


<210> 29
<211> 18
<212> PRT
<213> Artificial Sequence

<220>
<223> Description of Artificial Sequence: Synthetic peptide


<220>
<221> MOD_RES
<222> (16)..(16)
<223> Phosphorylated Tyr

<400> 29
Asp Gly His Pro Leu Ser Pro Glu Arg Asp His Leu Glu Gly Leu Xaa
1               5                   10                  15


Ala Lys



<210> 30
<211> 25
<212> PRT
<213> Artificial Sequence

<220>
<223> Description of Artificial Sequence: Synthetic peptide


<220>
<221> MOD_RES
<222> (16)..(16)
<223> Phosphorylated Tyr

<400> 30
Gly Gln Gln Gln Gln Gln Gln Gln Gln Gly Ala Val Gly His Gly Xaa
1               5                   10                  15


Tyr Met Ala Gly Gly Thr Ser Gln Lys
            20                  25

<210> 31
<211> 16
<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 31
Glu Glu Asp Glu His Pro Xaa Glu Leu Leu Leu Thr Ala Glu Thr Lys
1               5                   10                  15


<210> 32
<211> 13
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 32
Glu Arg Arg Val Xaa Val Thr Leu Thr Cys Ala Phe Arg
1               5                   10


<210> 33
<211> 35
<212> PRT
<213> Artificial Sequence

<220>
<223> Description of Artificial Sequence: Synthetic peptide


<220>
<221> MOD_RES
<222> (17)..(17)
<223> Phosphorylated Tyr

<400> 33
Ile Leu Val Leu Thr Glu Leu Leu Glu Arg Lys Ala His Ser Pro Phe
1               5                   10                  15


Xaa Gln Glu Gly Val Ser Asn Ala Leu Leu Lys Met Ala Glu Leu Gly
            20                  25                  30

Leu Thr Arg
        35


<210> 34
<211> 27
<212> PRT
<213> Artificial Sequence

<220>
<223> Description of Artificial Sequence: Synthetic peptide

<400> 34
Tyr Ser Pro Arg Ser Arg Ser Pro Asp His His His His His His Ser
1               5                   10                  15


Gln Tyr His Ser Leu Leu Pro Pro Gly Gly Lys
            20                  25


<210> 35
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 35
Arg Gly Leu Leu Pro Ser Gln Xaa Gly Gln Glu Val Tyr Asp Thr
1               5                   10                  15


<210> 36
<211> 36
<212> PRT
<213> Artificial Sequence

<220>
<223> Description of Artificial Sequence: Synthetic peptide


<220>
<221> MOD_RES
<222> (29)..(29)
<223> Phosphorylated Tyr

<400> 36
Thr Pro Leu Val Leu Ala Ala Pro Pro Pro Asp Ser Pro Pro Ala Glu
1               5                   10                  15


Asp Val Tyr Asp Val Pro Pro Pro Ala Pro Asp Leu Xaa Asp Val Pro

20                  25                  30

Pro Gly Leu Arg
        35


<210> 37
<211> 36
<212> PRT
<213> Artificial Sequence

<220>
<223> Description of Artificial Sequence: Synthetic peptide


<220>
<221> MOD_RES
<222> (19)..(19)
<223> Phosphorylated Tyr

<400> 37
Thr Pro Leu Val Leu Ala Ala Pro Pro Pro Asp Ser Pro Pro Ala Glu
1               5                   10                  15


Asp Val Xaa Asp Val Pro Pro Pro Ala Pro Asp Leu Tyr Asp Val Pro
            20                  25                  30


Pro Gly Leu Arg
        35


<210> 38
<211> 21
<212> PRT
<213> Artificial Sequence

<220>
<223> Description of Artificial Sequence: Synthetic peptide


<220>
<221> MOD_RES
<222> (11)..(11)
<223> Phosphorylated Tyr

<400> 38
Gly Thr Phe Pro Leu Asp Glu Asp Val Ser Xaa Lys Val Pro Ser Ser
1               5                   10                  15


Phe Leu Ile Pro Arg
            20


<210> 39
<211> 13
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 39
Ser Glu Trp Ile Xaa Asp Thr Pro Val Ser Pro Gly Lys
1               5                   10

<210> 40
<211> 24
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 40
Ser Trp Ala Glu Gly Pro Gln Pro Pro Thr Ala Gln Val Xaa Glu Phe
1               5                   10                  15

Pro Asp Pro Pro Thr Ser Ala Arg
            20

<210> 41
<211> 15
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 41
Val Glu Gln Gln Asn Thr Lys Pro Asn Ile Xaa Asp Ile Pro Lys
1               5                   10                  15

<210> 42
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 42
Glu Leu Ser Glu Lys Gln Val Xaa Asp Ala His Thr Lys Glu Ile
1               5                   10                  15

<210> 43
<211> 16
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 43
Gln Gly Ser Gly Val Ile Leu Arg Gln Glu Glu Ala Glu Xaa Val Arg
1               5                   10                  15

<210> 44
<211> 10
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 44
His Ile Asp Arg Asn Xaa Glu Pro Leu Lys
1               5                   10

<210> 45
<211> 26
<212> PRT
<213> Artificial Sequence

<220>
<223> Description of Artificial Sequence: Synthetic peptide

<220>
<221> MOD_RES
<222> (17)..(17)
<223> Phosphorylated Tyr

<400> 45
Leu Ser Thr Glu His Ser Ser Val Ser Glu Tyr His Pro Ala Asp Gly
1               5                   10                  15

Xaa Ala Phe Ser Ser Asn Ile Tyr Thr Arg
            20                  25

<210> 46
<211> 26
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 46
Leu Ser Thr Glu His Ser Ser Val Ser Glu Xaa His Pro Ala Asp Gly
1               5                   10                  15

Tyr Ala Phe Ser Ser Asn Ile Tyr Thr Arg
            20                  25

<210> 47
<211> 9
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 47
Val Xaa Ser Gln Ile Thr Val Gln Lys
1               5

<210> 48
<211> 13
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 48
Ala Gln Ala Asp Leu Ala Xaa Gln Leu Gln Val Ala Lys
1               5                   10

<210> 49
<211> 13
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 49
Val Ser Ala Gln Xaa Leu Ser Glu Ile Glu Met Ala Lys
1               5                   10

<210> 50
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 50
Gln Glu Asn Ala Asn Ser Gly Xaa Tyr Glu Ala His Pro Val Thr
1               5                   10                  15

<210> 51
<211> 12
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 51
Ala Ser Ser Cys Glu Thr Xaa Glu Tyr Pro Gln Arg
1               5                   10


<210> 52
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 52
Ser Glu Glu Gln Arg Val Asp Xaa Val Gln Val Asp Glu Gln Lys
1               5                   10                  15


<210> 53
<211> 24
<212> PRT
<213> Artificial Sequence

<220>
<223> Description of Artificial Sequence: Synthetic peptide


<220>
<221> MOD_RES
<222> (20)..(20)
<223> Phosphorylated Tyr

<400> 53
Arg Gly Ser Asn Pro Val Lys Arg Tyr Ala Pro Gly Leu Pro Cys Asp
1               5                   10                  15


Val Tyr Thr Xaa Leu His Glu Lys
            20


<210> 54
<211> 29
<212> PRT
<213> Artificial Sequence

<220>
<223> Description of Artificial Sequence: Synthetic peptide

<220>
<221> MOD_RES
<222> (26)..(26)
<223> Phosphorylated Tyr

<400> 54
Met Asn Lys Glu Ile Pro Asn Gly Asn Thr Ser Glu Leu Ile Phe Asn
1               5                   10                  15

Ala Val His Val Lys Asp Ala Gly Phe Xaa Val Cys Arg
            20                  25

<210> 55
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 55
Lys Phe Thr Ser Asp Asn Lys Xaa Lys Glu Asn Tyr Gln Asn His
1               5                   10                  15

<210> 56
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 56
Gln Asn His Met Arg Gly Arg Xaa Glu Gly Val Gly Met Asp Arg
1               5                   10                  15

<210> 57
<211> 13
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 57
Glu Gly His Met Met Asp Ala Leu Xaa Ala Gln Val Lys
1               5                   10


<210> 58
<211> 25
<212> PRT
<213> Artificial Sequence

<220>
<223> Description of Artificial Sequence: Synthetic peptide


<220>
<221> MOD_RES
<222> (15)..(15)
<223> Phosphorylated Tyr

<400> 58
Lys Asn Ala Ser Ser Val Ser Gln Asp Ser Trp Glu Gln Asn Xaa Ser
1               5                   10                  15


Pro Gly Glu Gly Phe Gln Ser Ala Lys
            20                  25


<210> 59
<211> 21
<212> PRT
<213> Artificial Sequence

<220>
<223> Description of Artificial Sequence: Synthetic peptide


<220>
<221> MOD_RES
<222> (16)..(16)
<223> Phosphorylated Tyr

<400> 59
Lys Ser Gly Asn Ser Val Thr Leu Leu Val Leu Asp Gly Asp Ser Xaa
1               5                   10                  15


Glu Lys Ala Val Lys
            20


<210> 60
<211> 17
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 60
Ser Ser Glu His Glu Asn Ala Xaa Glu Asn Val Pro Glu Glu Glu Gly
1               5                   10                  15

Lys

<210> 61
<211> 31
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 61
Ser Val His Glu Ser Gly Gln Asn Asn Arg Xaa Ser Pro Lys Lys Glu
1               5                   10                  15

Lys Ala Gly Gly Ser Glu Pro Gln Asp Glu Trp Gly Gly Ser Lys
            20                  25                  30

<210> 62
<211> 10
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 62
Ile Xaa Gln Phe Thr Ala Ala Ser Pro Lys
1               5                   10

<210> 63
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 63
Leu Ser Lys Thr Val Phe Tyr Xaa Tyr Gly Ser Asp Lys Asp Lys
1               5                   10                  15


<210> 64
<211> 22
<212> PRT
<213> Artificial Sequence

<220>
<223> Description of Artificial Sequence: Synthetic peptide


<220>
<221> MOD_RES
<222> (16)..(16)
<223> Phosphorylated Tyr

<400> 64
Thr Pro Ser Phe Glu Leu Pro Asp Ala Ser Glu Arg Pro Pro Thr Xaa
1               5                   10                  15


Cys Glu Leu Val Pro Arg
            20


<210> 65
<211> 15
<212> PRT
<213> Artificial Sequence

<220>
<223> Description of Artificial Sequence: Synthetic peptide

<400> 65
Leu Lys Tyr Glu Glu Pro Glu Tyr Asp Ile Pro Ala Phe Gly Phe
1               5                   10                  15


<210> 66
<211> 23
<212> PRT
<213> Artificial Sequence

<220>

<223> Description of Artificial Sequence: Synthetic peptide

<220>
<221> MOD_RES
<222> (8)..(8)
<223> Phosphorylated Tyr

<400> 66
Ile Gly Ile Phe Pro Ile Ser Xaa Val Glu Phe Asn Ser Ala Ala Lys
1               5                   10                  15

Gln Leu Ile Glu Trp Asp Lys
            20

<210> 67
<211> 27
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 67
Gly Ile Gln Leu Xaa Asp Thr Pro Tyr Glu Pro Glu Gly Gln Ser Val
1               5                   10                  15

Asp Ser Asp Ser Glu Ser Thr Val Ser Pro Arg
            20                  25

<210> 68
<211> 15
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 68
Leu Asp Xaa Cys Gly Gly Ser Gly Glu Pro Gly Gly Val Gln Arg
1               5                   10                  15

<210> 69
<211> 30

<212> PRT
<213> Artificial Sequence

<220>
<223> Description of Artificial Sequence: Synthetic peptide

<220>
<221> MOD_RES
<222> (27)..(27)
<223> Phosphorylated Tyr

<400> 69
Gln Ile Ile Ala Asn His His Met Arg Ser Ile Ser Phe Ala Ser Gly
1               5                   10                  15

Gly Asp Pro Asp Thr Thr Asp Tyr Val Ala Xaa Val Thr Lys
            20                  25                  30

<210> 70
<211> 30
<212> PRT
<213> Artificial Sequence

<220>
<223> Description of Artificial Sequence: Synthetic peptide

<220>
<221> MOD_RES
<222> (24)..(24)
<223> Phosphorylated Tyr

<400> 70
Gln Ile Ile Ala Asn His His Met Arg Ser Ile Ser Phe Ala Ser Gly
1               5                   10                  15

Gly Asp Pro Asp Thr Thr Asp Xaa Val Ala Tyr Val Thr Lys
            20                  25                  30

<210> 71
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 71
Ser Pro Arg Thr Ser Thr Ile Xaa Asp Met Tyr Arg Thr Arg Glu
1               5                   10                  15

<210> 72
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 72
Thr Ser Thr Ile Tyr Asp Met Xaa Arg Thr Arg Glu Pro Arg Val
1               5                   10                  15


<210> 73
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 73
Tyr Asp Pro Gln Glu Glu Val Xaa Leu Ser Leu Lys Glu Ala Gln
1               5                   10                  15


<210> 74
<211> 13
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 74
Gly Ser Asn Glu Xaa Thr Glu Gly Pro Ser Val Val Lys
1               5                   10


<210> 75
<211> 15

<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 75
Asp Ile Val Arg Ser Asn His Xaa Asp Pro Glu Glu Asp Glu Glu
1               5                   10                  15


<210> 76
<211> 20
<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 76
Asp Leu Glu Gln Pro Thr Xaa Arg Tyr Glu Ser Ser Ser Tyr Thr Asp
1               5                   10                  15


Gln Phe Ser Arg
            20


<210> 77
<211> 8
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 77
Ala Tyr Asp Pro Asp Xaa Glu Arg
1               5


<210> 78
<211> 16

<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 78
Ala Tyr Asp Pro Asp Tyr Glu Arg Ala Xaa Ser Pro Glu Tyr Arg Arg
1               5                   10                  15

<210> 79
<211> 21
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 79
Ser Tyr Ser Pro Xaa Asp Tyr Gln Pro Cys Leu Ala Gly Pro Asn Gln
1               5                   10                  15

Asp Phe His Ser Lys
            20

<210> 80
<211> 15
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 80
Phe Lys Val Glu Ser Glu Gln Gln Xaa Phe Glu Ile Glu Lys Arg
1               5                   10                  15

<210> 81
<211> 15

<212> PRT
<213> Artificial Sequence

<220>
<223> Description of Artificial Sequence: Synthetic peptide

<400> 81
Tyr Cys Thr Lys Glu Phe Val Phe Asp Thr Ile Gln Ser His Gln
1               5                   10                  15


<210> 82
<211> 21
<212> PRT
<213> Artificial Sequence

<220>
<223> Description of Artificial Sequence: Synthetic peptide


<220>
<221> MOD_RES
<222> (12)..(12)
<223> Phosphorylated Tyr

<400> 82
Val Asn Phe Cys Pro Leu Pro Ser Glu Gln Cys Xaa Gln Ala Pro Gly
1               5                   10                  15


Gly Pro Glu Asp Arg
            20


<210> 83
<211> 13
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 83
Phe Xaa Gly Pro Gln Val Asn Asn Ile Ser His Thr Lys
1               5                   10


<210> 84
<211> 27
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 84
Xaa Pro Pro Asn Lys Val Met Ile Trp Asp Asp Leu Lys Lys Lys Thr
1               5                   10                  15

Val Ile Glu Ile Glu Phe Ser Thr Glu Val Lys
            20                  25


<210> 85
<211> 17
<212> PRT
<213> Artificial Sequence

<220>
<223> Description of Artificial Sequence: Synthetic peptide


<220>
<221> MOD_RES
<222> (12)..(12)
<223> Phosphorylated Tyr

<400> 85
Val Phe Ser Asn Gly His Leu Gly Ser Glu Glu Xaa Asp Val Pro Pro
1               5                   10                  15

Arg



<210> 86
<211> 23
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 86
Val Ala Ser Asn Pro Xaa Thr Trp Phe Thr Met Glu Ala Leu Glu Glu
1               5                   10                  15

Thr Trp Arg Asn Leu Gln Lys
            20

<210> 87
<211> 36
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 87
Glu Leu Gly Glu Pro Thr Xaa Leu Val Ile Asp Pro Arg Ala Glu Glu
1               5                   10                  15

Glu Asp Glu Lys Gly Thr Ala Gly Gly Leu Leu Ser Ser Leu Glu Gly
            20                  25                  30

Leu Lys Met Arg
        35

<210> 88
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 88
Met Leu His Glu Phe Ser Lys Xaa Arg Gln Arg Ile Lys Gln His
1               5                   10                  15

<210> 89
<211> 17
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 89

His Val Ser Pro Val Thr Pro Pro Arg Glu Val Pro Ile Xaa Ala Asn
1               5                   10                  15

Arg

<210> 90
<211> 18
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 90
Lys Arg Ser Gln Thr Xaa Glu Ser Asp Gly Lys Asn Gln Ala Asn Pro
1               5                   10                  15

Ser Arg

<210> 91
<211> 14
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 91
Val Lys Met Pro Glu Gln Glu Xaa Glu Phe Pro Glu Pro Arg
1               5                   10

<210> 92
<211> 24
<212> PRT
<213> Artificial Sequence

<220>
<223> Description of Artificial Sequence: Synthetic peptide

<220>
<221> MOD_RES

<222> (15)..(15)
<223> Phosphorylated Tyr

<400> 92
Thr Tyr Arg Tyr Phe Leu Leu Leu Phe Trp Val Gly Gln Pro Xaa Pro
1               5                   10                  15

Thr Leu Ser Thr Pro Leu Ser Lys
            20

<210> 93
<211> 11
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 93
Val Thr Ile Asp Ser Ser Xaa Asp Ile Ala Lys
1               5                   10

<210> 94
<211> 15
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 94
Thr Glu Asp Glu Val Gln Ser Tyr Pro Ser Lys His Asp Xaa Val
1               5                   10                  15

<210> 95
<211> 15
<212> PRT
<213> Artificial Sequence

<220>
<223> Description of Artificial Sequence: Synthetic peptide

<220>
<221> MOD_RES

<222> (3)..(3)
<223> Phosphorylated Tyr

<400> 95
Ser Asn Xaa Tyr Asp Ala Tyr Gln Ala Gln Pro Leu Ala Thr Arg
1               5                   10                  15


<210> 96
<211> 12
<212> PRT
<213> Artificial Sequence

<220>
<223> Description of Artificial Sequence: Synthetic peptide


<220>
<221> MOD_RES
<222> (11)..(11)
<223> Phosphorylated Tyr

<400> 96
Ser Tyr Pro Lys Ser Asn Ser Ser Lys Glu Xaa Val
1               5                   10


<210> 97
<211> 13
<212> PRT
<213> Artificial Sequence

<220>
<223> Description of Artificial Sequence: Synthetic peptide


<220>
<221> MOD_RES
<222> (11)..(11)
<223> Phosphorylated Tyr

<400> 97
Cys Asn Glu Gln Pro Asn Arg Val Glu Ile Xaa Glu Lys
1               5                   10


<210> 98
<211> 32
<212> PRT
<213> Artificial Sequence

<220>
<223> Description of Artificial Sequence: Synthetic peptide


<220>
<221> MOD_RES
<222> (28)..(28)
<223> Phosphorylated Tyr

<400> 98

Phe Phe Lys Gln Leu Gln Val Val Pro Leu Phe Gly Asp Met Gln Ile
1               5                   10                  15

Glu Leu Ala Arg Tyr Ile Lys Thr Ser Ala His Xaa Glu Glu Asn Lys
            20                  25                  30

<210> 99
<211> 10
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 99
Glu Gln Leu Ile Asp Xaa Leu Met Leu Lys
1               5                   10

<210> 100
<211> 31
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 100
Met Pro Leu Ser Asn Gly Gln Met Gly Gln Pro Leu Arg Pro Gln Ala
1               5                   10                  15

Asn Xaa Ser Gln Ile His His Pro Pro Gln Ala Ser Val Ala Arg
            20                  25                  30

<210> 101
<211> 15
<212> PRT
<213> Artificial Sequence

<220>
<223> Description of Artificial Sequence: Synthetic peptide

<220>
<221> MOD_RES

<222> (7)..(7)
<223> Phosphorylated Tyr

<400> 101
Val Ala His Gln Pro Pro Xaa Thr Gln Pro His Cys Ser Pro Arg
1               5                   10                  15


<210> 102
<211> 14
<212> PRT
<213> Artificial Sequence

<220>
<223> Description of Artificial Sequence: Synthetic peptide


<220>
<221> MOD_RES
<222> (1)..(1)
<223> Phosphorylated Tyr

<400> 102
Xaa Pro Thr Pro Tyr Pro Asp Glu Leu Lys Asn Met Val Lys
1               5                   10


<210> 103
<211> 14
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 103
Tyr Pro Thr Pro Xaa Pro Asp Glu Leu Lys Asn Met Val Lys
1               5                   10


<210> 104
<211> 13
<212> PRT
<213> Artificial Sequence

<220>
<223> Description of Artificial Sequence: Synthetic peptide


<220>
<221> MOD_RES
<222> (13)..(13)
<223> Phosphorylated Tyr

<400> 104

Ser Gln Pro Ser Glu Thr Glu Arg Leu Thr Asp Asp Xaa
1               5                   10

<210> 105
<211> 30
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 105
Asp Asp Ile Leu Phe Tyr Thr Leu Gln Glu Met Thr Ala Gly Ala Ser
1               5                   10                  15

Asp Xaa Phe Ser Leu Val Ser Val Asn Arg Pro Ala Leu Arg
            20                  25                  30

<210> 106
<211> 15
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 106
Gly Gly Gly Pro Tyr Asp Ala Pro Gly Gly Asp Asp Ser Xaa Ile
1               5                   10                  15

<210> 107
<211> 15
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 107

Gly Gly Gly Pro Xaa Asp Ala Pro Gly Gly Asp Asp Ser Tyr Ile
1               5                   10                  15

<210> 108
<211> 12
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 108
Phe Ser Ser Xaa Ser Gln Met Glu Asn Trp Ser Arg
1               5                   10

<210> 109
<211> 18
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 109
Gly Ser Met Xaa Asp Gly Leu Ala Asp Asn Tyr Asn Tyr Gly Thr Thr
1               5                   10                  15

Ser Arg

<210> 110
<211> 18
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 110

Gly Ser Met Tyr Asp Gly Leu Ala Asp Asn Xaa Asn Tyr Gly Thr Thr
1               5                   10                  15

Ser Arg

<210> 111
<211> 13
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 111
Gln Asp Pro Val Xaa Ile Pro Pro Ile Ser Cys Asn Lys
1               5                   10

<210> 112
<211> 10
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 112
Ser Glu Pro Asp Leu Xaa Cys Asp Pro Arg
1               5                   10

<210> 113
<211> 11
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 113

Tyr Ser Phe Xaa Ser Thr Cys Ser Gly Gln Lys
1               5                   10

<210> 114
<211> 13
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 114
Ala Gly Thr Thr Ala Thr Xaa Glu Gly Arg Trp Gly Arg
1               5                   10

<210> 115
<211> 22
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 115
Ala Gly Thr Thr Ala Thr Tyr Glu Gly Arg Trp Gly Arg Gly Thr Ala
1               5                   10                  15

Gln Xaa Ser Ser Gln Lys
            20

<210> 116
<211> 13
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 116

Ala His Xaa Thr His Ser Asp Tyr Gln Tyr Ser Gln Arg
1               5                   10

<210> 117
<211> 34
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 117
Ser Met Gly Asn Leu Leu Glu Lys Glu Asn Xaa Leu Thr Ala Gly Leu
1               5                   10                  15

Thr Val Gly Gln Val Arg Pro Leu Val Pro Leu Gln Pro Val Thr Gln
            20                  25                  30

Asn Arg

<210> 118
<211> 11
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 118
Ser Pro Val Pro Lys Thr Xaa Asp Met Leu Lys
1               5                   10

<210> 119
<211> 22
<212> PRT
<213> Artificial Sequence

<220>
<223> Description of Artificial Sequence: Synthetic peptide

<220>
<221> MOD_RES

<222> (7)..(7)
<223> Phosphorylated Tyr

<400> 119
Thr Ser Ser Val Pro Glu Xaa Val Tyr Asn Leu His Leu Val Glu Asn
1               5                   10                  15

Asp Phe Val Gly Gly Arg
            20

<210> 120
<211> 14
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 120
Val Lys Glu Gln Xaa Gln Asp Val Pro Met Pro Glu Glu Lys
1               5                   10

<210> 121
<211> 18
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 121
Tyr Ser Gln Asn Ile Xaa Ile Gln Asn Arg Asn Ile Gln Thr Asp Asn
1               5                   10                  15

Asn Lys

<210> 122
<211> 18
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 122
Xaa Ser Gln Asn Ile Tyr Ile Gln Asn Arg Asn Ile Gln Thr Asp Asn
1               5                   10                  15

Asn Lys

<210> 123
<211> 27
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 123
Thr Ser Ser Val Pro Glu Tyr Val Xaa Asn Leu His Leu Val Glu Asn
1               5                   10                  15

Asp Phe Val Gly Gly Arg Ser Pro Val Pro Lys
            20                  25

<210> 124
<211> 17
<212> PRT
<213> Artificial Sequence

<220>
<223> Description of Artificial Sequence: Synthetic peptide

<220>
<221> MOD_RES
<222> (13)..(13)
<223> Phosphorylated Tyr

<400> 124
Leu Tyr Leu Gln Ser Pro His Ser Tyr Glu Asp Pro Xaa Phe Asp Asp
1               5                   10                  15

Arg

<210> 125
<211> 18
<212> PRT
<213> Artificial Sequence

<220>
<223> Description of Artificial Sequence: Synthetic peptide


<220>
<221> MOD_RES
<222> (17)..(17)
<223> Phosphorylated Tyr

<400> 125
Ser Pro Asn His Gly Thr Val Glu Leu Gln Gly Ser Gln Thr Ala Leu
1               5                   10                  15


Xaa Arg



<210> 126
<211> 32
<212> PRT
<213> Artificial Sequence

<220>
<223> Description of Artificial Sequence: Synthetic peptide


<220>
<221> MOD_RES
<222> (23)..(23)
<223> Phosphorylated Tyr

<400> 126
Thr Ser Leu Gly Ser Gly Phe Gly Ser Pro Ser Val Thr Asp Pro Arg
1               5                   10                  15


Pro Leu Asn Pro Ser Ala Xaa Ser Ser Thr Thr Leu Pro Ala Ala Arg
            20                  25                  30


<210> 127
<211> 34
<212> PRT
<213> Artificial Sequence

<220>
<223> Description of Artificial Sequence: Synthetic peptide


<220>
<221> MOD_RES
<222> (32)..(32)
<223> Phosphorylated Tyr

<400> 127
Lys Leu Asp Asp Gln Ser Glu Asp Glu Ala Leu Glu Leu Glu Gly Pro
1               5                   10                  15

Leu Ile Thr Pro Gly Ser Gly Ser Ile Tyr Ser Ser Pro Gly Leu Xaa
            20                  25                  30

Ser Lys

<210> 128
<211> 24
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 128
Gln Ala Glu Glu Glu Arg Gln Glu Glu Pro Glu Xaa Glu Asn Val Val
1               5                   10                  15

Pro Ile Ser Arg Pro Pro Glu Pro
            20

<210> 129
<211> 21
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 129
Asp Xaa Ser Leu Thr Met Gln Ser Ser Val Thr Val Gln Glu Gly Met
1               5                   10                  15

Cys Val His Val Arg
            20

<210> 130
<211> 13

<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 130
His Val Ala Tyr Gly Gly Xaa Ser Thr Pro Glu Asp Arg
1               5                   10


<210> 131
<211> 19
<212> PRT
<213> Artificial Sequence

<220>
<223> Description of Artificial Sequence: Synthetic peptide


<220>
<221> MOD_RES
<222> (12)..(12)
<223> Phosphorylated Tyr

<400> 131
Ile Leu Leu Arg Asn Pro Gly Asn Gln Ala Ala Xaa Glu His Phe Glu
1               5                   10                  15


Thr Met Lys



<210> 132
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 132
Arg Pro Leu Asn Pro Ser Ala Xaa Ser Ser Thr Thr Leu Pro Ala
1               5                   10                  15


<210> 133
<211> 33

<212> PRT
<213> Artificial Sequence

<220>
<223> Description of Artificial Sequence: Synthetic peptide


<220>
<221> MOD_RES
<222> (32)..(32)
<223> Phosphorylated Tyr

<400> 133
Thr Glu Pro Met Ala Trp Asn Glu Thr Ala Asp Leu Gly Leu Asp Ile
1               5                   10                  15


Gly Ala Gln Gly Glu Pro Leu Gly Tyr Arg Gln Asp Asp Pro Ser Xaa
            20                  25                  30


Arg



<210> 134
<211> 20
<212> PRT
<213> Artificial Sequence

<220>
<223> Description of Artificial Sequence: Synthetic peptide


<220>
<221> MOD_RES
<222> (9)..(9)
<223> Phosphorylated Tyr

<400> 134
Ala Glu Ser Gly Pro Asp Leu Arg Xaa Glu Val Thr Ser Gly Gly Gly
1               5                   10                  15


Gly Thr Ser Arg
            20


<210> 135
<211> 18
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 135
Gly Gly Gly Gly Xaa Thr Cys Gln Ser Gly Ser Gly Trp Asp Glu Phe
1               5                   10                  15

Thr Lys

<210> 136
<211> 14
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 136
Ile Thr Arg Leu Thr Xaa Glu Ile Glu Asp Glu Lys Arg Arg
1               5                   10

<210> 137
<211> 14
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 137
Thr Asp Lys Lys Tyr Leu Met Ile Glu Glu Xaa Leu Thr Lys
1               5                   10

<210> 138
<211> 9
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 138
His Lys Lys Leu Xaa Pro Ser Cys Arg
1               5

<210> 139
<211> 23
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 139
His Met Asn Gly Xaa Gly Ser His Thr Phe Lys Leu Val Asn Ala Asn
1               5                   10                  15

Gly Glu Ala Val Tyr Cys Lys
            20

<210> 140
<211> 28
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 140
Arg Xaa Val His Thr Phe Phe Gly Cys Lys Glu Cys Gly Glu His Phe
1               5                   10                  15

Glu Glu Met Ala Lys Glu Ser Met Asp Ser Val Lys
            20                  25

<210> 141
<211> 7
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 141
Asn Xaa Lys Asn Val Phe Lys
1               5


<210> 142
<211> 9
<212> PRT
<213> Artificial Sequence

<220>
<223> Description of Artificial Sequence: Synthetic peptide


<220>
<221> MOD_RES
<222> (3)..(3)
<223> Phosphorylated Tyr

<400> 142
Asp Gly Xaa Asn Tyr Thr Leu Ser Lys
1               5


<210> 143
<211> 29
<212> PRT
<213> Artificial Sequence

<220>
<223> Description of Artificial Sequence: Synthetic peptide


<220>
<221> MOD_RES
<222> (17)..(17)
<223> Phosphorylated Tyr

<400> 143
Ser Leu Leu Arg Asp Asn Val Asp Leu Leu Gly Ser Leu Ala Asp Leu
1               5                   10                  15


Xaa Phe Arg Ala Gly Asp Asn Lys Asn Ser Val Leu Lys
            20                  25


<210> 144
<211> 23
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 144
Thr Xaa Ile Thr Phe Gln Thr Trp Lys His Ala Ser Ile Leu Ile Gln
1               5                   10                  15

Gln His Tyr Arg Thr Tyr Arg
            20

<210> 145
<211> 23
<212> PRT
<213> Artificial Sequence

<220>
<223> Description of Artificial Sequence: Synthetic peptide

<220>
<221> MOD_RES
<222> (19)..(19)
<223> Phosphorylated Tyr

<400> 145
Thr Tyr Ile Thr Phe Gln Thr Trp Lys His Ala Ser Ile Leu Ile Gln
1               5                   10                  15

Gln His Xaa Arg Thr Tyr Arg
            20

<210> 146
<211> 23
<212> PRT
<213> Artificial Sequence

<220>
<223> Description of Artificial Sequence: Synthetic peptide

<220>
<221> MOD_RES
<222> (22)..(22)
<223> Phosphorylated Tyr

<400> 146
Thr Tyr Ile Thr Phe Gln Thr Trp Lys His Ala Ser Ile Leu Ile Gln
1               5                   10                  15

Gln His Tyr Arg Thr Xaa Arg
            20

<210> 147

<211> 11
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 147
Gly Ala Leu Thr Gly Gly Tyr Xaa Asp Thr Arg
1               5                   10


<210> 148
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 148
Glu Asn Gly Gly Gly Gln Gly Xaa Ser Ser Gly Pro Gly Thr Ser
1               5                   10                  15


<210> 149
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 149
Glu Arg Leu Gly Glu Asp Pro Xaa Tyr Thr Glu Asn Gly Gly Gly
1               5                   10                  15


<210> 150
<211> 15
<212> PRT
<213> Artificial Sequence

<220>
<223> Description of Artificial Sequence: Synthetic peptide

<400> 150
Gly Glu Arg Leu Gly Glu Asp Pro Tyr Tyr Thr Glu Asn Gly Gly
1               5                   10                  15


<210> 151
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 151
Gln Asn Gln Tyr Gln Ala Ser Xaa Asn Gln Ser Phe Ser Ser Gln
1               5                   10                  15


<210> 152
<211> 19
<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 152
Asn Leu Glu Glu Asp Asp Xaa Leu His Lys Asp Thr Gly Glu Thr Ser
1               5                   10                  15


Met Leu Lys



<210> 153
<211> 12
<212> PRT
<213> Artificial Sequence

<220>
<223> Description of Artificial Sequence: Synthetic peptide


<220>
<221> MOD_RES

<222> (4)..(4)
<223> Phosphorylated Tyr

<400> 153
Gln Cys Phe Xaa Glu Asp Ile Ala Gln Gly Thr Lys
1               5                   10


<210> 154
<211> 17
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 154
Leu Thr Asp Gly Ser Xaa Phe Gly Glu Ile Cys Leu Leu Thr Arg Gly
1               5                   10                  15

Arg



<210> 155
<211> 29
<212> PRT
<213> Artificial Sequence

<220>
<223> Description of Artificial Sequence: Synthetic peptide


<220>
<221> MOD_RES
<222> (27)..(27)
<223> Phosphorylated Tyr

<400> 155
Ala Pro Ile Leu Gln Ser Thr Pro Val Thr Pro Pro Pro Leu Pro Pro
1               5                   10                  15

Ala Phe Gly Gly Thr Ser Lys Ile Asp Gln Xaa Ser Arg
            20                  25


<210> 156
<211> 34
<212> PRT
<213> Artificial Sequence

<220>
<223> Description of Artificial Sequence: Synthetic peptide

<220>
<221> MOD_RES
<222> (31)..(31)
<223> Phosphorylated Tyr

<400> 156
Lys Ala Gln Phe Ala Ala Pro Pro Thr Val Thr Ile Ser Lys Ala Thr
1               5                   10                  15

Glu Pro Leu Glu Ala Glu Ile Val Leu His Pro Asp Ser Lys Xaa His
            20                  25                  30

Leu Lys

<210> 157
<211> 31
<212> PRT
<213> Artificial Sequence

<220>
<223> Description of Artificial Sequence: Synthetic peptide

<220>
<221> MOD_RES
<222> (22)..(22)
<223> Phosphorylated Tyr

<400> 157
Asn Glu Met Ala Thr Ser Glu Ala Val Met Gly Leu Gly Asp Pro Arg
1               5                   10                  15

Ser Thr Met Leu Ala Xaa Asp Ala Ser Ser Ile Gln Tyr Arg Lys
            20                  25                  30

<210> 158
<211> 31
<212> PRT
<213> Artificial Sequence

<220>
<223> Description of Artificial Sequence: Synthetic peptide

<220>
<221> MOD_RES
<222> (29)..(29)
<223> Phosphorylated Tyr

<400> 158
Asn Glu Met Ala Thr Ser Glu Ala Val Met Gly Leu Gly Asp Pro Arg
1               5                   10                  15

Ser Thr Met Leu Ala Tyr Asp Ala Ser Ser Ile Gln Xaa Arg Lys
            20                  25                  30

<210> 159
<211> 35
<212> PRT
<213> Artificial Sequence

<220>
<223> Description of Artificial Sequence: Synthetic peptide

<220>
<221> MOD_RES
<222> (27)..(27)
<223> Phosphorylated Tyr

<400> 159
Met Val Gln Val Gln Gly Met Thr Gly Asn Ile Gln Phe Asp Thr Tyr
1               5                   10                  15

Gly Arg Arg Thr Asn Tyr Thr Ile Asp Val Xaa Glu Met Lys Val Ser
            20                  25                  30

Gly Ser Arg
        35

<210> 160
<211> 17
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 160
Met Val Ala Glu Val Phe Ile Xaa Trp Ser Lys Ser His Asn Phe Lys
1               5                   10                  15

Arg

<210> 161
<211> 35
<212> PRT
<213> Artificial Sequence

<220>

<223> Description of Artificial Sequence: Synthetic peptide

<220>
<221> MOD_RES
<222> (34)..(34)
<223> Phosphorylated Tyr

<400> 161
Ile Asp Leu Lys Thr Lys Ser Cys Ser Gly Val Glu Phe Ser Thr Ser
1               5                   10                  15

Gly His Ala Tyr Thr Asp Thr Gly Lys Ala Ser Gly Asn Leu Glu Thr
            20                  25                  30

Lys Xaa Lys
        35

<210> 162
<211> 18
<212> PRT
<213> Artificial Sequence

<220>
<223> Description of Artificial Sequence: Synthetic peptide

<220>
<221> MOD_RES
<222> (16)..(16)
<223> Phosphorylated Tyr

<400> 162
Thr Val Met Tyr Thr Ala Val Gly Ser Glu Trp Arg Pro Phe Gly Xaa
1               5                   10                  15

Pro Arg

<210> 163
<211> 11
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 163
Thr Leu Ser Gly Met Glu Ser Xaa Cys Val Arg
1               5                   10

<210> 164
<211> 17
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 164
Thr Phe Val Glu Lys Xaa Glu Lys Gln Ile Lys His Phe Gly Met Leu
1               5                   10                  15


Arg



<210> 165
<211> 19
<212> PRT
<213> Artificial Sequence

<220>
<223> Description of Artificial Sequence: Synthetic peptide


<220>
<221> MOD_RES
<222> (14)..(14)
<223> Phosphorylated Tyr

<400> 165
Asn Val Val His Gln Leu Ser Val Thr Leu Glu Asp Leu Xaa Asn Gly
1               5                   10                  15


Ala Thr Arg



<210> 166
<211> 13
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 166
Ile Lys Glu Lys Xaa Ile Asp Gln Glu Glu Leu Asn Lys
1               5                   10

<210> 167
<211> 28
<212> PRT
<213> Artificial Sequence

<220>
<223> Description of Artificial Sequence: Synthetic peptide

<220>
<221> MOD_RES
<222> (19)..(19)
<223> Phosphorylated Tyr

<400> 167
Leu Val Gly Met Pro Ala Lys Arg Gln Ala Val Thr Asn Pro Asn Asn
1               5                   10                  15

Thr Phe Xaa Ala Thr Lys Arg Leu Ile Gly Arg Arg
            20                  25

<210> 168
<211> 24
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 168
Ser Val Pro His Ala His Pro Ala Thr Ala Glu Xaa Glu Phe Ala Asn
1               5                   10                  15

Pro Ser Arg Leu Gly Glu Gln Arg
            20

<210> 169
<211> 13
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 169
Cys Glu Phe Gln Asp Ala Xaa Val Leu Leu Ser Glu Lys
1               5                   10


<210> 170
<211> 20
<212> PRT
<213> Artificial Sequence

<220>
<223> Description of Artificial Sequence: Synthetic peptide


<220>
<221> MOD_RES
<222> (20)..(20)
<223> Phosphorylated Tyr

<400> 170
Arg Asn Ser Asn Arg Ala His Gln Gly Lys His Glu Thr Tyr Gly His
1               5                   10                  15


Lys Thr Pro Xaa
            20


<210> 171
<211> 13
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 171
Asp Ile Met Asp Leu Xaa Asn Gln Pro Glu Pro Val Lys
1               5                   10


<210> 172
<211> 21
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 172
Asp Leu Xaa Ala Asn Asn Val Met Ser Gly Gly Thr Thr Met Tyr Pro
1               5                   10                  15

Gly Ile Ala Asp Arg
            20


<210> 173
<211> 10
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 173
Gly Xaa Ser Phe Val Thr Thr Ala Glu Arg
1               5                   10


<210> 174
<211> 10
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 174
Gly Xaa Ser Phe Thr Thr Thr Ala Glu Arg
1               5                   10


<210> 175
<211> 31
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 175
Leu Gly Asp Glu Lys Leu Gln Ala Pro Met Ala Leu Phe Xaa Pro Ala
1               5                   10                  15

Thr Phe Gly Ile Val Gly Gln Lys Met Thr Thr Leu Gln His Arg
            20                  25                  30

<210> 176
<211> 14
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 176
Tyr Phe Asp Arg Ile Asn Glu Asn Asp Pro Glu Xaa Ile Arg
1               5                   10

<210> 177
<211> 19
<212> PRT
<213> Artificial Sequence

<220>
<223> Description of Artificial Sequence: Synthetic peptide

<220>
<221> MOD_RES
<222> (17)..(17)
<223> Phosphorylated Tyr

<400> 177
Ile His Gly Ser Gly His Val Glu Glu Pro Ala Ser Pro Leu Ala Ala
1               5                   10                  15

Xaa Gln Lys

<210> 178
<211> 33
<212> PRT
<213> Artificial Sequence

<220>
<223> Description of Artificial Sequence: Synthetic peptide

<220>
<221> MOD_RES
<222> (29)..(29)
<223> Phosphorylated Tyr

<400> 178
His Arg Gly Asn Glu Val Ser Thr Thr Pro Leu Leu Ala Asn Ser Leu
1               5                   10                  15

Ser Val His Gln Leu Ala Ala Gln Gly Glu Met Leu Xaa Leu Ala Thr
            20                  25                  30

Arg

<210> 179
<211> 23
<212> PRT
<213> Artificial Sequence

<220>
<223> Description of Artificial Sequence: Synthetic peptide

<220>
<221> MOD_RES
<222> (22)..(22)
<223> Phosphorylated Tyr

<400> 179
Lys Thr Thr Ser Tyr Pro Thr Pro Arg Pro Tyr Pro Lys Pro Ala Pro
1               5                   10                  15

Ser Ser Gly Lys Asp Xaa Val
            20

<210> 180
<211> 18
<212> PRT
<213> Artificial Sequence

<220>
<223> Description of Artificial Sequence: Synthetic peptide

<220>
<221> MOD_RES
<222> (17)..(17)
<223> Phosphorylated Tyr

<400> 180
Ser Thr Gly Pro Gly Ala Ser Leu Gly Thr Gly Tyr Asp Arg Lys Asp

1               5                   10                  15

Xaa Val

<210> 181
<211> 17
<212> PRT
<213> Artificial Sequence

<220>
<223> Description of Artificial Sequence: Synthetic peptide

<220>
<221> MOD_RES
<222> (13)..(13)
<223> Phosphorylated Tyr

<400> 181
His Val Phe Gly Gln Pro Ala Lys Ala Asp Gln Cys Xaa Glu Asp Val
1               5                   10                  15

Arg

<210> 182
<211> 14
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 182
Gln Leu Gln Tyr Cys Pro Ser Val Glu Ser Pro Xaa Ser Lys
1               5                   10

<210> 183
<211> 33
<212> PRT
<213> Artificial Sequence

<220>
<223> Description of Artificial Sequence: Synthetic peptide

<220>
<221> MOD_RES
<222> (5)..(5)

<223> Phosphorylated Tyr

<400> 183
Ser Thr Gly Asn Xaa Val Asp Phe Tyr Ser Ala Ala Arg Pro Tyr Ser
1               5                   10                  15

Glu Leu Asn Tyr Glu Thr Ser His Tyr Pro Ala Ser Pro Asp Ser Trp
            20                  25                  30

Val

<210> 184
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 184
Gln Ser Ala Val Gly Phe Glu Xaa Gln Gly Lys Thr Glu Lys His
1               5                   10                  15

<210> 185
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 185
Ser Phe Lys Ala Glu Leu Ser Xaa Arg Gly Pro Val Ser Gly Thr
1               5                   10                  15

<210> 186
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 186
Ser Ser Gln Gln Gly Leu Ala Xaa Ala Thr Glu Ala Val Tyr Glu
1               5                   10                  15


<210> 187
<211> 20
<212> PRT
<213> Artificial Sequence

<220>
<223> Description of Artificial Sequence: Synthetic peptide


<220>
<221> MOD_RES
<222> (9)..(9)
<223> Phosphorylated Tyr

<400> 187
Phe Gln Arg Val Asn Phe Gly Pro Xaa Asp Asn Tyr Ile Pro Val Ser
1               5                   10                  15


Glu Leu Ser Lys
            20


<210> 188
<211> 14
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 188
Asn Met Thr Pro Xaa Arg Ser Pro Pro Pro Tyr Val Pro Pro
1               5                   10


<210> 189
<211> 24
<212> PRT
<213> Artificial Sequence

<220>
<223> Description of Artificial Sequence: Synthetic peptide

<220>
<221> MOD_RES
<222> (22)..(22)
<223> Phosphorylated Tyr

<400> 189
Ala Pro His Leu Gln Leu Ile Glu Gly Lys Lys Asn Ser Leu Arg Val
1               5                   10                  15

Glu Gly Asp Asn Ile Xaa Val Arg
            20

<210> 190
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 190
Thr Glu Thr Lys Thr Ile Thr Xaa Glu Ser Pro Gln Ile Asp Gly
1               5                   10                  15

<210> 191
<211> 22
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 191
Thr Leu Ala Glu His Lys Glu Leu Ile Asn Thr Gly Pro Pro Xaa Thr
1               5                   10                  15

Leu Tyr Phe Gly Ile Lys
            20

<210> 192
<211> 22
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 192
Thr Leu Ala Glu His Lys Glu Leu Ile Asn Thr Gly Pro Pro Tyr Thr
1               5                   10                  15

Leu Xaa Phe Gly Ile Lys
            20

<210> 193
<211> 16
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 193
Ser Xaa Glu Leu Pro Asp Gly Gln Val Ile Thr Ile Gly Asn Glu Arg
1               5                   10                  15

<210> 194
<211> 23
<212> PRT
<213> Artificial Sequence

<220>
<223> Description of Artificial Sequence: Synthetic peptide

<220>
<221> MOD_RES
<222> (17)..(17)
<223> Phosphorylated Tyr

<400> 194
Gln Met Lys Thr His Pro Pro Tyr Thr Met Cys Phe Arg Val Lys Phe
1               5                   10                  15

Xaa Pro His Glu Pro Leu Lys
            20

<210> 195
<211> 23
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 195
Gln Met Lys Thr His Pro Pro Xaa Thr Met Cys Phe Arg Val Lys Phe
1               5                   10                  15


Tyr Pro His Glu Pro Leu Lys
            20


<210> 196
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 196
His Gln Val Glu Ile Lys Val Xaa Lys Gln Lys Val Lys His Leu
1               5                   10                  15


<210> 197
<211> 18
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 197
Gln Ala Leu Asp Xaa Val Glu Leu Ser Pro Leu Thr Gln Ala Ser Pro
1               5                   10                  15

Gln Arg

<210> 198
<211> 25
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 198
Lys Thr Thr Thr Xaa Thr Gln Gly Val Pro Pro Ser Gln Gly Asp Leu
1               5                   10                  15

Glu Tyr Gln Met Ser Thr Thr Ala Arg
            20                  25

<210> 199
<211> 22
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 199
Met Asp Met Asp Gly Asp Tyr Pro Ile Asp Thr Xaa Ser Asp Gly Leu
1               5                   10                  15

Arg Pro Pro Tyr Pro Thr
            20

<210> 200
<211> 28
<212> PRT
<213> Artificial Sequence

<220>
<223> Description of Artificial Sequence: Synthetic peptide

<220>
<221> MOD_RES

<222> (10)..(10)
<223> Phosphorylated Tyr

<400> 200
Val Thr Glu Trp Gln Gln Thr Tyr Thr Xaa Asp Ser Gly Ile His Ser
1               5                   10                  15

Gly Ala Asn Thr Cys Val Pro Ser Val Ser Ser Lys
            20                  25

<210> 201
<211> 32
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 201
Gly Gly Phe Ser Gly Cys Ser Ala Val Leu Ser Gly Gly Ser Ser Ser
1               5                   10                  15

Ser Xaa Arg Ala Gly Gly Lys Gly Leu Ser Gly Gly Phe Ser Ser Arg
            20                  25                  30

<210> 202
<211> 9
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 202
Ala Gln Xaa Glu Asp Ile Ala Asn Arg
1               5

<210> 203
<211> 15
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 203
Asp Val Asp Glu Ala Xaa Met Asn Lys Val Glu Leu Glu Ser Arg
1               5                   10                  15


<210> 204
<211> 24
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 204
Gln Phe Ser Ser Ser Xaa Leu Thr Ser Gly Gly Gly Gly Gly Gly Gly
1               5                   10                  15


Leu Gly Ser Gly Gly Ser Ile Arg
            20


<210> 205
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 205
Arg Thr Pro Gln Ala Ser Thr Xaa Ser Tyr Glu Thr Ser Asp Leu
1               5                   10                  15


<210> 206
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 206
Ser Ala Gly His Thr Pro Tyr Xaa Gln Ser Pro Thr Asp Glu Lys
1               5                   10                  15


<210> 207
<211> 11
<212> PRT
<213> Artificial Sequence

<220>
<223> Description of Artificial Sequence: Synthetic peptide


<220>
<221> MOD_RES
<222> (9)..(9)
<223> Phosphorylated Tyr

<400> 207
Thr Ser Asp Val Gly Gly Tyr Tyr Xaa Glu Lys
1               5                   10


<210> 208
<211> 24
<212> PRT
<213> Artificial Sequence

<220>
<223> Description of Artificial Sequence: Synthetic peptide


<220>
<221> MOD_RES
<222> (17)..(17)
<223> Phosphorylated Tyr

<400> 208
Gln His Ser Leu Pro Ser Ser Glu His Leu Gly Ala Asp Gly Gly Leu
1               5                   10                  15


Xaa Gln Ile Pro Pro Gln Pro Arg
            20


<210> 209
<211> 21
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 209
Ala Lys Arg Gly Gln Lys Leu Gln Ser Gln Xaa Leu Tyr Val Glu Leu
1               5                   10                  15

Ala Thr Lys Glu Arg
            20

<210> 210
<211> 23
<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 210
Lys Asn Tyr Glu Asn Thr Lys Thr Ser Xaa His Thr Pro Gly Asp Met
1               5                   10                  15

Val Thr Ile Thr Ala Ala Lys
            20

<210> 211
<211> 21
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 211
Ala Lys Arg Gly Gln Lys Leu Gln Ser Gln Xaa Leu Tyr Val Glu Leu
1               5                   10                  15

Ala Thr Lys Glu Arg
            20

<210> 212
<211> 15
<212> PRT
<213> Artificial Sequence

<220>
<223> Description of Artificial Sequence: Synthetic peptide


<220>
<221> MOD_RES
<222> (1)..(1)
<223> Phosphorylated Tyr

<400> 212
Xaa Thr Pro Val Pro Asp Thr Pro Ile Leu Ile Arg Ala Lys Arg
1               5                   10                  15


<210> 213
<211> 30
<212> PRT
<213> Artificial Sequence

<220>
<223> Description of Artificial Sequence: Synthetic peptide


<220>
<221> MOD_RES
<222> (30)..(30)
<223> Phosphorylated Tyr

<400> 213
Thr Pro Gly Asp Met Val Thr Ile Thr Ala Ala Lys Met Ala Gln Asp
1               5                   10                  15


Val Ala Thr Asn Val Asn Tyr Lys Gln Pro Leu His His Xaa
            20                  25                  30


<210> 214
<211> 18
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 214
Gly Tyr Tyr Ser Pro Xaa Ser Val Ser Gly Ser Gly Ser Thr Ala Gly
1               5                   10                  15

Ser Arg

<210> 215
<211> 18
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 215
Gly Tyr Tyr Ser Pro Xaa Ser Val Ser Gly Ser Gly Ser Thr Ala Gly
1               5                   10                  15

Ser Arg

<210> 216
<211> 19
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 216
Asp Ala Ser Val Ala Glu Ala Trp Leu Leu Gly Gln Glu Pro Xaa Leu
1               5                   10                  15

Ser Ser Arg

<210> 217
<211> 17
<212> PRT
<213> Artificial Sequence

<220>
<223> Description of Artificial Sequence: Synthetic peptide

<220>

<221> MOD_RES
<222> (12)..(12)
<223> Phosphorylated Tyr

<400> 217
Asn Leu Thr Ala Gly Asp Pro Ala Glu Thr Asp Xaa Thr Ala Val Gly
1               5                   10                  15

Cys

<210> 218
<211> 21
<212> PRT
<213> Artificial Sequence

<220>
<223> Description of Artificial Sequence: Synthetic peptide

<220>
<221> MOD_RES
<222> (19)..(19)
<223> Phosphorylated Tyr

<400> 218
Gln Leu Phe His Pro Glu Gln Leu Ile Thr Gly Lys Glu Asp Ala Ala
1               5                   10                  15

Asn Asn Xaa Ala Arg
            20

<210> 219
<211> 14
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 219
Thr Xaa Ser Leu Gly Ser Ala Leu Arg Pro Ser Thr Ser Arg
1               5                   10

<210> 220
<211> 15
<212> PRT
<213> Artificial Sequence

<220>

<223> Description of Artificial Sequence: Synthetic peptide

<400> 220
Ala Asn Gly Pro Ala Ser His Phe Glu Thr Arg Pro Gln Thr Tyr
1               5                   10                  15


<210> 221
<211> 16
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 221
Ser Xaa His Val Gln Glu Ser Leu Gln Asp Glu Gly Ala Glu Pro Thr
1               5                   10                  15


<210> 222
<211> 12
<212> PRT
<213> Artificial Sequence

<220>
<223> Description of Artificial Sequence: Synthetic peptide


<220>
<221> MOD_RES
<222> (11)..(11)
<223> Phosphorylated Tyr

<400> 222
Met Gln Asn His Gly Tyr Glu Asn Pro Thr Xaa Lys
1               5                   10


<210> 223
<211> 25
<212> PRT
<213> Artificial Sequence

<220>
<223> Description of Artificial Sequence: Synthetic peptide


<220>
<221> MOD_RES
<222> (16)..(16)
<223> Phosphorylated Tyr

<400> 223
Gly Arg Leu Asp Ser Ser Glu Met Asp His Ser Glu Asn Glu Asp Xaa

1               5                   10                  15

Thr Met Ser Ser Pro Leu Pro Gly Lys
            20                  25

<210> 224
<211> 10
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 224
Lys Glu Ser Tyr Ser Val Xaa Val Tyr Lys
1               5                   10

<210> 225
<211> 9
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 225
Glu Ser Tyr Ser Ile Xaa Val Tyr Lys
1               5

<210> 226
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 226
Val Thr Ala Met Asp Val Val Xaa Ala Leu Lys Arg Gln Gly Arg

1                   5                   10                  15

<210> 227
<211> 32
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 227
Val Glu Leu Ile Ala Xaa Phe Glu Lys Val Gly Asp Thr Ser Leu Asp
1               5                   10                  15

Pro Asn Asp Phe Asp Phe Thr Val Thr Gly Arg Gly Ser Pro Ser Arg
            20                  25                  30

<210> 228
<211> 15
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 228
Asp Leu Ala Gln Xaa Asp Ala Ala His His Glu Glu Phe Lys Arg
1               5                   10                  15

<210> 229
<211> 12
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 229
Ala Arg Asp Xaa Asp Ala Met Gly Ser Gln Thr Lys

1                   5                   10

<210> 230
<211> 30
<212> PRT
<213> Artificial Sequence

<220>
<223> Description of Artificial Sequence: Synthetic peptide

<220>
<221> MOD_RES
<222> (17)..(17)
<223> Phosphorylated Tyr

<400> 230
Pro Asp Gly Pro Gly Gly Gly Pro Gly Gly Ser His Met Gly Gly Asn
1               5                   10                  15

Xaa Gly Asp Asp Arg Arg Gly Gly Arg Gly Gly Tyr Asp Arg
            20                  25                  30

<210> 231
<211> 19
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 231
Pro Glu Xaa Ser Ala Ser Gln Leu Lys Gly Phe Ser Leu Leu Ala Thr
1               5                   10                  15

Glu Asp Lys

<210> 232
<211> 19
<212> PRT
<213> Artificial Sequence

<220>
<223> Description of Artificial Sequence: Synthetic peptide

<220>
<221> MOD_RES
<222> (14)..(14)

<223> Phosphorylated Tyr

<400> 232
Cys Thr His Leu Ile Val Pro Glu Pro Lys Gly Glu Lys Xaa Glu Cys
1               5                   10                  15

Ala Leu Lys

<210> 233
<211> 16
<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 233
Leu Met Ala Tyr Leu Ala Gly Ala Lys Xaa Thr Gly Tyr Leu Cys Arg
1               5                   10                  15

<210> 234
<211> 16
<212> PRT
<213> Artificial Sequence

<220>
<223> Description of Artificial Sequence: Synthetic peptide

<220>
<221> MOD_RES
<222> (13)..(13)
<223> Phosphorylated Tyr

<400> 234
Leu Met Ala Tyr Leu Ala Gly Ala Lys Tyr Thr Gly Xaa Leu Cys Arg
1               5                   10                  15

<210> 235
<211> 15
<212> PRT
<213> Artificial Sequence

<220>
<223> Description of Artificial Sequence: Synthetic peptide

<220>
<221> MOD_RES
<222> (13)..(13)

<223> Phosphorylated Tyr

<400> 235
Ala Phe His Glu Leu Ser Arg Glu Glu Gln Ala Lys Xaa Glu Lys
1               5                   10                  15


<210> 236
<211> 31
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 236
Ser Xaa Met Leu Gln Arg Trp Gln Glu Asp Gly Gly Val Met Ile Ile
1               5                   10                  15


Gly Tyr Glu Met Tyr Arg Asn Leu Ala Gln Gly Arg Asn Val Lys
            20                  25                  30


<210> 237
<211> 13
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 237
Leu Thr Val Glu Phe Met His Xaa Ile Ile Ala Ala Arg
1               5                   10


<210> 238
<211> 25
<212> PRT
<213> Artificial Sequence

<220>
<223> Description of Artificial Sequence: Synthetic peptide


<220>
<221> MOD_RES
<222> (23)..(23)

<223> Phosphorylated Tyr

<400> 238
Asp Pro Asn Gly Pro Thr His Ser Ser Thr Leu Phe Val Arg Asp Asp
1               5                   10                  15

Gly Ser Ser Met Ser Phe Xaa Val Arg
            20                  25

<210> 239
<211> 15
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 239
Phe Asp Thr Gln Tyr Pro Xaa Gly Glu Lys Gln Asp Glu Phe Lys
1               5                   10                  15

<210> 240
<211> 30
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 240
Ala Ile Phe Asp Thr Pro Asp Glu Asp Pro Asn Xaa Asn Pro Leu Pro
1               5                   10                  15

Glu Glu Arg Pro Gly Gly Phe Ala Trp Gly Glu Gly Gln Arg
            20                  25                  30

<210> 241
<211> 19
<212> PRT
<213> Artificial Sequence

<220>
<223> Description of Artificial Sequence: Synthetic peptide

<220>
<221> MOD_RES
<222> (16)..(16)
<223> Phosphorylated Tyr

<400> 241
Glu Ile Phe Asp Ser Arg Gly Asn Pro Thr Val Glu Val Asp Leu Xaa
1               5                   10                  15

Thr Ala Lys

<210> 242
<211> 23
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 242
Ala Ala Asn Leu Xaa Ala Ser Ser Pro His Ser Asp Phe Leu Asp Tyr
1               5                   10                  15

Val Ser Ala Pro Ile Gly Lys
            20

<210> 243
<211> 24
<212> PRT
<213> Artificial Sequence

<220>
<223> Description of Artificial Sequence: Synthetic peptide

<220>
<221> MOD_RES
<222> (20)..(20)
<223> Phosphorylated Tyr

<400> 243
Cys Trp Gly Arg Asp Thr Phe Ile Ala Leu Arg Gly Ile Leu Leu Ile
1               5                   10                  15

Thr Gly Arg Xaa Val Glu Ala Arg
            20

<210> 244
<211> 15
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 244
Phe Thr Asp Phe Xaa Val Pro Val Ser Leu Cys Thr Pro Ser Arg
1               5                   10                  15


<210> 245
<211> 17
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 245
Leu Pro Val Arg Met Gly Met Xaa Pro Gly Val Leu Val Pro Ser Ser
1               5                   10                  15


Arg



<210> 246
<211> 28
<212> PRT
<213> Artificial Sequence

<220>
<223> Description of Artificial Sequence: Synthetic peptide


<220>
<221> MOD_RES
<222> (21)..(21)
<223> Phosphorylated Tyr

<400> 246
Gln Asn Lys Thr Thr Leu Thr Tyr Val Ala Ala Val Ala Val Gly Met
1               5                   10                  15

Leu Gly Ala Ser Xaa Ala Ala Val Pro Leu Tyr Arg
            20                  25

<210> 247
<211> 24
<212> PRT
<213> Artificial Sequence

<220>
<223> Description of Artificial Sequence: Synthetic peptide

<220>
<221> MOD_RES
<222> (13)..(13)
<223> Phosphorylated Tyr

<400> 247
Asp Met Ser Lys Ser Leu Thr Asn Phe Ser Lys Val Xaa Gly Pro Val
1               5                   10                  15

Phe Thr Val Tyr Phe Gly Leu Lys
            20

<210> 248
<211> 18
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 248
Tyr Gly Ile Val Leu Asp Ala Gly Ser Ser His Thr Ser Leu Xaa Ile
1               5                   10                  15

Tyr Lys

<210> 249
<211> 19
<212> PRT
<213> Artificial Sequence

<220>
<223> Description of Artificial Sequence: Synthetic peptide

<220>
<221> MOD_RES

<222> (12)..(12)
<223> Phosphorylated Tyr

<400> 249
Gln Gly Pro Trp Leu Glu Glu Glu Glu Glu Ala Xaa Gly Trp Met Asp
1               5                   10                  15

Phe Gly Arg

<210> 250
<211> 13
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 250
Gly His Phe Xaa Gly His Leu Asp Phe Asn Leu Asp Lys
1               5                   10

<210> 251
<211> 26
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 251
Ala Asp Gln Asp Ile Asn Tyr Xaa Ile Pro Ala Glu Asp Phe Ser Gly
1               5                   10                  15

Leu Ala Val Ile Asp Trp Glu Tyr Trp Arg
            20                  25

<210> 252
<211> 26
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 252
Ala Asp Gln Asp Ile Asn Xaa Tyr Ile Pro Ala Glu Asp Phe Ser Gly
1               5                   10                  15

Leu Ala Val Ile Asp Trp Glu Tyr Trp Arg
            20                  25

<210> 253
<211> 20
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 253
Ser Leu Ala Glu Gly Xaa Phe Asp Ala Ala Gly Arg Leu Thr Pro Glu
1               5                   10                  15

Phe Ser Gln Arg
            20

<210> 254
<211> 25
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 254
Ser Ile Met Ala Ala Ala Gly Val Pro Val Val Glu Gly Xaa His Gly
1               5                   10                  15

Glu Asp Gln Ser Asp Gln Cys Leu Lys
            20                  25

<210> 255
<211> 18
<212> PRT
<213> Artificial Sequence

<220>
<223> Description of Artificial Sequence: Synthetic peptide


<220>
<221> MOD_RES
<222> (11)..(11)
<223> Phosphorylated Tyr

<400> 255
Ala Lys Val Pro Ile Trp Lys Lys Glu Ile Xaa Glu Glu Ser Ser Thr
1               5                   10                  15


Trp Lys



<210> 256
<211> 15
<212> PRT
<213> Artificial Sequence

<220>
<223> Description of Artificial Sequence: Synthetic peptide


<220>
<221> MOD_RES
<222> (12)..(12)
<223> Phosphorylated Tyr

<400> 256
Ile Val Ser Leu Pro Glu Cys Phe Asn Ser Pro Xaa Gly Ala Lys
1               5                   10                  15


<210> 257
<211> 25
<212> PRT
<213> Artificial Sequence

<220>
<223> Description of Artificial Sequence: Synthetic peptide


<220>
<221> MOD_RES
<222> (16)..(16)
<223> Phosphorylated Tyr

<400> 257
Leu Ala Lys Val Asp Ala Thr Glu Glu Ser Asp Leu Ala Gln Gln Xaa
1               5                   10                  15

Gly Val Arg Gly Tyr Pro Thr Ile Lys
            20                  25

<210> 258
<211> 17
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 258
Val Glu Leu Phe His Xaa Gln Asp Gly Ala Phe His Thr Glu Tyr Asn
1               5                   10                  15

Arg

<210> 259
<211> 22
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 259
Val Xaa Met Gly Glu Met Gly Arg Leu Lys Ser Tyr Glu Asn Gln Lys
1               5                   10                  15

Pro Pro Phe Asp Ala Lys
            20

<210> 260
<211> 16
<212> PRT
<213> Artificial Sequence

<220>
<223> Description of Artificial Sequence: Synthetic peptide

<220>

<221> MOD_RES
<222> (6)..(6)
<223> Phosphorylated Tyr

<400> 260
Glu Leu Asn Lys Leu Xaa Pro Thr His Ala Cys Arg Glu Tyr Leu Lys
1               5                   10                  15


<210> 261
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 261
Asp Leu Asn Gly Gln Ala Val Xaa Ala Ala Cys Gln Thr Ile Leu
1               5                   10                  15


<210> 262
<211> 22
<212> PRT
<213> Artificial Sequence

<220>
<223> Description of Artificial Sequence: Synthetic peptide


<220>
<221> MOD_RES
<222> (13)..(13)
<223> Phosphorylated Tyr

<400> 262
Gln Arg Gly Tyr Lys Gly Leu Ile Gly Asp Asp Asn Xaa Leu Ala Leu
1               5                   10                  15


Lys Asn Ser Gln Gly Lys
            20


<210> 263
<211> 26
<212> PRT
<213> Artificial Sequence

<220>
<223> Description of Artificial Sequence: Synthetic peptide


<220>

<221> MOD_RES
<222> (17)..(17)
<223> Phosphorylated Tyr

<400> 263
Arg Ser Met Glu Glu Lys Val Thr Glu Lys Ser Ala Leu His Ser His
1               5                   10                  15

Xaa Cys Gly Ile Ile Ser Asp Lys Gly Arg
            20                  25

<210> 264
<211> 35
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 264
Gly Asp Ala Ser Ser Ile Val Ser Ala Ile Cys Xaa Thr Val Pro Lys
1               5                   10                  15

Ser Ala Met Gly Ser Ser Leu Tyr Ala Leu Glu Ser Gly Ser Asp Phe
            20                  25                  30

Lys Ser Arg
        35

<210> 265
<211> 17
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 265
Ala Pro Cys Gly Gly Arg Gly Arg Pro Gly Ile Arg Ser Xaa Gly Pro
1               5                   10                  15

Arg

<210> 266
<211> 22
<212> PRT
<213> Artificial Sequence

<220>
<223> Description of Artificial Sequence: Synthetic peptide


<220>
<221> MOD_RES
<222> (4)..(4)
<223> Phosphorylated Tyr

<400> 266
Gly Pro His Xaa Phe Tyr Trp Ser Arg Glu Asp Gly Arg Pro Val Pro
1               5                   10                  15


Ser Gly Thr Gln Gln Arg
            20


<210> 267
<211> 26
<212> PRT
<213> Artificial Sequence

<220>
<223> Description of Artificial Sequence: Synthetic peptide


<220>
<221> MOD_RES
<222> (21)..(21)
<223> Phosphorylated Tyr

<400> 267
Ile His Asp Gly Glu Ala Asp Ile Met Ile Asn Phe Gly Arg Trp Glu
1               5                   10                  15


His Gly Asp Gly Xaa Pro Phe Asp Gly Lys
            20                  25


<210> 268
<211> 17
<212> PRT
<213> Artificial Sequence

<220>
<223> Description of Artificial Sequence: Synthetic peptide


<220>
<221> MOD_RES
<222> (16)..(16)
<223> Phosphorylated Tyr

<400> 268
Glu Pro Gly Glu Gly Leu Ala Val Thr Val Ser Leu Ile Gly Ala Xaa
1               5                   10                  15

Lys

<210> 269
<211> 17
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 269
Lys Glu Xaa Ser Gln Asn Leu Thr Ser Glu Pro Thr Leu Leu Gln His
1               5                   10                  15

Arg

<210> 270
<211> 25
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 270
Arg Ala Xaa Ser Asn Leu Gly Asn Ala His Val Phe Leu Gly Arg Phe
1               5                   10                  15

Asp Val Ala Ala Glu Tyr Tyr Lys Lys
            20                  25

<210> 271
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 271
Val Pro Thr Val Phe Glu Asn Xaa Thr Ala Cys Leu Glu Thr Glu
1               5                   10                  15

<210> 272
<211> 16
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 272
Gly Lys Tyr Pro Asp Pro Ser Glu Asp Ala Asp Ser Ser Xaa Val Arg
1               5                   10                  15

<210> 273
<211> 14
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 273
Ser Asn Xaa Asn Phe Glu Lys Pro Phe Leu Trp Leu Ala Arg
1               5                   10

<210> 274
<211> 21
<212> PRT
<213> Artificial Sequence

<220>
<223> Description of Artificial Sequence: Synthetic peptide

<220>
<221> MOD_RES
<222> (20)..(20)
<223> Phosphorylated Tyr

<400> 274
Asp Arg Asp Leu Val Thr Glu Asp Thr Gly Val Arg Asn Glu Ala Gln
1               5                   10                  15

Glu Glu Ile Xaa Lys
            20

<210> 275
<211> 16
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 275
Ala Ala Ser Gln Ser Thr Thr Asp Xaa Asn Gln Val Val Pro Asn Arg
1               5                   10                  15

<210> 276
<211> 13
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 276
Ile Ile Ala Met Cys Gly Asp Xaa Tyr Ile Gly Gly Arg
1               5                   10

<210> 277
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 277
Ala Cys Gly Ser Lys Ser Val Xaa Asp Gly Pro Glu Gln Glu Glu
1               5                   10                  15


<210> 278
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 278
Lys Pro Lys Arg Gly Ile Gln Xaa Leu Gln Glu Gln Gly Met Leu
1               5                   10                  15


<210> 279
<211> 33
<212> PRT
<213> Artificial Sequence

<220>
<223> Description of Artificial Sequence: Synthetic peptide


<220>
<221> MOD_RES
<222> (14)..(14)
<223> Phosphorylated Tyr

<400> 279
Ala Val Leu Arg Lys Phe Phe Leu Arg Ile Ser Val Val Xaa Lys Ile
1               5                   10                  15


Trp Ile Pro Glu Glu Pro Ser Gln Val Pro Ala Ala Leu Ser Pro Val
            20                  25                  30


Trp


<210> 280
<211> 19
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 280
Ser Gly Arg Asp Xaa Ser Thr Val Ser Ala Ser Pro Thr Ala Leu Ser
1               5                   10                  15

Thr Leu Lys

<210> 281
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 281
Arg Lys Gln Ala Leu Glu Gln Xaa Glu Glu Val Lys Lys Lys Leu
1               5                   10                  15

<210> 282
<211> 11
<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 282
Gln Leu Thr Leu Leu Glu Ser Asp Leu Xaa Arg
1               5                   10

<210> 283
<211> 19
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 283
Xaa Pro Phe Lys Lys Arg Ala Ser Leu Gln Ala Ser Thr Ala Ala Pro
1               5                   10                  15

Glu Ala Arg

<210> 284
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 284
Val Cys Met Val Tyr Asp Leu Xaa Lys Thr Leu Thr Pro Ile Ser
1               5                   10                  15

<210> 285
<211> 14
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 285
Xaa Arg Gly Ala Ile Ala Arg Lys Arg Ile Arg Leu Gly Arg
1               5                   10

<210> 286
<211> 22
<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 286
Tyr Tyr Ile Ala Ala Ser Tyr Val Lys Xaa Leu Glu Ser Ala Gly Ala
1               5                   10                  15

Arg Val Val Pro Val Arg
            20

<210> 287
<211> 22
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 287
Lys Leu Val Gln Thr Thr Xaa Glu Cys Leu Met Gln Ala Ile Asp Ala
1               5                   10                  15

Val Lys Pro Gly Val Arg
            20

<210> 288
<211> 30
<212> PRT
<213> Artificial Sequence

<220>
<223> Description of Artificial Sequence: Synthetic peptide

<220>
<221> MOD_RES
<222> (21)..(21)
<223> Phosphorylated Tyr

<400> 288
Ile Leu Leu Arg Met Thr Leu Gly Arg Glu Val Met Ser Pro Leu Gln
1               5                   10                  15

Ala Met Ser Ser Xaa Thr Val Ala Gly Arg Asn Val Leu Arg

20                  25                  30

<210> 289
<211> 33
<212> PRT
<213> Artificial Sequence

<220>
<223> Description of Artificial Sequence: Synthetic peptide

<220>
<221> MOD_RES
<222> (28)..(28)
<223> Phosphorylated Tyr

<400> 289
Gln Ala Glu Ala Ile Met Gly Ala Pro Gly Pro Ser Leu Thr Gly Ser
1               5                   10                  15

Pro Trp Pro Gly Thr Ala Ala Pro Ala Ala Ser Xaa Thr Pro Thr Pro
            20                  25                  30

Arg

<210> 290
<211> 26
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 290
Phe Ile Thr Ser Ser Ala Ser Asp Phe Ser Asp Pro Val Xaa Lys Glu
1               5                   10                  15

Ile Ala Ile Thr Asn Gly Cys Ile Asn Arg
            20                  25

<210> 291
<211> 32
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 291
Xaa Arg Glu Leu Leu Ala Lys Pro Ile Gly Pro Asp Asp Ala Ile Asp
1               5                   10                  15

Ala Leu Ser Ser Asp Phe Thr Cys Gly Ser Pro Thr Ala Ala Gly Lys
            20                  25                  30

<210> 292
<211> 9
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 292
Ala Lys His Asp Glu Leu Thr Xaa Phe
1               5

<210> 293
<211> 20
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 293
Leu Lys Ala Lys Xaa Pro Ser Leu Gly Gln Lys Pro Gly Gly Ser Asp
1               5                   10                  15

Phe Leu Met Lys
            20

<210> 294
<211> 1[?]
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 294
Tyr Phe Asp Ser Gly Asp Xaa Asn Met Ala Lys
1               5                   10

<210> 295
<211> 22
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 295
Trp Ala Ala Gln Thr Gly Phe Val Glu Asn Ile Asn Thr Ile Leu Lys
1               5                   10                  15

Glu Xaa Lys Gln Ser Arg
            20

<210> 296
<211> 27
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 296
Ala Ala Lys Gly Ile Pro Val Met Gly His Ser Glu Gly Ile Cys His
1               5                   10                  15

Met Xaa Val Asp Ser Glu Ala Ser Val Asp Lys
            20                  25

<210> 297
<211> 14
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 297
Pro Xaa Glu Ser Ile Pro His Gln Tyr Lys Leu Asp Gly Lys
1               5                   10


<210> 298
<211> 14
<212> PRT
<213> Artificial Sequence

<220>
<223> Description of Artificial Sequence: Synthetic peptide


<220>
<221> MOD_RES
<222> (9)..(9)
<223> Phosphorylated Tyr

<400> 298
Gly Gly Asp Asp Leu Asp Pro Asn Xaa Val Leu Ser Ser Arg
1               5                   10


<210> 299
<211> 32
<212> PRT
<213> Artificial Sequence

<220>
<223> Description of Artificial Sequence: Synthetic peptide


<220>
<221> MOD_RES
<222> (18)..(18)
<223> Phosphorylated Tyr

<400> 299
Ser Arg Pro Glu Ala Val Ser His Pro Leu Asn Thr Val Thr Glu Asp
1               5                   10                  15


Met Xaa Thr Asn Gly Ser Pro Ala Pro Gly Ser Pro Ala Gln Val Lys
            20                  25                  30

<210> 300
<211> 26
<212> PRT
<213> Artificial Sequence

<220>
<223> Description of Artificial Sequence: Synthetic peptide


<220>
<221> MOD_RES
<222> (17)..(17)
<223> Phosphorylated Tyr

<400> 300
Val Asn Val Phe Ser Arg Gln Leu Val Leu Ile Asp Tyr Gly Asp Gln
1               5                   10                  15


Xaa Thr Ala Arg Gln Leu Gly Ser Arg Lys
            20                  25


<210> 301
<211> 26
<212> PRT
<213> Artificial Sequence

<220>
<223> Description of Artificial Sequence: Synthetic peptide


<220>
<221> MOD_RES
<222> (13)..(13)
<223> Phosphorylated Tyr

<400> 301
Val Asn Val Phe Ser Arg Gln Leu Val Leu Ile Asp Xaa Gly Asp Gln
1               5                   10                  15


Tyr Thr Ala Arg Gln Leu Gly Ser Arg Lys
            20                  25


<210> 302
<211> 32
<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 302

Gly Ser Leu Ser Gly Asp Xaa Leu Tyr Ile Phe Asp Gly Ser Asp Gly
1               5                   10                  15

Gly Val Ser Ser Ser Pro Gly Pro Gly Asp Ile Glu Gly Ser Cys Lys
            20                  25                  30

<210> 303
<211> 23
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 303
Asn Glu Asp Ala Asp Glu Asn Xaa Phe Ile Asn Glu Glu Asp Glu Asn
1               5                   10                  15

Leu Pro His Tyr Asp Glu Lys
            20

<210> 304
<211> 10
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 304
Phe Lys Thr Xaa Ala Pro Val Ala Phe Arg
1               5                   10

<210> 305
<211> 18
<212> PRT
<213> Artificial Sequence

<220>
<223> Description of Artificial Sequence: Synthetic peptide

<220>
<221> MOD_RES

<222> (8)..(8)
<223> Phosphorylated Tyr

<400> 305
Phe Leu Leu Arg Arg Gly Glu Xaa Val Leu His Met Trp Gln Ile Ser
1               5                   10                  15

Gly Lys

<210> 306
<211> 13
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 306
Asp Asn Asn Xaa Leu Pro Tyr Pro Ile His Gln Val Arg
1               5                   10

<210> 307
<211> 8
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 307
Ile Xaa Gln Tyr Ile Gln Ser Arg
1               5

<210> 308
<211> 17
<212> PRT
<213> Artificial Sequence

<220>
<223> Description of Artificial Sequence: Synthetic peptide

<220>
<221> MOD_RES

<222> (12)..(12)
<223> Phosphorylated Tyr

<400> 308
Leu Gln Glu Asp Leu Val Leu Arg Met Leu Glu Xaa Glu Pro Ala Ala
1               5                   10                  15

Arg

<210> 309
<211> 34
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 309
Gln Leu Ile Asp Leu Gly Xaa Leu Ser Ser Ser His Trp Asn Cys Gly
1               5                   10                  15

Ala Pro Gly Gln Asp Thr Lys Ala Gln Ser Met Leu Val Glu Gln Ser
            20                  25                  30

Glu Lys

<210> 310
<211> 33
<212> PRT
<213> Artificial Sequence

<220>
<223> Description of Artificial Sequence: Synthetic peptide

<220>
<221> MOD_RES
<222> (24)..(24)
<223> Phosphorylated Tyr

<400> 310
Trp Arg Thr Glu Tyr Ala Ile Lys Cys Ala Pro Cys Leu Pro Pro Asp
1               5                   10                  15

Ala Ala Ser Ser Asp Val Asn Xaa Leu Ile Glu Glu Ala Ala Lys Met
            20                  25                  30

Lys

<210> 311
<211> 33
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 311
Trp Arg Thr Glu Xaa Ala Ile Lys Cys Ala Pro Cys Leu Pro Pro Asp
1               5                   10                  15

Ala Ala Ser Ser Asp Val Asn Tyr Leu Ile Glu Glu Ala Ala Lys Met
            20                  25                  30

Lys

<210> 312
<211> 16
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 312
Gly Xaa Leu Ser Pro Asp Leu Ser Lys Ile Ser Ser Asn Cys Pro Lys
1               5                   10                  15

<210> 313
<211> 31
<212> PRT
<213> Artificial Sequence

<220>
<223> Description of Artificial Sequence: Synthetic peptide

<220>

<221> MOD_RES
<222> (12)..(12)
<223> Phosphorylated Tyr

<400> 313
Phe Asp Ile Ile Gly Ile Ile Gly Glu Gly Thr Xaa Gly Gln Val Tyr
1               5                   10                  15

Lys Ala Arg Asp Lys Asp Thr Gly Glu Met Val Ala Leu Lys Lys
            20                  25                  30

<210> 314
<211> 22
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 314
Asn Lys Pro Xaa Ile Ser Trp Pro Ser Ser Gly Gly Ser Glu Pro Ser
1               5                   10                  15

Val Thr Val Pro Leu Arg
            20

<210> 315
<211> 26
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 315
Cys Ser Pro Ser Gly Asp Ser Glu Gly Gly Glu Xaa Cys Ser Ile Leu
1               5                   10                  15

Asp Cys Cys Pro Gly Ser Pro Val Ala Lys
            20                  25

<210> 316
<211> 19

<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 316
Lys Leu Glu Arg Xaa Leu Ser Gly Lys Ser Asp Ile Gln Asp Ser Leu
1               5                   10                  15


Cys Tyr Lys



<210> 317
<211> 24
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 317
Leu Gly Gly Gly Thr Xaa Gly Glu Val Phe Lys Ala Arg Asp Lys Val
1               5                   10                  15


Ser Gly Asp Leu Val Ala Leu Lys
            20


<210> 318
<211> 30
<212> PRT
<213> Artificial Sequence

<220>
<223> Description of Artificial Sequence: Synthetic peptide


<220>
<221> MOD_RES
<222> (14)..(14)
<223> Phosphorylated Tyr

<400> 318
Val Gln Arg Ser Val Ser Ser Ser Gln Lys Gln Arg Arg Xaa Ser Asp
1               5                   10                  15

His Ala Gly Pro Ala Ile Pro Ser Val Val Ala Tyr Pro Lys
            20                  25                  30

<210> 319
<211> 30
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 319
Ile Ile Lys Asp Val Val Leu Gln Trp Gly Glu Met Pro Thr Ser Val
1               5                   10                  15

Ala Xaa Ile Cys Ser Asn Gln Ile Met Gly Trp Gly Glu Lys
            20                  25                  30

<210> 320
<211> 25
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 320
Arg Ile Pro Tyr Arg Xaa Ser Asp Glu Leu Asn Glu Ile Ile Thr Arg
1               5                   10                  15

Met Leu Asn Leu Lys Asp Tyr His Arg
            20                  25

<210> 321
<211> 28
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 321
Asn Glu Xaa Ser Ile Pro Lys His Ile Asn Pro Val Ala Ala Ser Leu
1               5                   10                  15

Ile Gln Lys Met Leu Gln Thr Asp Pro Thr Ala Arg
            20                  25

<210> 322
<211> 16
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 322
Xaa Tyr Leu Arg Gln Ile Leu Ser Gly Leu Lys Tyr Leu His Gln Arg
1               5                   10                  15

<210> 323
<211> 16
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 323
Tyr Xaa Leu Arg Gln Ile Leu Ser Gly Leu Lys Tyr Leu His Gln Arg
1               5                   10                  15

<210> 324
<211> 29
<212> PRT
<213> Artificial Sequence

<220>
<223> Description of Artificial Sequence: Synthetic peptide

<220>
<221> MOD_RES
<222> (25)..(25)
<223> Phosphorylated Tyr

<400> 324
Gly Ile Ile Tyr Arg Asp Leu Lys Leu Asp Asn Val Leu Leu Asp Ser
1               5                   10                  15

Glu Gly His Ile Lys Leu Thr Asp Xaa Gly Met Cys Lys
            20                  25

<210> 325
<211> 9
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 325
Lys Ala Gln Asp Cys Xaa Phe Met Lys
1               5

<210> 326
<211> 28
<212> PRT
<213> Artificial Sequence

<220>
<223> Description of Artificial Sequence: Synthetic peptide

<220>
<221> MOD_RES
<222> (21)..(21)
<223> Phosphorylated Tyr

<400> 326
Pro Gly Val Thr Asn Val Ala Arg Ser Ala Met Met Lys Asp Ser Pro
1               5                   10                  15

Phe Tyr Gln His Xaa Asp Leu Asp Leu Lys Asp Lys
            20                  25

<210> 327
<211> 28
<212> PRT
<213> Artificial Sequence

<220>
<223> Description of Artificial Sequence: Synthetic peptide

<220>
<221> MOD_RES
<222> (18)..(18)
<223> Phosphorylated Tyr

<400> 327
Pro Gly Val Thr Asn Val Ala Arg Ser Ala Met Met Lys Asp Ser Pro
1               5                   10                  15

Phe Xaa Gln His Tyr Asp Leu Asp Leu Lys Asp Lys
            20                  25

<210> 328
<211> 31
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 328
Gln Xaa Glu His Val Lys Arg Asp Leu Asn Pro Glu Asp Phe Trp Glu
1               5                   10                  15

Ile Ile Gly Glu Leu Gly Asp Gly Ala Phe Gly Lys Val Tyr Lys
            20                  25                  30

<210> 329
<211> 31
<212> PRT
<213> Artificial Sequence

<220>
<223> Description of Artificial Sequence: Synthetic peptide

<220>
<221> MOD_RES
<222> (30)..(30)
<223> Phosphorylated Tyr

<400> 329
Gln Tyr Glu His Val Lys Arg Asp Leu Asn Pro Glu Asp Phe Trp Glu
1               5                   10                  15

Ile Ile Gly Glu Leu Gly Asp Gly Ala Phe Gly Lys Val Xaa Lys

20                  25                  30

<210> 330
<211> 21
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 330
Val Ser Thr His Ser Gln Glu Met Asp Ser Gly Thr Glu Xaa Gly Met
1               5                   10                  15

Gly Ser Ser Thr Lys
            20

<210> 331
<211> 26
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 331
Ser Thr Pro Ile Thr Ile Ala Arg Xaa Gly Arg Ser Arg Asn Lys Thr
1               5                   10                  15

Gln Asp Phe Glu Glu Leu Ser Ser Ile Arg
            20                  25

<210> 332
<211> 18
<212> PRT
<213> Artificial Sequence

<220>
<223> Description of Artificial Sequence: Synthetic peptide

<220>
<221> MOD_RES
<222> (14)..(14)

<223> Phosphorylated Tyr

<400> 332
Arg Gly Tyr Leu Leu Gly Ile Asn Leu Gly Glu Gly Ser Xaa Ala Lys
1               5                   10                  15

Val Lys

<210> 333
<211> 19
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 333
Pro Met Tyr Asp Gly Gly Thr Asp Ile Val Gly Xaa Val Leu Glu Met
1               5                   10                  15

Gln Glu Lys

<210> 334
<211> 19
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 334
Pro Met Tyr Asp Gly Gly Thr Asp Ile Val Gly Xaa Val Leu Glu Met
1               5                   10                  15

Gln Glu Lys

<210> 335
<211> 13
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 335
Val Glu Asn Leu Thr Glu Gly Ala Ile Tyr Xaa Phe Arg
1               5                   10

<210> 336
<211> 30
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 336
Val Thr Gly Leu Val Glu Gly Leu Glu Tyr Gln Phe Arg Thr Xaa Ala
1               5                   10                  15

Leu Asn Ala Ala Gly Val Ser Lys Ala Ser Glu Ala Ser Arg
            20                  25                  30

<210> 337
<211> 15
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 337
Xaa Gly Val Ser Gln Pro Leu Val Ser Ser Ile Ile Val Ala Lys
1               5                   10                  15

<210> 338
<211> 20
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 338
Val Gln Glu Asn Asp Gly Lys Glu Pro Pro Pro Val Val Asn Xaa Glu
1               5                   10                  15

Glu Asp Ala Arg
            20

<210> 339
<211> 12
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 339
Thr Leu Asp Asn Gly Gly Phe Xaa Ile Ser Pro Arg
1               5                   10

<210> 340
<211> 24
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 340
Leu Asp Ala Ser Arg Leu Leu Leu Xaa Ser Ser Gln Ile Cys Lys Gly
1               5                   10                  15

Met Glu Tyr Leu Gly Ser Arg Arg
            20

<210> 341
<211> 17
<212> PRT
<213> Artificial Sequence

<220>
<223> Description of Artificial Sequence: Synthetic peptide


<220>
<221> MOD_RES
<222> (9)..(9)
<223> Phosphorylated Tyr

<400> 341
Leu Gly Asp Phe Gly Leu Ser Arg Xaa Met Glu Asp Ser Thr Tyr Tyr
1               5                   10                  15


Lys



<210> 342
<211> 33
<212> PRT
<213> Artificial Sequence

<220>
<223> Description of Artificial Sequence: Synthetic peptide


<220>
<221> MOD_RES
<222> (17)..(17)
<223> Phosphorylated Tyr

<400> 342
Tyr Arg Pro Glu Asn Thr Pro Glu Pro Val Ser Thr Ser Val Ser His
1               5                   10                  15


Xaa Gly Ala Glu Pro Thr Thr Val Ser Pro Cys Pro Ser Ser Ser Ala
            20                  25                  30


Lys



<210> 343
<211> 26
<212> PRT
<213> Artificial Sequence

<220>
<223> Description of Artificial Sequence: Synthetic peptide


<220>
<221> MOD_RES

<222> (22)..(22)
<223> Phosphorylated Tyr

<400> 343
Arg Glu Glu Ile Pro Val Ser Asn Val Ala Glu Leu Leu His Gln Val
1               5                   10                  15

Ser Met Gly Met Lys Xaa Leu Glu Glu Lys
            20                  25

<210> 344
<211> 26
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 344
Gly Leu Ala Xaa Leu His Glu Asp Ile Pro Gly Leu Lys Asp Gly His
1               5                   10                  15

Lys Pro Ala Ile Ser His Arg Asp Ile Lys
            20                  25

<210> 345
<211> 35
<212> PRT
<213> Artificial Sequence

<220>
<223> Description of Artificial Sequence: Synthetic peptide

<220>
<221> MOD_RES
<222> (34)..(34)
<223> Phosphorylated Tyr

<400> 345
Glu Pro Pro Pro Tyr Gln Glu Pro Arg Pro Arg Gly Asn Pro Pro His
1               5                   10                  15

Ser Ala Pro Cys Val Pro Asn Gly Ser Ala Leu Leu Leu Ser Asn Pro
            20                  25                  30

Ala Xaa Arg
        35

<210> 346
<211> 9
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 346
Asn Leu Tyr Ala Gly Asp Tyr Xaa Arg
1               5


<210> 347
<211> 9
<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 347
Asn Leu Tyr Ala Gly Asp Xaa Tyr Arg
1               5


<210> 348
<211> 9
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 348
Asn Leu Tyr Ala Gly Asp Tyr Xaa Arg
1               5


<210> 349
<211> 15
<212> PRT

<213> Artificial Sequence

<220>
<223> Description of Artificial Sequence: Synthetic peptide


<220>
<221> MOD_RES
<222> (8)..(8)
<223> Phosphorylated Tyr

<400> 349
Gly Pro Glu Gly Val Pro His Xaa Ala Glu Ala Asp Ile Val Asn
1               5                   10                  15


<210> 350
<211> 14
<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 350
Ala Val Gly Asn Pro Glu Xaa Leu Asn Thr Val Gln Pro Thr
1               5                   10


<210> 351
<211> 17
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 351
Gly Ile Ala Ala Gly Met Lys Xaa Leu Ala Asn Met Asn Tyr Val His
1               5                   10                  15


Arg


<210> 352
<211> 21
<212> PRT

<213> Artificial Sequence

<220>
<223> Description of Artificial Sequence: Synthetic peptide

<220>
<221> MOD_RES
<222> (20)..(20)
<223> Phosphorylated Tyr

<400> 352
Phe Val Val Ile Gln Asn Glu Asp Leu Gly Pro Ala Ser Pro Leu Asp
1               5                   10                  15

Ser Thr Phe Xaa Arg
            20

<210> 353
<211> 12
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 353
Val Leu Gly Ser Gly Ala Phe Gly Thr Val Xaa Lys
1               5                   10

<210> 354
<211> 30
<212> PRT
<213> Artificial Sequence

<220>
<223> Description of Artificial Sequence: Synthetic peptide

<220>
<221> MOD_RES
<222> (26)..(26)
<223> Phosphorylated Tyr

<400> 354
Glu Gly Thr Leu Ser Ser Val Gly Leu Ser Ser Val Leu Gly Thr Glu
1               5                   10                  15

Glu Glu Asp Glu Asp Glu Glu Tyr Glu Xaa Met Asn Arg Arg
            20                  25                  30

<210> 355
<211> 13
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 355
Gly Glu Leu Asp Glu Glu Gly Xaa Met Thr Pro Met Arg
1               5                   10


<210> 356
<211> 29
<212> PRT
<213> Artificial Sequence

<220>
<223> Description of Artificial Sequence: Synthetic peptide


<220>
<221> MOD_RES
<222> (11)..(11)
<223> Phosphorylated Tyr

<400> 356
Ile Arg Pro Ile Val Ala Glu Asn Pro Glu Xaa Leu Ser Glu Phe Ser
1               5                   10                  15


Leu Lys Pro Gly Thr Val Leu Pro Pro Pro Pro Tyr Arg
            20                  25


<210> 357
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 357
Ser Thr Leu Gln His Pro Asp Xaa Leu Gln Glu Tyr Ser Thr Lys
1               5                   10                  15

<210> 358
<211> 15
<212> PRT
<213> Artificial Sequence

<220>
<223> Description of Artificial Sequence: Synthetic peptide


<220>
<221> MOD_RES
<222> (12)..(12)
<223> Phosphorylated Tyr

<400> 358
Ser Thr Leu Gln His Pro Asp Tyr Leu Gln Glu Xaa Ser Thr Lys
1               5                   10                  15


<210> 359
<211> 33
<212> PRT
<213> Artificial Sequence

<220>
<223> Description of Artificial Sequence: Synthetic peptide


<220>
<221> MOD_RES
<222> (32)..(32)
<223> Phosphorylated Tyr

<400> 359
Pro Ala Val Met Thr Ser Pro Leu Tyr Leu Glu Ile Ile Ile Tyr Cys
1               5                   10                  15


Thr Gly Ala Phe Leu Ile Ser Cys Met Val Gly Ser Val Ile Val Xaa
            20                  25                  30


Lys



<210> 360
<211> 10
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 360
Asp Ile Tyr Lys Asn Pro Asp Xaa Val Arg
1               5                   10


<210> 361
<211> 14
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 361
Asp Ile Leu Asp Arg Glu Tyr Xaa Ser Val Gln Gln His Arg
1               5                   10


<210> 362
<211> 28
<212> PRT
<213> Artificial Sequence

<220>
<223> Description of Artificial Sequence: Synthetic peptide


<220>
<221> MOD_RES
<222> (21)..(21)
<223> Phosphorylated Tyr

<400> 362
Phe Ile Pro Ile Asn Gly Tyr Pro Ile Pro Pro Gly Tyr Ala Ala Phe
1               5                   10                  15


Pro Ala Ala His Xaa Gln Pro Thr Gly Pro Pro Arg
            20                  25


<210> 363
<211> 9
<212> PRT
<213> Artificial Sequence

<220>
<223> Description of Artificial Sequence: Synthetic peptide


<220>
<221> MOD_RES
<222> (3)..(3)
<223> Phosphorylated Tyr

<400> 363
Asp Ile Xaa Lys Asn Asp Tyr Tyr Arg
1               5

<210> 364
<211> 9
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 364
Asp Ile Tyr Lys Asn Asp Xaa Tyr Arg
1               5

<210> 365
<211> 19
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 365
Pro Xaa Thr Gly Lys Val Gly Ala Glu Asp Ala Asp Gly Ile Asp Met
1               5                   10                  15

Ala Tyr Arg

<210> 366
<211> 35
<212> PRT
<213> Artificial Sequence

<220>
<223> Description of Artificial Sequence: Synthetic peptide

<220>
<221> MOD_RES
<222> (19)..(19)
<223> Phosphorylated Tyr

<400> 366
Leu Ala Lys Met Lys Ile Pro Pro Ser Glu Met Phe Leu Ser Glu Thr
1               5                   10                  15

Asp Lys Xaa Ser Lys Phe Asp Glu Asn Gly Leu Pro Thr His Asp Met
            20                  25                  30

Glu Gly Lys
        35

<210> 367
<211> 29
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 367
Thr Gly Asn Lys Tyr Asn Val Tyr Pro Thr Xaa Asp Phe Ala Cys Pro
1               5                   10                  15

Ile Val Asp Ser Ile Glu Gly Val Thr His Ala Leu Arg
            20                  25

<210> 368
<211> 7
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 368
Tyr Leu Xaa Glu Ile Ala Arg
1               5

<210> 369
<211> 15
<212> PRT
<213> Artificial Sequence

<220>

<223> Description of Artificial Sequence: Synthetic peptide

<220>
<221> MOD_RES
<222> (3)..(3)
<223> Phosphorylated Tyr

<400> 369
Ser Leu Xaa His Asp Ile Ser Gly Asp Thr Ser Gly Asp Tyr Arg
1               5                   10                  15

<210> 370
<211> 10
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 370
Ala Leu Leu Xaa Leu Cys Gly Gly Asp Asp
1               5                   10

<210> 371
<211> 11
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 371
Ser Leu Tyr Tyr Xaa Ile Gln Gln Asp Thr Lys
1               5                   10

<210> 372
<211> 11
<212> PRT
<213> Artificial Sequence

<220>
<223> Description of Artificial Sequence: Synthetic peptide

<220>

<221> MOD_RES
<222> (3)..(3)
<223> Phosphorylated Tyr

<400> 372
Ser Leu Xaa Tyr Tyr Ile Gln Gln Asp Thr Lys
1               5                   10


<210> 373
<211> 11
<212> PRT
<213> Artificial Sequence

<220>
<223> Description of Artificial Sequence: Synthetic peptide


<220>
<221> MOD_RES
<222> (4)..(4)
<223> Phosphorylated Tyr

<400> 373
Ser Leu Tyr Xaa Tyr Ile Gln Gln Asp Thr Lys
1               5                   10


<210> 374
<211> 19
<212> PRT
<213> Artificial Sequence

<220>
<223> Description of Artificial Sequence: Synthetic peptide


<220>
<221> MOD_RES
<222> (18)..(18)
<223> Phosphorylated Tyr

<400> 374
Leu Tyr Gly Lys Ser Leu Tyr Ser Phe Ile Lys Gly Asp Thr Ser Gly
1               5                   10                  15


Asp Xaa Arg


<210> 375
<211> 19
<212> PRT
<213> Artificial Sequence

<220>
<223> Description of Artificial Sequence: Synthetic peptide


<220>

<221> MOD_RES
<222> (2)..(2)
<223> Phosphorylated Tyr

<400> 375
Leu Xaa Gly Lys Ser Leu Tyr Ser Phe Ile Lys Gly Asp Thr Ser Gly
1               5                   10                  15

Asp Tyr Arg

<210> 376
<211> 8
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 376
Leu Tyr Asp Ala Xaa Glu Leu Lys
1               5

<210> 377
<211> 8
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 377
Glu Phe Ile Glu Lys Xaa Asp Lys
1               5

<210> 378
<211> 29
<212> PRT
<213> Artificial Sequence

<220>
<223> Description of Artificial Sequence: Synthetic peptide

<220>

<221> MOD_RES
<222> (13)..(13)
<223> Phosphorylated Tyr

<400> 378
Glu Arg Pro Ile Ser Met Ile Asn Glu Ala Ser Asn Xaa Asn Val Thr
1               5                   10                  15

Ser Asp Tyr Ala Val His Pro Met Ser Pro Val Gly Arg
            20                  25

<210> 379
<211> 29
<212> PRT
<213> Artificial Sequence

<220>
<223> Description of Artificial Sequence: Synthetic peptide

<220>
<221> MOD_RES
<222> (19)..(19)
<223> Phosphorylated Tyr

<400> 379
Glu Arg Pro Ile Ser Met Ile Asn Glu Ala Ser Asn Tyr Asn Val Thr
1               5                   10                  15

Ser Asp Xaa Ala Val His Pro Met Ser Pro Val Gly Arg
            20                  25

<210> 380
<211> 12
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 380
Ser Met Gly Phe Ile Gly His Xaa Leu Asp Gln Lys
1               5                   10

<210> 381
<211> 15
<212> PRT
<213> Artificial Sequence

<220>

<223> Description of Artificial Sequence: Synthetic peptide

<220>
<221> MOD_RES
<222> (8)..(8)
<223> Phosphorylated Tyr

<400> 381
Val Leu Glu Ala Ile Asp Thr Xaa Cys Glu Gln Lys Glu Trp Ala
1               5                   10                  15

<210> 382
<211> 20
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 382
Arg Asn Ile Ile Lys Asp Tyr Ser Asp Tyr Ala Ser Gln Val Xaa Gly
1               5                   10                  15

Pro Leu Ser Arg
            20

<210> 383
<211> 15
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 383
Lys Val Leu His Ile Pro Glu Phe Xaa Val Gly Ser Ile Leu Arg
1               5                   10                  15

<210> 384
<211> 24
<212> PRT
<213> Artificial Sequence

<220>

<223> Description of Artificial Sequence: Synthetic peptide

<220>
<221> MOD_RES
<222> (12)..(12)
<223> Phosphorylated Tyr

<400> 384
Met Gln Ser Leu Ser Pro Asp Pro Lys Ala Gln Xaa Thr Ser Ile Tyr
1               5                   10                  15

Gly Ala Leu Lys Lys Ile Met Arg
            20

<210> 385
<211> 11
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 385
Ala Ala Tyr Phe Gly Val Xaa Asp Thr Ala Lys
1               5                   10

<210> 386
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 386
Lys Lys Glu Phe Asp Lys Lys Xaa Asn Pro Thr Trp His Cys Ile
1               5                   10                  15

<210> 387
<211> 21
<212> PRT
<213> Artificial Sequence

<220>

<223> Description of Artificial Sequence: Synthetic peptide

<220>
<221> MOD_RES
<222> (4)..(4)
<223> Phosphorylated Tyr

<400> 387
Ala Gln Glu Xaa Asp Ser Ser Phe Pro Asn Trp Glu Phe Ala Arg Met
1               5                   10                  15

Ile Lys Glu Phe Arg
            20

<210> 388
<211> 26
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 388
Asn Asn Leu Ala Ser Ala Xaa Leu Lys Gln Asn Lys Tyr Gln Gln Ala
1               5                   10                  15

Glu Glu Leu Tyr Lys Glu Ile Leu His Lys
            20                  25

<210> 389
<211> 26
<212> PRT
<213> Artificial Sequence

<220>
<223> Description of Artificial Sequence: Synthetic peptide

<220>
<221> MOD_RES
<222> (13)..(13)
<223> Phosphorylated Tyr

<400> 389
Asn Asn Leu Ala Ser Ala Tyr Leu Lys Gln Asn Lys Xaa Gln Gln Ala
1               5                   10                  15

Glu Glu Leu Tyr Lys Glu Ile Leu His Lys
            20                  25

<210> 390
<211> 34
<212> PRT
<213> Artificial Sequence

<220>
<223> Description of Artificial Sequence: Synthetic peptide


<220>
<221> MOD_RES
<222> (31)..(31)
<223> Phosphorylated Tyr

<400> 390
Pro Glu Asp Val Tyr Ala Met Asn Pro Pro Lys Phe Asp Arg Ile Glu
1               5                   10                  15


Asp Met Ala Met Leu Thr His Leu Asn Glu Pro Ala Val Leu Xaa Asn
            20                  25                  30


Leu Lys



<210> 391
<211> 34
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 391
Pro Glu Asp Val Xaa Ala Met Asn Pro Pro Lys Phe Asp Arg Ile Glu
1               5                   10                  15


Asp Met Ala Met Leu Thr His Leu Asn Glu Pro Ala Val Leu Tyr Asn
            20                  25                  30


Leu Lys



<210> 392
<211> 11
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 392
Glu Leu Thr Xaa Gln Thr Glu Glu Asp Arg Lys
1               5                   10

<210> 393
<211> 10
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 393
Thr Lys Xaa Glu Thr Asp Ala Ile Gln Arg
1               5                   10

<210> 394
<211> 10
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 394
Val Lys Val Gly Asn Glu Xaa Val Thr Lys
1               5                   10

<210> 395
<211> 15
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 395
Asn Ile Cys Xaa Val Ile Thr His Gly Asp Ala Lys Asp Gln Glu
1               5                   10                  15


<210> 396
<211> 20
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 396
Asn Arg Asn Phe Xaa Glu Leu Ser Pro His Ile Phe Ala Leu Ser Asp
1               5                   10                  15


Glu Ala Tyr Arg
            20


<210> 397
<211> 35
<212> PRT
<213> Artificial Sequence

<220>
<223> Description of Artificial Sequence: Synthetic peptide


<220>
<221> MOD_RES
<222> (12)..(12)
<223> Phosphorylated Tyr

<400> 397
His Leu Lys Leu Gly Ser Ala Glu Glu Phe Asn Xaa Thr Arg Met Gly
1               5                   10                  15


Gly Asn Thr Val Ile Glu Gly Val Asn Asp Arg Ala Glu Met Val Glu
            20                  25                  30


Thr Gln Lys
        35


<210> 398

<211> 30
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 398
Met Xaa Asp Asn His Gln Leu Gly Lys Pro Glu Pro His Ile Tyr Ala
1               5                   10                  15


Val Ala Asp Val Ala Tyr His Ala Met Leu Gln Arg Lys Lys
            20                  25                  30


<210> 399
<211> 15
<212> PRT
<213> Artificial Sequence

<220>
<223> Description of Artificial Sequence: Synthetic peptide


<220>
<221> MOD_RES
<222> (10)..(10)
<223> Phosphorylated Tyr

<400> 399
His Ile Ala Glu Asp Ala Asp Arg Lys Xaa Glu Glu Val Ala Arg
1               5                   10                  15


<210> 400
<211> 15
<212> PRT
<213> Artificial Sequence

<220>
<223> Description of Artificial Sequence: Synthetic peptide


<220>
<221> MOD_RES
<222> (10)..(10)
<223> Phosphorylated Tyr

<400> 400
His Ile Ala Glu Asp Ser Asp Arg Lys Xaa Glu Glu Val Ala Arg
1               5                   10                  15


<210> 401

<211> 19
<212> PRT
<213> Artificial Sequence

<220>
<223> Description of Artificial Sequence: Synthetic peptide


<220>
<221> MOD_RES
<222> (19)..(19)
<223> Phosphorylated Tyr

<400> 401
Ala Cys Asn Val Leu Gln Ser Ser His Leu Glu Asp Tyr Pro Phe Asp
1               5                   10                  15


Ala Glu Xaa




<210> 402
<211> 14
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 402
Gln Ser Ser His Leu Glu Asp Xaa Pro Phe Asp Ala Glu Tyr
1               5                   10


<210> 403
<211> 22
<212> PRT
<213> Artificial Sequence

<220>
<223> Description of Artificial Sequence: Synthetic peptide


<220>
<221> MOD_RES
<222> (15)..(15)
<223> Phosphorylated Tyr

<400> 403
Leu Asn Asp Gly His Phe Met Pro Val Leu Gly Phe Gly Thr Xaa Ala
1               5                   10                  15


Pro Ala Glu Val Pro Lys

            20

<210> 404
<211> 27
<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 404
Tyr Val Glu Gly Ile Val Ser Leu His Xaa Lys Thr Asp Val Ala Val
1               5                   10                  15

Lys Asp Asp Pro Glu Leu Gln Thr Trp Cys Arg
            20                  25

<210> 405
<211> 12
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 405
Xaa Thr Arg Asn Leu Val Asp Gln Gly Asn Gly Lys
1               5                   10

<210> 406
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 406
Gln Asp Asp Ile Ser Ser Ser Xaa Thr Thr Thr Thr Thr Ile Thr

1                   5                   10                  15

<210> 407
<211> 15
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 407
Ala Lys Tyr Pro Asp Xaa Glu Val Thr Trp Ala Asn Asp Gly Tyr
1               5                   10                  15

<210> 408
<211> 24
<212> PRT
<213> Artificial Sequence

<220>
<223> Description of Artificial Sequence: Synthetic peptide

<220>
<221> MOD_RES
<222> (19)..(19)
<223> Phosphorylated Tyr

<400> 408
Gln Leu Ile Ile Glu Asp Pro Tyr Tyr Gly Asn Asp Ser Asp Phe Glu
1               5                   10                  15

Thr Val Xaa Gln Gln Cys Val Arg
            20

<210> 409
<211> 26
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 409
Glu Asp Xaa Val Leu Val Ala Gly His Tyr Pro Val Trp Ser Ile Ala

1               5                   10                  15

Glu His Gly Pro Thr His Cys Leu Val Lys
            20                  25

<210> 410
<211> 26
<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 410
Glu Asp Tyr Val Leu Val Ala Gly His Xaa Pro Val Trp Ser Ile Ala
1               5                   10                  15

Glu His Gly Pro Thr His Cys Leu Val Lys
            20                  25

<210> 411
<211> 23
<212> PRT
<213> Artificial Sequence

<220>
<223> Description of Artificial Sequence: Synthetic peptide

<220>
<221> MOD_RES
<222> (13)..(13)
<223> Phosphorylated Tyr

<400> 411
Lys Tyr Met Phe Pro Met Gly Thr Pro Asp Pro Glu Xaa Pro Ala Asp
1               5                   10                  15

Ala Ser Gln Asn Gly Ile Arg
            20

<210> 412
<211> 24
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 412
Leu Arg Glu Leu Glu Ala Glu Gly Xaa Lys Leu Val Ile Phe Thr Asn
1               5                   10                  15

Gln Met Ser Ile Gly Arg Gly Lys
            20

<210> 413
<211> 16
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 413
Ala Ser Glu Ser Ile Ser Arg Ala Xaa Ala Leu Cys Val Leu Tyr Arg
1               5                   10                  15

<210> 414
<211> 9
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 414
Ala Tyr Ala Leu Cys Val Leu Xaa Arg
1               5

<210> 415
<211> 27
<212> PRT
<213> Artificial Sequence

<220>
<223> Description of Artificial Sequence: Synthetic peptide

<220>
<221> MOD_RES
<222> (17)..(17)
<223> Phosphorylated Tyr

<400> 415
Thr Met Asn Asn Ser Ala Glu Asn His Thr Ala Asn Ser Ser Met Ala
1               5                   10                  15

Xaa Pro Ser Leu Val Ala Met Ala Ser Gln Arg
            20                  25

<210> 416
<211> 11
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 416
Xaa Ala Asp Pro Val Ala Asp Leu Leu Asp Lys
1               5                   10

<210> 417
<211> 16
<212> PRT
<213> Artificial Sequence

<220>
<223> Description of Artificial Sequence: Synthetic peptide

<220>
<221> MOD_RES
<222> (5)..(5)
<223> Phosphorylated Tyr

<400> 417
Val Val Gln Glu Xaa Ile Asp Ala Phe Ser Asp Tyr Ala Asn Phe Lys
1               5                   10                  15

<210> 418
<211> 10
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 418
Arg Leu Asn Xaa Gln Thr Pro Gly Met Arg
1               5                   10


<210> 419
<211> 13
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 419
Met Ser Phe Ile Asp Xaa Gln His Ile Ile Val Glu His
1               5                   10


<210> 420
<211> 15
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 420
Pro Xaa Leu Thr Val Asp Gln Met Met Asp Phe Ile Asn Leu Lys
1               5                   10                  15


<210> 421
<211> 15
<212> PRT
<213> Artificial Sequence

<220>
<223> Description of Artificial Sequence: Synthetic peptide


<220>
<221> MOD_RES
<222> (14)..(14)

<223> Phosphorylated Tyr

<400> 421
Arg Lys Ala Gln Gln Gly Val Arg Ile Phe Ile Met Leu Xaa Lys
1               5                   10                  15


<210> 422
<211> 21
<212> PRT
<213> Artificial Sequence

<220>
<223> Description of Artificial Sequence: Synthetic peptide


<220>
<221> MOD_RES
<222> (4)..(4)
<223> Phosphorylated Tyr

<400> 422
Glu Ile Thr Xaa Gly Asn Asn Lys Pro Val Lys Ala Pro Val Gln Glu
1               5                   10                  15


Arg Tyr Val Glu Lys
            20


<210> 423
<211> 23
<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 423
Lys Ala Leu Gln Ala Val Xaa Ser Met Met Ser Trp Pro Asp Asp Val
1               5                   10                  15


Pro Pro Glu Gly Trp Asn Arg
            20


<210> 424
<211> 20
<212> PRT
<213> Artificial Sequence

<220>
<223> Description of Artificial Sequence: Synthetic peptide

<220>
<221> MOD_RES
<222> (12)..(12)
<223> Phosphorylated Tyr

<400> 424
Pro Ala Ile Thr Tyr Gly Thr Arg Gly Asn Ser Xaa Phe Met Val Glu
1               5                   10                  15

Val Lys Cys Arg
            20

<210> 425
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 425
Ala Ala Arg Ala Lys Leu Gln Xaa Met Met Val Met Val Gly Tyr
1               5                   10                  15

<210> 426
<211> 19
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 426
Gly Leu Gly Glu His Glu Met Glu Glu Asp Glu Glu Asp Xaa Glu Ser
1               5                   10                  15

Ser Ala Lys

<210> 427
<211> 13
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 427
Leu Gln Glu Glu Ser Asp Xaa Ile Thr His Tyr Thr Arg
1               5                   10

<210> 428
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 428
Ile Lys Glu Gln Leu His Lys Xaa Val Arg Glu Leu Glu Gln Ala
1               5                   10                  15

<210> 429
<211> 17
<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 429
Tyr Ala Leu Leu Ala Val Met Gly Ala Xaa Val Leu Leu Lys Arg Glu
1               5                   10                  15

Ser

<210> 430
<211> 17
<212> PRT
<213> Artificial Sequence

<220>
<223> Description of Artificial Sequence: Synthetic peptide

<220>
<221> MOD_RES
<222> (1)..(1)
<223> Phosphorylated Tyr

<400> 430
Xaa Ala Leu Leu Ala Val Met Gly Ala Tyr Val Leu Leu Lys Arg Glu
1               5                   10                  15

Ser

<210> 431
<211> 21
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 431
Gly Met Val Cys Gly Xaa Lys Glu Gln Gly Lys Asp Ser Cys Gln Gly
1               5                   10                  15

Asp Ser Gly Gly Arg
            20

<210> 432
<211> 20
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 432
Met Ala Phe Ala Lys Met Asp Pro Ser Cys Thr Val Gly Phe Xaa Ala
1               5                   10                  15

Gly Asp Arg Lys
            20

<210> 433
<211> 11
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 433
Cys Asp Pro Ala Gly Tyr Xaa Cys Gly Phe Lys
1               5                   10

<210> 434
<211> 35
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 434
Met Ala Ala Val Ser Val Xaa Ala Pro Pro Val Gly Gly Phe Ser Phe
1               5                   10                  15

Asp Asn Cys Arg Arg Asn Ala Val Leu Glu Ala Asp Phe Ala Lys Arg
            20                  25                  30

Gly Tyr Lys
        35

<210> 435
<211> 24
<212> PRT
<213> Artificial Sequence

<220>
<223> Description of Artificial Sequence: Synthetic peptide

<220>
<221> MOD_RES

<222> (7)..(7)
<223> Phosphorylated Tyr

<400> 435
Val Ile Glu Ile Asn Pro Xaa Leu Leu Gly Thr Met Ser Gly Cys Ala
1               5                   10                  15

Ala Asp Cys Gln Tyr Trp Glu Arg
            20

<210> 436
<211> 16
<212> PRT
<213> Artificial Sequence

<220>
<223> Description of Artificial Sequence: Synthetic peptide

<220>
<221> MOD_RES
<222> (10)..(10)
<223> Phosphorylated Tyr

<400> 436
Val Val Ser Ser Ser Ile Val Asp Lys Xaa Ile Gly Glu Ser Ala Arg
1               5                   10                  15

<210> 437
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 437
Phe Tyr Asp Leu Ser Ser Lys Xaa Tyr Gln Thr Ile Gly Asn His
1               5                   10                  15

<210> 438
<211> 15
<212> PRT
<213> Artificial Sequence

<220>
<223> Description of Artificial Sequence: Synthetic peptide

<220>
<221> MOD_RES

<222> (8)..(8)
<223> Phosphorylated Tyr

<400> 438
Thr Ile Gly Asn His Ala Ser Xaa Tyr Lys Asp Ala Leu Arg Phe
1               5                   10                  15


<210> 439
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 439
Thr Ser Val His Ser Arg Phe Xaa Asp Leu Ser Ser Lys Tyr Tyr
1               5                   10                  15


<210> 440
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 440
Tyr Asp Leu Ser Ser Lys Tyr Xaa Gln Thr Ile Gly Asn His Ala
1               5                   10                  15


<210> 441
<211> 10
<212> PRT
<213> Artificial Sequence

<220>
<223> Description of Artificial Sequence: Synthetic peptide


<220>
<221> MOD_RES
<222> (3)..(3)
<223> Phosphorylated Tyr

<400> 441

Leu Ala Xaa Val Ala Pro Thr Ile Pro Arg
1               5                   10

<210> 442
<211> 25
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 442
Val Xaa Phe Gln Ser Pro Pro Gly Ala Ala Gly Glu Gly Pro Gly Gly
1               5                   10                  15

Ala Asp Asp Glu Gly Pro Val Arg Arg
            20                  25

<210> 443
<211> 28
<212> PRT
<213> Artificial Sequence

<220>
<223> Description of Artificial Sequence: Synthetic peptide

<220>
<221> MOD_RES
<222> (7)..(7)
<223> Phosphorylated Tyr

<400> 443
Ala Phe Leu Pro Ala Leu Xaa Ser Leu Leu Phe Leu Leu Gly Leu Leu
1               5                   10                  15

Gly Asn Gly Ala Val Ala Ala Val Leu Leu Ser Arg
            20                  25

<210> 444
<211> 15
<212> PRT
<213> Artificial Sequence

<220>
<223> Description of Artificial Sequence: Synthetic peptide

<220>
<221> MOD_RES

<222> (8)..(8)
<223> Phosphorylated Tyr

<400> 444
Thr Thr Ile Ala Val Asp Arg Xaa Val Val Leu Val His Pro Leu
1               5                   10                  15


<210> 445
<211> 17
<212> PRT
<213> Artificial Sequence

<220>
<223> Description of Artificial Sequence: Synthetic peptide


<220>
<221> MOD_RES
<222> (13)..(13)
<223> Phosphorylated Tyr

<400> 445
Ser Leu Ser Ser Ser Ser Ile Gly Ser Asn Ser Thr Xaa Leu Thr Ser
1               5                   10                  15


Lys



<210> 446
<211> 26
<212> PRT
<213> Artificial Sequence

<220>
<223> Description of Artificial Sequence: Synthetic peptide


<220>
<221> MOD_RES
<222> (25)..(25)
<223> Phosphorylated Tyr

<400> 446
Ile Leu Ile Gln Leu Cys Ala Ala Leu Leu Leu Leu Asn Leu Val Phe
1               5                   10                  15


Leu Leu Asp Ser Trp Ile Ala Leu Xaa Lys
            20                  25


<210> 447
<211> 14
<212> PRT
<213> Artificial Sequence

<220>
<223> Description of Artificial Sequence: Synthetic peptide

<220>
<221> MOD_RES
<222> (11)..(11)
<223> Phosphorylated Tyr

<400> 447
Ala His Ala Trp Pro Ser Pro Tyr Lys Asp Xaa Glu Val Lys
1               5                   10


<210> 448
<211> 14
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 448
Ala His Ala Trp Pro Ser Pro Xaa Lys Asp Tyr Glu Val Lys
1               5                   10


<210> 449
<211> 17
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 449
Ala Glu Asp Met Xaa Ser Ala Gln Ser His Gln Ala Ala Thr Pro Pro
1               5                   10                  15


Lys


<210> 450
<211> 15
<212> PRT
<213> Artificial Sequence

<220>
<223> Description of Artificial Sequence: Synthetic peptide

<220>
<221> MOD_RES
<222> (8)..(8)
<223> Phosphorylated Tyr

<400> 450
Lys Val Pro Ser Glu Gly Ala Xaa Asp Ile Ile Leu Pro Arg Ala
1               5                   10                  15


<210> 451
<211> 11
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 451
Ser Gln Val Phe Arg Asn Pro Xaa Val Trp Asp
1               5                   10


<210> 452
<211> 13
<212> PRT
<213> Artificial Sequence

<220>
<223> Description of Artificial Sequence: Synthetic peptide


<220>
<221> MOD_RES
<222> (7)..(7)
<223> Phosphorylated Tyr

<400> 452
Val Pro Ser Glu Gly Ala Xaa Asp Ile Ile Leu Pro Arg
1               5                   10


<210> 453
<211> 21
<212> PRT
<213> Artificial Sequence

<220>
<223> Description of Artificial Sequence: Synthetic peptide


<220>
<221> MOD_RES

<222> (2)..(2)
<223> Phosphorylated Tyr

<400> 453
Ile Xaa Phe Ala Val Arg Asn Pro Glu Leu Met Ala Thr Asn Lys Asp
1               5                   10                  15

Thr Lys Ile Ala Lys
            20

<210> 454
<211> 18
<212> PRT
<213> Artificial Sequence

<220>
<223> Description of Artificial Sequence: Synthetic peptide

<220>
<221> MOD_RES
<222> (14)..(14)
<223> Phosphorylated Tyr

<400> 454
Glu Ile Lys Thr Ala Met Trp Arg Leu Phe Val Lys Ile Xaa Phe Leu
1               5                   10                  15

Gln Lys

<210> 455
<211> 23
<212> PRT
<213> Artificial Sequence

<220>
<223> Description of Artificial Sequence: Synthetic peptide

<220>
<221> MOD_RES
<222> (6)..(6)
<223> Phosphorylated Tyr

<400> 455
Val Val Ser Val Leu Xaa Thr Glu Val Ile Pro Met Leu Asn Pro Leu
1               5                   10                  15

Ile Tyr Ser Leu Arg Asn Lys
            20

<210> 456
<211> 24
<212> PRT

<213> Artificial Sequence

<220>
<223> Description of Artificial Sequence: Synthetic peptide


<220>
<221> MOD_RES
<222> (11)..(11)
<223> Phosphorylated Tyr

<400> 456
Leu Gln Arg Phe Ile Phe His Val Asn Leu Xaa Gly Ser Ile Leu Phe
1               5                   10                  15


Leu Thr Cys Ile Ser Ala His Arg
            20


<210> 457
<211> 21
<212> PRT
<213> Artificial Sequence

<220>
<223> Description of Artificial Sequence: Synthetic peptide


<220>
<221> MOD_RES
<222> (6)..(6)
<223> Phosphorylated Tyr

<400> 457
Asp Val Phe Asn Val Xaa Val Asn Ser Ser Ile Pro Ile Pro Ser Ser
1               5                   10                  15


Asn Ser Thr Glu Lys
            20


<210> 458
<211> 9
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 458
Asn Gln Glu Thr Xaa Glu Thr Leu Lys
1               5

<210> 459
<211> 25
<212> PRT
<213> Artificial Sequence

<220>
<223> Description of Artificial Sequence: Synthetic peptide


<220>
<221> MOD_RES
<222> (21)..(21)
<223> Phosphorylated Tyr

<400> 459
Gly Leu Lys Asp Met Gly Tyr Gly Asn Trp Ile Ser Lys Pro Gln Glu
1               5                   10                  15


Glu Lys Asn Phe Xaa Leu Cys Pro Val
            20                  25


<210> 460
<211> 15
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 460
Lys Asp Pro Asp Ser Asn Pro Xaa Ser Leu Leu Asp Thr Ser Glu
1               5                   10                  15


<210> 461
<211> 28
<212> PRT
<213> Artificial Sequence

<220>
<223> Description of Artificial Sequence: Synthetic peptide


<220>
<221> MOD_RES
<222> (24)..(24)
<223> Phosphorylated Tyr

<400> 461
Gly Phe Gly Asp Gly Tyr Asn Gly Tyr Gly Gly Gly Pro Gly Gly Gly
1               5                   10                  15

Asn Phe Gly Gly Ser Pro Gly Xaa Gly Gly Gly Arg
            20                  25

<210> 462
<211> 31
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 462
Gly Gly Asp Gly Tyr Asp Gly Gly Tyr Gly Gly Phe Asp Asp Xaa Gly
1               5                   10                  15

Gly Tyr Asn Asn Tyr Gly Tyr Gly Asn Asp Gly Phe Asp Asp Arg
            20                  25                  30

<210> 463
<211> 18
<212> PRT
<213> Artificial Sequence

<220>
<223> Description of Artificial Sequence: Synthetic peptide

<220>
<221> MOD_RES
<222> (2)..(2)
<223> Phosphorylated Tyr

<400> 463
Asp Xaa Phe Glu Lys Cys Ser Lys Ile Glu Thr Ile Glu Val Met Glu
1               5                   10                  15

Asp Arg

<210> 464
<211> 21
<212> PRT
<213> Artificial Sequence

<220>
<223> Description of Artificial Sequence: Synthetic peptide

<220>

<221> MOD_RES
<222> (13)..(13)
<223> Phosphorylated Tyr

<400> 464
Pro Asp Gly Lys Leu Arg Tyr Ala Asn Asn Ser Asn Xaa Lys Asn Asp
1               5                   10                  15

Val Met Ile Arg Lys
            20

<210> 465
<211> 9
<212> PRT
<213> Artificial Sequence

<220>
<223> Description of Artificial Sequence: Synthetic peptide

<220>
<221> MOD_RES
<222> (3)..(3)
<223> Phosphorylated Tyr

<400> 465
Met Asp Xaa Glu Asp Asp Arg Leu Arg
1               5

<210> 466
<211> 34
<212> PRT
<213> Artificial Sequence

<220>
<223> Description of Artificial Sequence: Synthetic peptide

<220>
<221> MOD_RES
<222> (4)..(4)
<223> Phosphorylated Tyr

<400> 466
Ala Ala Gln Xaa Gln Val Asn Gln Ala Ala Ala Ala Gln Ala Ala Ala
1               5                   10                  15

Thr Ala Ala Ala Met Gly Ile Pro Gln Ala Val Leu Pro Pro Leu Pro
            20                  25                  30

Lys Arg

<210> 467
<211> 21

<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 467
Ala Asp Lys Asp Xaa His Phe Lys Val Asp Asn Asp Glu Asn Glu His
1               5                   10                  15


Gln Leu Ser Leu Arg
            20


<210> 468
<211> 21
<212> PRT
<213> Artificial Sequence

<220>
<223> Description of Artificial Sequence: Synthetic peptide


<220>
<221> MOD_RES
<222> (18)..(18)
<223> Phosphorylated Tyr

<400> 468
Asn Gly Ile Pro Tyr Leu Asn Gln Glu Glu Glu Arg Gln Leu Arg Glu
1               5                   10                  15


Gln Xaa Asp Glu Lys
            20


<210> 469
<211> 21
<212> PRT
<213> Artificial Sequence

<220>
<223> Description of Artificial Sequence: Synthetic peptide


<220>
<221> MOD_RES
<222> (5)..(5)
<223> Phosphorylated Tyr

<400> 469
Asn Gly Ile Pro Xaa Leu Asn Gln Glu Glu Glu Arg Gln Leu Arg Glu
1               5                   10                  15

Gln Tyr Asp Glu Lys
            20


<210> 470
<211> 28
<212> PRT
<213> Artificial Sequence

<220>
<223> Description of Artificial Sequence: Synthetic peptide


<220>
<221> MOD_RES
<222> (19)..(19)
<223> Phosphorylated Tyr

<400> 470
Ala Gln Glu Tyr Ile Arg Gln Lys Asn Lys Gly Ala Lys Leu Lys Val
1               5                   10                  15


Gly Gln Xaa Leu Asn Cys Ile Val Glu Lys Val Lys
            20                  25


<210> 471
<211> 8
<212> PRT
<213> Artificial Sequence

<220>
<223> Description of Artificial Sequence: Synthetic peptide


<220>
<221> MOD_RES
<222> (2)..(2)
<223> Phosphorylated Tyr

<400> 471
Ile Xaa Glu Tyr Val Glu Ser Arg
1               5


<210> 472
<211> 19
<212> PRT
<213> Artificial Sequence

<220>
<223> Description of Artificial Sequence: Synthetic peptide


<220>
<221> MOD_RES
<222> (8)..(8)
<223> Phosphorylated Tyr

<400> 472
Asn Gln Gly Gly Tyr Asp Arg Xaa Ser Gly Gly Asn Tyr Arg Asp Asn
1               5                   10                  15

Tyr Asp Asn

<210> 473
<211> 19
<212> PRT
<213> Artificial Sequence

<220>
<223> Description of Artificial Sequence: Synthetic peptide

<220>
<221> MOD_RES
<222> (15)..(15)
<223> Phosphorylated Tyr

<400> 473
Asp Asp Gly Tyr Ser Thr Lys Asp Ser Tyr Ser Ser Arg Asp Xaa Pro
1               5                   10                  15

Ser Ser Arg

<210> 474
<211> 11
<212> PRT
<213> Artificial Sequence

<220>
<223> Description of Artificial Sequence: Synthetic peptide

<220>
<221> MOD_RES
<222> (9)..(9)
<223> Phosphorylated Tyr

<400> 474
His Gly Val Val Pro Leu Ala Thr Xaa Met Arg
1               5                   10